(19) Europäisches Patentamt
European Patent Office
Office européen des brevets

(11) EP 1 616 862 A1

(12) EUROPEAN PATENT APPLICATION
published in accordance with Art. 158(3) EPC

(43) Date of publication:
**18.01.2006 Bulletin 2006/03**

(21) Application number: **04728041.7**

(22) Date of filing: **16.04.2004**

(51) Int Cl.:
*C07D 211/26* (1985.01) *C07D 211/52* (1985.01)
*C07D 211/28* (1985.01) *C07D 211/70* (1985.01)
*C07D 307/68* (1985.01) *C07D 295/12* (1985.01)
*C07D 215/50* (1985.01) *C07D 213/60* (1985.01)
*C07D 213/20* (1985.01) *C07D 409/06* (1990.01)
*C07D 401/10* (1990.01) *C07D 333/28* (1985.01)
*A61K 31/451* (2000.01) *A61K 31/454* (2000.01)
*A61K 31/44* (1985.01) *A61K 31/381* (2000.01)
*A61K 31/341* (2000.01) *A61K 31/495* (1985.01)
*A61K 31/496* (2000.01) *A61K 31/40* (1985.01)
*A61K 31/4709* (2000.01)

(86) International application number:
**PCT/JP2004/005504**

(87) International publication number:
**WO 2004/092136 (28.10.2004 Gazette 2004/44)**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **18.04.2003 JP 2003114172**
**30.10.2003 JP 2003346384**

(71) Applicant: **ONO PHARMACEUTICAL CO., LTD.**
**Osaka-shi,**
**Osaka 541-8526 (JP)**

(72) Inventors:
- **HABASHITA, Hiromu,**
  **Ono Pharmaceutical Co., Ltd.**
  **Mishima-gun, Osaka 618-8585 (JP)**
- **NISHIZAKI, Minoru,**
  **Ono Pharmaceutical Co., Ltd.**
  **Mishima-gun, Osaka 618-8585 (JP)**
- **HAYASHI, Kazuya,**
  **Ono Pharmaceutical Co., Ltd.**
  **Sakai-gun,**
  **Fukui 913-0032 (JP)**
- **SHIBAYAMA, Shiro,**
  **Ono Pharmaceutical Co., Ltd.**
  **Tsukuba-shi, Ibaraki 300-4247 (JP)**

(74) Representative: **Henkel, Feiler & Hänzel**
**Möhlstrasse 37**
**81675 München (DE)**

(54) **NITROGENOUS HETEROCYCLIC COMPOUND AND USE THEREOF**

(57) The present invention relates to a medicament comprising the compound of formula (I)

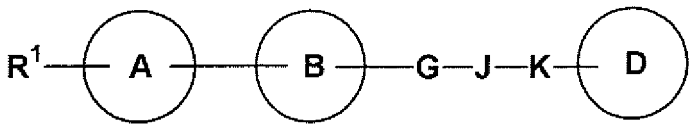

**(I)**

,

wherein all symbols have the same meanings as defined in the specification, a salt thereof or a prodrug thereof The compound of the present invention is useful for the prevention and/or treatment of immune diseases such as various types of inflammation, autoimmune disease, allergic diseases, etc.; infection concerning inflammation or HIV infections (e.g. asthma, nephritis, nephropathy, hepatitis, arthritis, rheumatoid arthritis, rhinitis, conjunctivitis, ulcerative colitis, *etc.*), organ transplantation rejection, immunosuppression, psoriasis, multiple sclerosis, optic neuritis, polymyalgia rheumatica syndrome, uveitis, vasculitis, human immunodeficiency virus infection (acquired immunodeficiency syndrome *etc.),* atopic dermatitis, urticaria, allergic bronchopulmonary aspergillosis, allergic eosinophilic gastroenteritis, osteoarthritis, ischemic reperfusion injury, acute respiratory distress syndrome, shock accompanying bacteria infection, diabetes, cancer metastasis, atherosclerosis, *etc.*).

EP 1 616 862 A1

**Description**

Technical Field

The present invention relates to a nitrogen-containing heterocyclic compound having an antagonistic effect against chemokine receptors (particularly, CCR1 and/or CCR5), which is useful as a medicament, a method for the preparation thereof and use thereof

Background Art

Chemokine is known as a basic protein having endogeneous leukocyte chemotactic and activating abilities and strong heparin-binding ability. At present, it is considered that chemokine is related not only to the control of infiltration of specific leukocyte at the time of inflammations and immune responses but also to genesis, to homing of lymphocyte under physiological conditions and to migration of hemocyte precursor cells and somatic cells.

Differentiation, proliferation and cell death of hemocytes are controlled by various types of cytokine. In the living body, inflammations are found topically and differentiation, maturation and the like of lymphocytes are carried out at certain specified sites. That is, various necessary cells migrate into certain specified sites and accumulate therein to cause a series of inflammations and immune responses. Accordingly, migration of cells is also an indispensable phenomenon to immune system as well as differentiation, proliferation and death of cells.

Migration of hemocytes in the living body starts first in the development stage by the shift of hematopoiesis started in the AGM (aorta gonad mesonephros) region into permanent hematopoiesis in bone marrow via fetal liver. Furthermore, precursor cells of T cells and thymus dendritic cells migrate from the fetal into the bone marrow and then into the thymus gland and cytodifferentiate under thymus environment. The T cell which received clone selection migrates into secondary lymphoid tissues and takes part in an immune response in the periphery. The Langerhans' cell of the skin activated and differentiated by capturing an antigen migrates into the T cell region of a topical lymph node and activates naïve T cell therein as a dendritic cell. The memory T cell performs its homing again into the lymph node via lymphatic and blood vessels. Also, B cell, T cell in the intestinal epithelium, $\gamma\delta$ T cell, NKT cell and dendritic cell migrate from bone marrow without passing through the thymus gland and differentiate to take part in immune responses.

Chemokine and its receptor are deeply related to the migration of these various cells. For example, MIP3β. (macrophage inflammatory protein 3β), SLC (secondary lymphoid tissue chemokine) and a receptor thereof (CCR7) play important roles in the migration and homing of naive T cells, memory T cells and the mature dendritic cells which captured an antigen into a topical lymphoid tissue for the dendritic cells to encounter the T cells efficiently. The T cells and dendritic cells necessary for controlling antigen-specific immune responses are hardly observed in the secondary lymph node of a PLT mouse having deficiency in the expression of SLC (*J. Exp. Med.*, 189(3), 451 (1999)).

MDC (macrophage-derived chemokine), TARC (thymus and activation - regulated chemokine) and a receptor thereof (CCR4) play important roles in the migration of Th2 cells into topical sites in immune and inflammatory responses to which the Th2 cells are related. In a rat fluminant hepatitis model (P. acnes + LPS) an anti-TARC antibody suppressed increase of the amount ofALT in blood and increase of the expressing amounts of TNFα and FasL in the liver and also improved lethality of the rats (*J. Clin. Invest.,* 102, 1933 (1998)). Also, an anti-MDC antibody decreased the number of eosinophils accumulated in the lung interstitum and suppressed airway hypersensitivity in a mouse OVA-induced airway hypersensitivity model (*J. Immunology,* 163, 403 (1999)).

MCP-1 (monocyte chemoattractant protein-1) and a receptor thereof (CCR2) are related to the infiltration of macrophages into inflammation sites. An anti-MCP-I antibody showed an effect to suppress infiltration of monocyte and macrophage into glomerulus in a rat anti-Thy1.1 antibody glomerular nephritis model *(Kidney Int.,* 51, 770 (1997)).

Thus, chemokine receptors are greatly related to the control of inflammation and immune responses through a mechanism in which they are expressed at certain specified periods in variously specific cells and the effector cells are accumulated in the region where chemokine is produced. Therefore, chemokine receptors are considered to be deeply related to immune diseases such as various inflammatory diseases, autoimmune diseases, allergic diseases, *etc.,* infections associated with inflammation, or HIV infection. For example, asthma, nephritis, nephropathy, hepatitis, arthritis, rheumatoid arthritis, rhinitis, conjunctivitis, ulcerative colitis, organ rejection in transplantation, immunosuppression, psoriasis, multiple sclerosis, optic neuritis, polymyalgia rheumatica syndrome, uveitis, vasculitis, human immunodeficiency virus infection (acquired immunodeficiency syndrome *etc.),* atopic dermatitis, urticaria, allergic bronchopulmonary aspergillosis, allergic eosinophilic gastroenteritis, osteoarthritis, ischemic reperfusion injury, acute respiratory distress syndrome, shock accompanying bacteria infection, diabetes, cancer metastasis, atherosclerosis, *etc.*

It is considered that in autoimmune diseases such as multiple sclerosis, rheumatoid arthritis, etc., overexpressed inflammation and tissue damages occur by interactions of T cells infiltrating into the affected part of inflammation and macrophages existing in the tissues, resulting in progressing to chronic symptoms. It is reported that CCR1 (one of chemokine receptors) is expressed in these cells,. Also, results in various animal models of autoimmune diseases imply

that CCR1 is concerned with autoimmune diseases.

For example, it was reported that in a mouse EAE model (a model of multiple sclerosis), an antibody to MIP-1α (one of ligands to CCRI) inhibited the infiltration of monocyte into central nervous system, and at the same time, it suppressed the progress of early and reentry pararisis symptoms. This fact implies that MIP-1α plays an important role in this disease, which mediates T cells. Moreover, it was reported that in a mouse EAE model, CCR1 knockout mice showed a significantly low incidence compared with wildtype mice. From these results, it is considered that CCR1 is involved with symptoms and progression of diseases in a mouse EAE model. This implies that CCR1 is concerned with symptoms and progression of diseases of multiple sclerosis, which is a human disease.

The effect of a selective CCR1 antagonist on rheumatoid arthritis, one of autoimmune diseases, has been reported. In a group to which a CCR1 antagonist was administered, some clinical improvements were confirmed such as inhibition of anlargement of joint, improvement of QOL, *etc.*

A selective CCR1 antagonist BX471 is reported to delay the rejection in a transplantation model of kidney or heart.

On the other hand, acquired immunodeficiency syndrome (what is called AIDS), which is caused by human immunodeficiency virus (abbreviated to HIV hereafter) is one of those diseases whose method of treatment have been longed for recently. Once the infection of HIV is formed on CD4 positive cells (main target cells), HIV repeats multiplication in the patient's body, resulting in catastrophically breaking the T cells which govern immune function before long. In this process, the immune function is declined gradually, resulting in various immunodeficiency symptoms such as fever, diarrhea, anlargement of lymph node, *etc.* and the patient will be prone to combine opportunistic infection such as carinii pneumonia *etc*. These conditions are occurrences of AIDS; and it is well-known that they induce malignant tumors such as Kaposi's sarcoma, and the conditions become severe.

At present, for the prevention and/or treatment of AIDS, for example, the followings are experimented: (1) inhibition of multiplication of HIV by administration of reverse transcriptase inhibitors or protease inhibitors, (2) prevention, alleviation, *etc.* of optimistic infectious diseases by administration of immunostimulant drugs

HIV mainly infects on helper T cells which control the immune system center. At the time, it has been known that HIV makes use of membrane protein CD4 expressing on the membrane of T cells since 1985 (*Cell*, 52, 631 (1985)). CD4 molecule consists of 433 amino acid residues and it is expressed in macrophages, some of the B cells, vascular endothelial cells, islet of Langerhans of skin tissue, dendritic cells on lymphatic tissue, glia cells of central nervous system, *etc.* as well as mature helper T cells. However, as it was revealed that HIV infection was not structured only with CD4 molecule, another factor than CD4 molecule was indicated concerning when HIV infects on cells.

In 1996, a cell membrane protein, called Fusin, was identified as a factor which is involved with HIV infection other than CD4 molecule. This Fusin molecule was demonstrated to be a receptor of stromal derived factor-1 (SDF-1), i.e. CXCR4. Moreover, it was also demonstrated that SDF-1 inhibited the T cell oriented (X4) HIV infection specifically *in vitro* (*Nature,* 382, 829 (1996), *Nature,* 382, 833 (1996)). That is, it is conceivable that SDF-1 binds to CXCR4 former than HIV does, thereby it deprives HIV of the toehold of infecting the cells, resulting in inhibiting HIV infection.

Around the same time, it was discovered that another chemokine receptor CCR5, a receptor of RANTES, MIP-1α and MIP-1β, is utilized when macrophage-oriented (R5) HIV infects *(Science,* 272, 1955 (1996)).

Therefore, whatever competes with HIV for CXCR4 or CCR5, or whatever prevents HIV from binding to CXCR4 or CCR5 by binding to HIV, should be used as an HIV infection inhibitor. And as another example, a low molecule compound which was discovered as an HIV infection inhibitor turned out afterward to be a CXCR4 antagonist *(Nature Medicine,* 4, 72 (1998)).

On the other hand, many bipiperidine derivatives are known as CCR5 antagonists (e.g. WO01/77101, WO02/81449). Also, many 1-(4-pyridinyl)piperazine derivatives are also known (e.g. WO00/66558, WO00/66559, WO00/66141, WO02/79157). Further, triazaspiro[5.5]undecane derivatives are also known (e.g. WO01/40227, WO02/74770).

Also, piperazine derivatives are known as CCR1 antagonists (e.g. WO02/36581, WO03/35627).

Disclosure of the Invention

The objective of the present invention is to develop a drug for the prevention and/or treatment of acquired immunodeficiency syndrome, HIV infection, organ rejection in transplantation, multiple sclerosis, rheumatoid arthritis, *etc.* as a CCR1 and/or CCR5 antagonist which is useful as a pharmaceutical drug, and possesses good oral absorbability and is safe.

As a result of energetic investigation in order to find out a compound antagonizing CCR1 and/or CCR5, the present inventors have found that the compound of formula (I) of the present invention achieves the purpose and completed the present invention.

The present invention relates to

[1] a compound of formula (I)

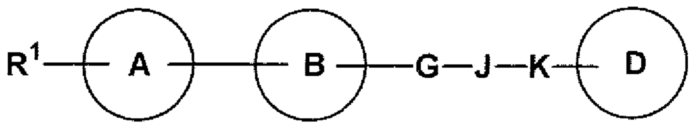

(I)

wherein $R^1$ represents aliphatic hydrocarbon optionally having substituent(s),
ring A represents a cyclic group comprising at least one nitrogen atom optionally having further substituent(s) besides $R^1$,
ring B represents a cyclic group optionally having substituent(s) and is attached to ring A via a bond,
G represents a bond or a spacer comprising 1-4 atoms in the main chain,
J represents a spacer having a hydrogen-bond accepting group optionally having substituent(s),
K represents a bond or a spacer comprising 1-4 atoms in the main chain, and
ring D represents a cyclic group optionally having substituent(s), which may form a ring together with a substituent on J,
a salt thereof, an N-oxide thereof, a solvate thereof or a prodrug thereof,
[2] the compound according to the above 1, wherein the hydrogen-bond accepting group in J is carbonyl, thiocarbonyl, imino, sulfonyl or sulfinyl, a salt thereof, an N-oxide thereof, a solvate thereof or a prodrug thereof,
[3] the compound according to the above 1,
wherein J is -CO-, $-CONR^2-$, $-NR^2CO-$, -OCO-, -COO-, -CS-, $-CSNR^2-$,- $NR^2CS-$, -O-CS-, -CS-O-, $-SO_2-$, $-SO_2NR^2-$, $-NR^2SO_2-$, $-O-SO_2-$, $-SO_2-O-$, -S(O)-, $-S(O)NR^2-$, $-NR^2S(O)-$, -O-S(O)-, -S(O)-O-, or $-C(=NR^3)-$,
wherein $R^2$ represents a hydrogen atom, optionally substituted aliphatic hydrocarbon or an optionally substituted cyclic group and $R^3$ represents a hydrogen atom, cyano, optionally protected hydroxy, optionally substituted amino, optionally substituted aliphatic hydrocarbon or an optionally substituted cyclic group,
a salt thereof, an N-oxide thereof, a solvate thereof or a prodrug thereof,
[4] the compound according to the above 1,
wherein J is $-N(COR^4)-$, $-N(CONHR^5)-$, $-N(COOR^6)-$, or $-N(SO_2R^7)-$,
wherein $R^4$, $R^5$, $R^6$ and $R^7$ each represents a hydrogen atom, optionally substituted aliphatic hydrocarbon or an optionally substituted cyclic group,
a salt thereof, an N-oxide thereof, a solvate thereof or a prodrug thereof,
[5] the compound according to the above 1, wherein the cyclic group represented by ring D is a C3-15 mono-, bi- or tri-cyclic aromatic carbocyclic ring which may be partially or completely saturated, or a 3-15 membered mono-, bi- or tri-cyclic aromatic heterocyclic ring comprising 1-5 of heteroatom selected from oxygen, nitrogen and sulfur which may be partially or completely saturated,
a salt thereof, an N-oxide thereof, a solvate thereof or a prodrug thereof,
[6] the compound according to the above 1, wherein the cyclic group represented by ring D is a C3-15 mono-, bi- or tri-cyclic aromatic carbocyclic ring, or a 3-15 membered mono-, bi- or tri-cyclic aromatic heterocyclic ring containing 1-5 of heteroatom,
a salt thereof, an N-oxide thereof, a solvate thereof or a prodrug thereof,
[7] the compound according to the above 1, wherein
wherein

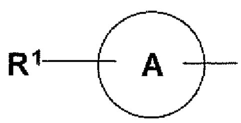

,

wherein all symbols have the same meanings as the above 1, is

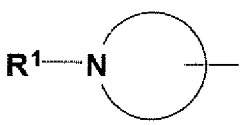

,

wherein

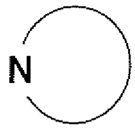

is a cyclic ring comprising at least one nitrogen atom and optionally having substituent(s) and $R^1$ has the same meaning as the above 1,
a salt thereof, an N-oxide thereof, a solvate thereof or a prodrug thereof,
[8] the compound according to the above 7,
wherein

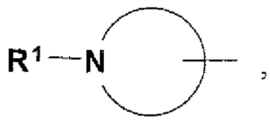

wherein all symbols have the same meanings as the above 1,
is piperidine, piperazine, pyrrolidine, 1,4-diazepane, 1,2,3,6-tetrahydropyridine or 8-azabicyclo[3.2.1]octane ring optionally having substituent(s),
a salt thereof, an N-oxide thereof, a solvate thereof or a prodrug thereof,
[9] a pharmaceutical composition comprising a compound of formula (I)

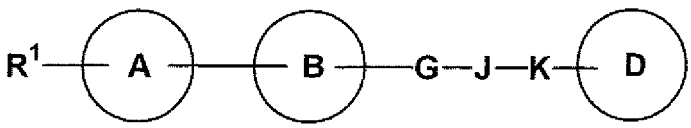

(I)

wherein $R^1$ represents aliphatic hydrocarbon optionally having substituent(s),
ring A represents a cyclic group comprising at least one nitrogen atom optionally having further substituent(s) besides $R^1$,
ring B represents a cyclic group optionally having substituent(s) and is attached to ring A via a bond,
G represents a bond or a spacer comprising 1-4 atoms in the main chain,
J represents a spacer having a hydrogen-bond accepting group optionally having substituent(s),
K represents a bond or a spacer comprising 1-4 atoms in the main chain, and
ring D represents a cyclic group optionally having substituent(s), which may form a ring together with a substituent on J,
a salt thereof, an N-oxide thereof, a solvate thereof or a prodrug thereof,
[10] the composition according to the above 9, which is a chemokine receptor antagonist,
[11] the composition according to the above 10, wherein the chemokine receptor is CCR1,
[12] the composition according to the above 10, wherein the chemokine receptor is CCR5,
[13] the composition according to the above 10, which is a medicament for the prevention and/or treatment of human immunodeficiency virus infectious disease, acquired immunodeficiency syndrome and/or organ rejection in transplantation,
[14] the composition according to the above 10, which is a medicament for the prevention and/or treatment of multiple sclerosis and/or arthritis,
[15] a method for the prevention and/or treatment of diseases induced by a chemokine receptor in a mammal, which comprises administering to an mammal an effective amount of the compound described in the above 1, a salt thereof, an N-oxide thereof, a solvate thereof or a prodrug thereof,
[16] use of the compound described in the above 1, a salt thereof, an N-oxide thereof, a solvate thereof or a prodrug thereof, for the manufacture of a medicament for the prevention and/or treatment of the diseases induced by chemokine receptors,
[17] a medicament comprising the compound described in the above 1, a salt thereof, an N-oxide thereof, a solvate thereof or a prodrug thereof, and one or more selected from the group consisting of a protease inhibitor, a reverse

transcriptase inhibitor, a fusion inhibitor, an HIV integrase inhibitor, a chemokine inhibitor, a steroidal drug, interferon, an immunosuppressant, an aldose reductase inhibitor, a cannabinoid-2 receptor agonist, adrenocorticotropic hormone, a metalloproteinase inhibitor, a non-steroidal anti-inflammatory drug, a prostaglandin drug, a phosphodiesterase inhibitor, a disease modifying anti-rheumatic drug, an anti-inflammatory enzyme drug, a cartilage-protecting drug, a T-cell inhibitor, a TNF-α inhibitor, an IL-6 inhibitor, an interferon γ agonist, an IL-1 inhibitor and an NF-κB inhibitor, and

[18] a method for the preparation thereof

Among the formula (I), in the "aliphatic hydrocarbon optionally having substituent(s)" represented by $R^1$, the "aliphatic hydrocarbon" includes, "straight or branched hydrocarbon", and the "straight or branched hydrocarbon" includes "straight or branched alkyl, alkenyl, or alkynyl".

The "straight or branched alkyl" includes, for example, straight or branched C1-10 alkyl such as methyl, ethyl, propyl, isopropyl, butyl, isobutyl, sec-butyl, t-butyl, pentyl, isopentyl, neopentyl, hexyl, heptyl, octyl, nonyl, decyl, *etc.*

The "straight or branched alkenyl" includes straight or branched alkenyl such as ethenyl, propenyl, butenyl, butadienyl, pentenyl, pentadienyl, hexenyl, hexadienyl, heptenyl, heptadienyl, octenyl, octadienyl, nonenyl, nonadienyl, decenyl, decadienyl, *etc.*

The "straight or branched alkynyl" includes, for example, straight or branched C2-10 alkynyl such as ethynyl, propynyl, butynyl, butadiynyl, pentynyl, pentadiynyl, hexynyl, hexadiynyl, heptynyl, heptadiynyl, octynyl, octadiynyl, nonynyl, nonadiynyl, decynyl, decadiynyl, *etc.*

In the "aliphatic hydrocarbon optionally having substituent(s)", the "substituent" is, (1) optionally substituted cyclic group, (2) optionally protected hydroxy, (3) optionally protected mercapto, (4) optionally substituted amino, *etc.,* and 1-5 of these substituent(s) may be placed where acceptable.

In this "optionally substituted cyclic group", the "cyclic group" includes, for example, a carbocyclic ring or a heterocyclic ring. The "carbocyclic ring" is for example, a partially or completely saturated C3-15 mono-, bi- or tri-cyclic aromatic carbocyclic ring, a spiro bi-cyclic carbocyclic ring, a bridged bi-cyclic carbocyclic ring, *etc.,* e.g. cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indan, naphthalene, dihydronaphthalene, tetrahydronaphthalene, perhydronaphthalene, heptalene, perhydroheptalene, biphenylene, as-indacene, s-indacene, acenaphthylene, acenaphthene, fluorene, phenalene, phenanthrene, anthracene, spiro[4.4]nonane, spiro[4.5]decane, spiro[5.5]undecane, bicyclo[2.2.1]heptane, bicyclo[2.2. 1]hept-2-ene, bicyclo[3.1. 1]heptane, bicyclo[3.1. 1]hept-2-ene, bicyclo[2.2.2]octane, bicyclo[2.2.2]oct-2-ene, adamantane, noradamantane ring, *etc.*

The heterocyclic ring includes, for example, a 3-15 membered mono-, bi- or tri-cyclic aromatic heterocyclic ring comprising 1-5 of heteroatom selected from oxygen, nitrogen and sulfur, *etc.* which may be partially or completely saturated. In the heterocyclic ring, a 3-15 membered mono-, bi- or tri-cyclic aromatic heterocyclic ring comprising 1-5 of heteroatom selected from oxygen, nitrogen and sulfur includes, e.g. pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazane, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolidine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzazepine, benzodiazepine, benzofurazane, benzothiadiazole, benzotriazole, carbazole, β-carboline, acridine, phenazine, dibenzofuran, xanthene, dibenzothiophene, phenothiazine, phenoxazine, phenoxathiine, thianthrene, phenanthridine, phenanthriline, perimidine ring, *etc.*

In the heterocyclic ring, a partially or completely saturated 3-15 membered mono-, bi- or tri-cyclic aromatic heterocyclic ring includes, e.g. aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiilane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazane, tetrahydrofurazane, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, pcrhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinaxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinoxnarpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindan, benzodioxane, chroman, benzodithiolane, benzodithiane ring, *etc*.

In the "optionally substituted cyclic group", the "substituent" is, for example, (a) optionally substituted alkyl, (b) optionally substituted alkenyl, (c) optionally substituted alkynyl, (d) an optionally substituted carbocyclic ring, (e) an optionally substituted heterocyclic ring, (f) optionally protected hydroxy, (g) optionally protected mercapto, (h) optionally substituted amino, (i) optionally substituted carbamoyl, (j) optionally substituted sulfamoyl, (k) carboxy, (1) alkoxycarbonyl (e.g. C1-6 alkoxycarbonyl, *etc*. such as methoxycarbonyl, ethoxycarbonyl, t-butoxycarbonyl *etc*.), (m) sulfo (-$SO_3H$), (n) sulfino, (o) phosphono, (p) nitro, (q) oxo, (r) thioxo, (s) cyano, (t) amidino, (u) imino, (v) -$B(OH)_2$, (w) a halogen atom (e.g. fluorine, chlorine, bromine, iodine, *etc*.), (x) alkylsulfinyl (e.g. C1-6 alkylsulfinyl *etc*. such as methylsulfinyl, ethylsulfinyl, *etc.),* (y) arylsulfinyl (e.g. C6-10 arylsulfinyl *etc*. such as phenylsulfinyl *etc*.), (z) alkylsulfonyl (e.g. C1-6 alkylsulfonyl such as methylsulfonyl, ethylsulfonyl, *etc*.), (aa) arylsulfonyl (e.g. C6-10 arylsulfonyl*etc*. such as phenylsulfonyl *etc*.), (bb) acyl (e.g. C1-6 alkanoyl such as formyl, acetyl, propanoyl, pivaloyl, *etc.,* C6-10 arylcarbonyl such as benzoyl *etc.), etc.,* and 1-5 of these substituent may be placed where acceptable.

In the "optionally substituted alkyl" as a substituent, "alkyl" includes, e.g. straight or branched C1-6 alkyl such as methyl, ethyl, propyl, isopropyl, butyl, isobutyl, sec-butyl, t-butyl, pentyl, hexyl, *etc.* Here, the substituent of alkyl includes, e.g. hydroxy, amino, carboxy, nitro, mono- or di- C1-6 alkylamino (e.g. methylamino, ethylamino, propylamino, dimethylamino, diethylamino, *etc*.), C1-6 alkoxy (e.g. methoxy, ethoxy, propoxy, hexyloxy, *etc*.), C1-6 alkylcarbonyloxy (e.g. acetoxy, ethylcarbonyloxy, *etc*.), a halogen atom (e.g. fluorine, chlorine, bromine, iodine), etc., and 1-4 of these substituents may be placed where acceptable.

In the "optionally substituted alkenyl" as a substitumt, "alkenyl" includes, e.g. straight or branched C2-6 alkenyl such as ethenyl, propenyl, butenyl, butadienyl, pentenyl, pentadienyl, hexenyl, hexadienyl, *etc*. Here, the substituent of alkenyl includes the same ones as described in the above "optionally substituted alkyl".

In the "optionally substituted alkynyl" as a substituent, "alkynyl" includes, e.g. straight or branched C2-6 alkynyl such as ethynyl, propnyl, butynyl, butadiynyl, pentynyl, pentadiynyl, hexynyl, hexadiynyl, *etc*. Here, the substituent of alkynyl includes the same ones as described in the above "optionally substituted alkyl".

The carbocyclic ring in the "optionally substituted carbocyclic ring" as a substituent has the same meaning as the carbocyclic ring in the "cyclic group" in the above "cyclic group optionally having substituent(s)". Here the substituent of the carbocyclic ring includes, e.g. straight or branched C1-6 alkyl (it has the same meaning as alkyl as the above "optionally substituted alkyl" ), staright or branched C2-6 alkenyl (it has the same meaning as alkenyl in the above "optionally substituted alkenyl"), straight or branched C2-6 alkynyl (it has the same meaning as the alkynyl in the above "optionally substituted alkynyl"), hydroxy, C1-6 alkoxy (e.g. methoxy, ethoxy, propoxy, isopropoxy, butoxy, isobutyloxy, t-butoxy, pentyloxy, hexyloxy, *etc*.), mercapto, C1-6 alkylthio (e.g. methylthio, ethylthio, propylthio, isopropylthio, butylthio, isobutylthio, tert-butylthio, pentylthio, hexylthio, *etc*.), amino, mono- or di-C1-6 alkylamino (e.g. methylamino, ethylamino, propylamino, isopropylamino, butylamino, isobutylamino, t-butylamino, pentylamino, hexylamino, dimethylamino, diethylamino, dipropylamino, N-methyl-N-ethylamino, *etc*.), halogen atom (it has the same meaning as hereinbefore), cyano, nitro, *etc*., and 1-5 of these substituents may be placed where acceptable.

The heterocyclic ring in the "optionally substituted heterocyclic ring" as a substituent has the same meaning as the heterocyclic ring in the "cyclic group" in the "heterocyclic ring optionally having substituent(s)". Here the substituent of the heterocyclic ring has the same meaning as the carbocyclic ring optionally having substituent(s) as hereinbefore described. The "optionally substituted carbamoyl" as a substituent includes, for example, unsubstituted carbamoyl, N-mono-C1-6 alkylcarbamoyl (e.g. N-methylcarbamoyl, N-ethylcarbamoyl, N-propylcarbamoyl, N-isopropylcarbamoyl, N-butylcarbamoyl, N-isobutylcarbamoyl, N-(t-butyl)carbamoyl, N-pentylcarbamoyl, N-hexylcarbamoyl, *etc.),* N,N-di-C1-6 alkylcarbamoyl (e.g. N,N-dimethylcarbamoyl, N,N-diethylcarbamayl, N,N-dipropylcarbamoyl, N,N-dibutylcarbamoyl, N,N-dipentylcarbaznoyl, N,N-dihexylcarbamoyl, N-methyl-N-ethylcarbamoyl, *etc.), etc.*

The "optionally substituted sulfamoyl" as a substituent includes, for example, unsubstituted sulfamoyl, N-mono-C1-6 alkylsulfamoyl (e.g. N-methylsulfamoyl, N-ethylsulfamoyl, N-propylsulfamoyl, N-isopropylsulfamoyl, N-butylsulfamoyl, N-isobutylsulfamoyl, N-(t-butyl)sulfamoyl, N-pentylsulfamoyl, N-hexylsulfamoyl, *etc.*), N,N-di-C1-6 alkylsulfamoyl (e.g. N,N-dimethylsulfamoyl, N,N-diethylsulfamoyl, N,N-dipropylsulfamoyl, N,N-dibutylsulfamoyl, N,N-dipentylsulfamoyl, N,N-dihexylsulfamoyl, N-methyl-N-ethylsulfamoyl, *etc.*), *etc.*

Among $R^1$, in the "optionally protected hydroxy", the "optionally protected mercapto" and the "optionally substituted amino" as a "substituent", the "substitutive group" or "protective group" includes, e.g. (i) optionally substituted alkyl (it has the same meaning as hereinbefore.), (ii) an optionally substituted carbocyclic ring (it has the same meaning as hereinbefore.), (iii) an optionally substituted heterocyclic ring (it has the same meaning as hereinbefore.), (iv) acyl (e.g. C1-6 alkanoyl such as formyl, acetyl, propanoyl, pivaloyl, butanoyl, pentanoyl, hexanoyl, *etc.* or isomers thereof etc., C6-10 aromatic carbocyclylcarbonyl such as benzoyl *etc.*), *etc.*

The "cyclic group comprising at least one nitrogen atom" in the "cyclic group comprising at least one nitrogen atom and optionally having substituent(s)" represented by ring A includes, e.g. 3-15 membered mono-, bi- or tri-cyclic aromatic heterocyclic ring comprising at least one nitrogen atom and 0-4 of heteroatom selected from oxygen, nitrogen and sulfur which may be partially or completely saturated *etc.* In the heterocyclic ring, 3-15 membered mono-, bi- or tri-cyclic aromatic heterocyclic ring comprising at least one nitrogen atom and 0-4 of heteroatom selected from oxygen, nitrogen and sulfur includes, e.g. pyrrole, imidazole, triazole, tetrazole, pyrazole, pyridine, pyrazine, pyrimidine, pyridazine, azepine, diazepine, oxazole, isooxazole, thiazole, isothiazole, furazane, oxadiazole, oxazine, oxadiazine, oxazepine, oxadiazepine, thiadiazole, thiazine, thiadiazine, thiazepine, thiadiazepine, indole, isoindole, indolizine, indazole, quinoline, isoquinoline, quinolizine, purine, phthalazine, pteridine, naphthyridine, , quinoxaline, quinazoline, cinnoline, benzooxazole, benzothiazole, benzoimidazole, benzooxazepine, benzooxadiazepine, benzothiazepine, benzothiadiazepine, benzoazepine, benzodiazepine, benzafurazane, benzothiadiazole, benzotriazole, carbazole, β-carboline, acridine, phenazine, phenothiazine, phenoxazine, phenanthridine, phenanthroline, perimidine ring, *etc.* In the heterocyclic ring, a 3-15 membered partially or completely saturated mono-, bi- or tri-cyclic heterocyclic ring comprising at least one nitrogen atom and 0-4 of heteroatom selected from oxygen, nitrogen and sulfur includes, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazin, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydroxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazane, tetrahydrofurazane, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, indoline, isoindoline, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, dihydrobenzoxazepine, tetrahydrobenzoxazepine, dihydrocarbazole, tetrahydrocarbazole, perhydrocarbazole, dihydroacridine, tetrahydroacridine, perhydroacridine ring, *etc.*

In the "cyclic group comprising at least one nitrogen atom and optionally having substituent(s)", the "substituent" has the same meaning as the substituent in the above "cyclic group optionally having substituent(s)".

The "cyclic group optionally having substituent(s)" represented by ring B has the same meaning as the above "cyclic group optionally having substituent(s)".

The "spacer comprising 1-4 atoms in the main chain" represented by G means an interval of 1-4 of atom in succession. Here the "atom in the main chain" will be counted so as to minimize the atom in the main chain.

The "spacer comprising 1-4 atoms in the main chain" represented by G is, for example, C1-4 alkylene optionally having substituent(s) (e.g. methylene optionally having substituent(s), ethylene optionally having substituent(s), propylene optionally having substituent(s), tetramethylene optionally having substituent(s), *etc.*), C2-4 alkenylene optionally having substituent(s) (e.g. vinylene optionally having substituent(s), propenylene optionally having substituent(s), butenylene optionally having substituent(s), *etc.*), C2-4 alkynylene optionally having substituent(s), (e.g. ethynylene optionally having substituent(s), propylene optionally having substituent(s), butynylene optionally having substituent(s) *etc.), etc.* Here the carbon atom in C1-4 alkylene, C2-4 alkenylene and C2-4 alkynylene may be replaced by oxygen atom, sulfur

atom or nitrogen atom optionally having substituent [as a substituent, (i) optionally substituted alkyl (it has the same meaning as hereinbefore.), (ii) optionally substituted carbocyclic ring (it has the same meaning as hereinbefore.), (iii) optionally substituted heterocyclic ring (it has the same meaning as hereinbefore.), (iv) acyl (it has the same meaning as hereinbefore.), *etc*.]. Here the substituents of C1-4 alkylene, C2-4 alkenylene and C2-4 alkynylene include, e.g. C1-4 alkyl (methyl, ethyl, propyl, isopropyl, butyl, isobutyl, sec-butyl, t-butyl, *etc.),* a halogen atom (e.g. fluorine, chlorine, bromine, iodine), hydroxy, amino, *etc.,* and 1-3 of these substituents may be placed where acceptable.

In the "spacer having a hydrogen-bond accepting group optionally having substituent(s)" represented by J, the " hydrogen-bond accepting group" includes a group having an atom comprising unshared electron pair.

The "spacer having a hydrogen-bond accepting group optionally having substituent(s)" includes for example, a group comprising carbonyl which may have substituent(s) (e.g. -CO-, $-CONR^2-$, $-NR^2CO-$, -OCO-, -COO-, $-N(COR^4)-$, $-N(CONHR^5)-$, $-N(COOR^6)-$, *etc.),* a group comprising thiocarbonyl which may have substituent(s) (e.g. -CS-, $-CSNR^2-$, $-NR^2CS-$, -O-CS-, -CS-O-, *etc.),* a group comprising imino which may have substituent(s) (e.g. $-C(=NR^3)-$ *etc*.), a group comprising sulfonyl which may have substituent(s) (e.g. $-SO_2-$, $-SO_2NR^2-$, $-NR^2SO_2-$, $-O-SO_2-$, $-SO_2-O-$, $-N(SO_2R^7)-$, *etc.),* a group comprising sulfinyl which may have substituent(s) (e.g. -S(O)-, $-S(O)NR^2-$, $-NR^2S(O)-$, -O-S(O)-, -S(O)-O-, *etc*.), *etc.*

The "optionally substituted aliphatic hydrocarbon" represented by $R^2$, $R^3$, $R^4$, $R^5$, $R^5$ or $R^7$ has the same meaning as the "optionally substituted aliphatic hydrocarbon" as described hereinbefore. And the "optionally substituted cyclic group" represented by $R^2$, $R^3$, $R^4$, $R^5$, $R^6$ or $R^7$ has the same meaning as the "optionally substituted cyclic group" as described hereinbefore.

The "optionally protected hydroxy" represented by $R^3$ has the same meaning as the "optionally protected hydroxy" as described hereinbefore. The "optionally substituted amino" has the same meaning as the "optionally substituted amino" as described hereinbefore.

The "spacer comprising 1-4 atoms in the main chain" represented by K has the same meaning as the "spacer having 1-4 of atom in the main chain"as described hereinbefore.

The "cyclic group optionally having substituent(s)" as the "cyclic group optionally having substituent(s) which may form a ring together with the substituent of J" represented by ring D has the same meaning as the "cyclic group optionally having substituent(s)" as described hereinbefore.

The "cyclic group optionally having substituent(s) which may form a ring together with the substituent of J" represented by ring D means that the substituent of J and the substituent of ring D may be taken together to form a ring.

In the present invention, any group represented by $R^1$, ring A, ring B, G, J, K and ring D is preferable.

$R^1$ is preferably, C1-6 alkyl which may have substituent(s), C2-6 alkenyl which may have substituent(s) or C2-6 alkynyl which may have substituent(s), etc.; more preferably, C1-6 alkyl, substituted C1-6 alkyl or substituted C2-6 alkenyl, *etc*.; most preferably, optionally substituted methyl, optionally substituted ethyl, optionally substituted propyl, *etc*. Preferable substituent therein is, optionally protected hydroxy, optionally protected mercapto, or an optionally substituted cyclic group, etc.; more preferably, C1-6 alkoxy, C1-6 alkylthio, optionally substituted benzene, optionally substituted thiophene, optionally substituted pyrrole or optionally substituted benzodioxane, etc.; most preferably, methoxy, methylthio, benzene optionally substituted by halogen atom(s), benzene which may be substituted by C1-6 alkoxy or benzene which may be substituted by C1-6 alkyl, *etc*. The number of the substituents is preferably 1-3.

The ring A is preferably, a partially or completely saturated 3-10 membered mono- or bi-cyclic aromatic heterocyclic ring *etc.* comprising at least one nitrogen atom and 0-4 of heteroatom selected from oxygen, nitrogen, sulfur; more preferably a partially or completely saturated 4-8 membered mono-cyclic aromatic heterocyclic ring comprising at least one nitrogen atom and 0-2 of heteroatom selected from oxygen, nitrogen and sulfur; most preferably, piperidine, piperazine, tetrahydropyridine, pyrrolidine or 1,4-diazepane, 1,2,3,6-tetrahydropyridine, 8-azabicyclo[3.2.1]octane ring, *etc.*

The substituent of the ring A is preferably, optionally substituted alkyl, optionally protected hydroxy, or cyano; more preferably, C1-6 alkyl, hydroxy, C1-6 alkoxy or cyano; most preferably, methyl, hydroxy or cyano. The number of the substituents is preferably 1-3.

The ring B is preferably, a C3-15 mono-, bi- or tri-cyclic aromatic carbocyclic ring, a spiro bi-cyclic carbocyclic ring or a bridged bi-cyclic carbocyclic ring which may be partially or completely saturated or a 3-15 membered mono-, bi- or tri-cyclic aromatic heterocyclic ring comprising 1-5 of heteroatom selected from oxygen, nitrogen and sulfur, *etc*. which may be partially or completely saturated; more preferably a C5-10 mono- or bi-cyclic aromatic carbocyclic ring, a spiro bi-cyclic carbocyclic ring and a bridged bi-cyclic carbocyclic ring which may be partially or completely saturated or a 5-10 membered mono- or bi-cyclic heterocyclic ring comprising 1-5 of heteroatom selected from oxygen, nitrogen and sulfur which may be partially or completely saturated, etc.; most preferably, benzene, pyrimidine, pyridine or thiazole ring, *etc.*

G is preferably, C1-4 alkylene which may have substituent(s) (wherein the carbon atom(s) in the C1-4 alkylene may be replaced by oxygen, sulfur, or nitrogen which may have a substituent.), *etc.;* more preferably, C1-2 alkylene (wherein the carbon atom(s) in the C1-2 alkylene may be replaced by oxygen, sulfur or nitrogen which may have a

substituent.), *etc.*; most preferably, methylene, oxygen, sulfur or nitrogen which may have a substituent, *etc.*

J is preferably, a group comprising carbonyl which may have substituent(s) or a group comprising sulfonyl which may have substituent(s), etc.; more preferably, $-CONR^2-$, $-NR^2CO-$, -OCO-, -COO-, $-SO_2NR^2-$, $-NR^2SO_2-$, $-N(COR^4)-$, $-N(CONHR^5)-$, $-N(COOR^6)-$ or $-N(SO_2R^7)-$, *etc.;* most preferably, $-NR^2CO-$, -OCO- or $-NR^2SO_2-$, *etc.*

K is preferably, a bond or C1-4 alkylene (wherein the carbon atom(s) in the C1-4 alkylene may be replaced by oxygen, sulfur or nitrogen which may have a substituent.), etc.; more preferably, a bond or C1-3 alkylene (wherein the carbon atom(s) in the C1-3 alkylene may be replaced by oxygen, sulfur or nitrogen which may have substituent(s).), etc.; most preferably, a bond, methylene, $-CH_2-O-$, $-O-CH_2-$, $-CH_2-O-CH_2-$, $-CH_2-S-$, $-S-CH_2-$ or $-CH_2-S-CH_2-$, *etc.*

The ring D is preferably, a C3-15 mono-, bi- or tri-cyclic aromatic carbocyclic ring or a 3-15 membered mono-, bi- or tri-cyclic aromatic heterocyclic ring comprising 1-5 of heteroatom selected from oxygen, nitrogen and sulfur *etc.*; more preferably a C5-10 mono- or bi-cyclic aromatic carbocyclic ring or a 5-10 membered mono- or bi-cyclic aromatic heterocyclic ring comprising 1-5 of heteroatom selected from oxygen, nitrogen and sulfur; most preferably, benzene, thiophene, imidazole, quinoline, furan, benzofuran, pyridine, or pyrimidine ring, *etc.*

The substituent of the ring D is preferably, optionally substituted alkyl, optionally protected hydroxy, optionally protected mercapto, optionally substituted amino, a halogen atom, cyano, *etc.*; more preferably, C1-6 alkyl, trifluoromethyl, C1-6 alkoxy, C1-6 alkylthio, hydroxy, a halogen atom, *etc.;* most preferably methoxy, methylthio, trifluoromethyl, chlorine, fluorine, hydroxy, *etc.* The number of the substituent is preferably 1-3.

In the present invention, the preferable is the compound of formula (I) having the combinations of the preferable groups, preferable rings and preferable atoms as described above. Particularly preferable are, for example, the compound of

formula (I-A)

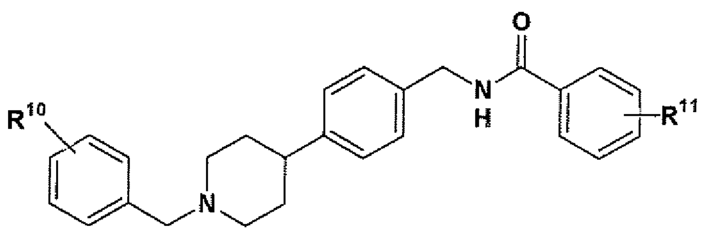

(I-A)

,

wherein $R^{10}$ and $R^{11}$ have each independently, the same meanings as the "substituent" in the "optionally substituted cyclic group",

formula (I-A-1)

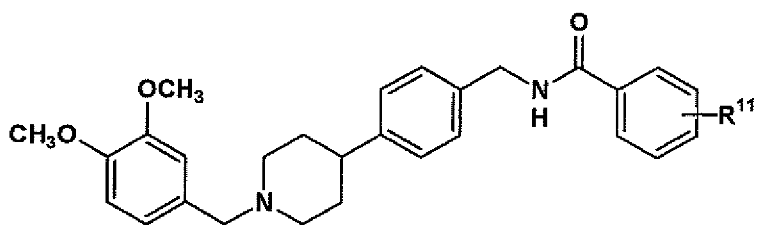

(I-A-1)

,

wherein $R^{11}$ has the same meaning as hereinbefore,

formula (I-A-2)

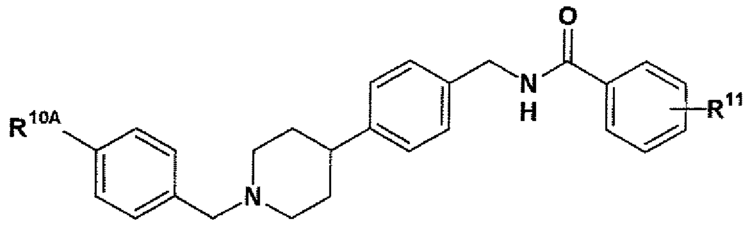 (I-A-2)

,

wherein $R^{10A}$ is a chlorine atom or a fluorine atom, and $R^{11}$ has the same meaning as hereinbefore,

formula (I-B)

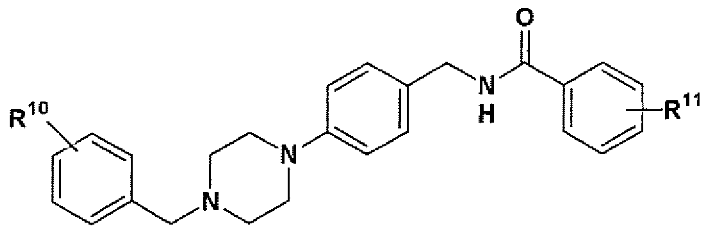 (I-B)

,

wherein all symbols have the same meanings as hereinbefore,

formula (I-B-1)

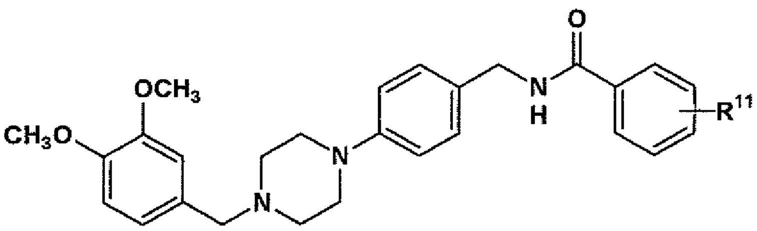 (I-B-1)

,

wherein $R^{11}$ has the same meaning as hereinbefore,

formula (I-B-2)

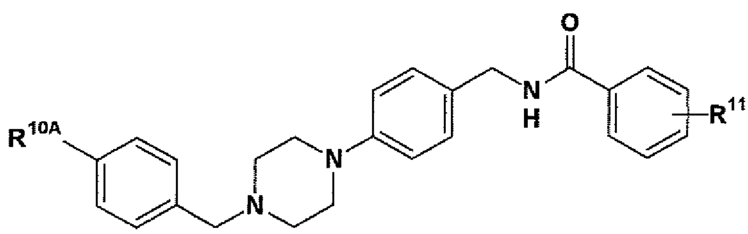 (I-B-2)

,

wherein all symbols have the same meanings as hereinbefore,

formula (I-C)

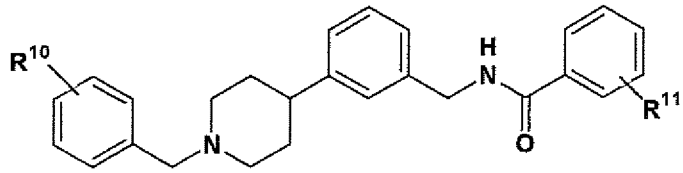

(I-C)

,

wherein all symbols have the same meanings as hereinbefore,

formula (I-C-1)

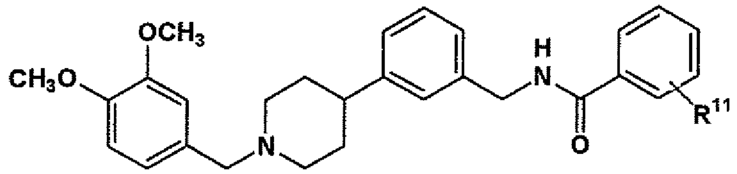

(I-C-1)

,

wherein $R^{11}$ has the same meaning as hereinbefore,

formula (I-C-2)

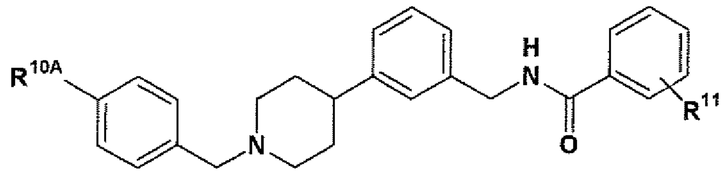

(I-C-2)

,

wherein all symbols have the same meanings as hereinbefore,

formula (I-D)

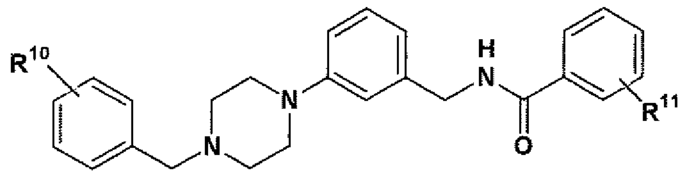

(I-D)

,

*wherein* all symbols have the same meanings as hereinbefore,

formula (I-D-1)

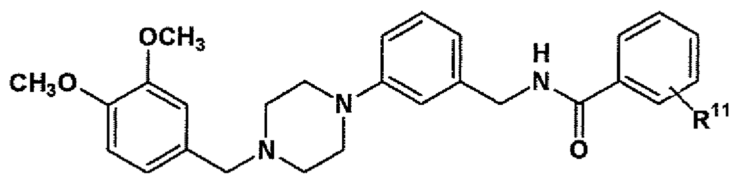

(I-D-1)

,

*wherein* $R^{11}$ has the same meaning as hereinbefore,

formula (I-D-2)

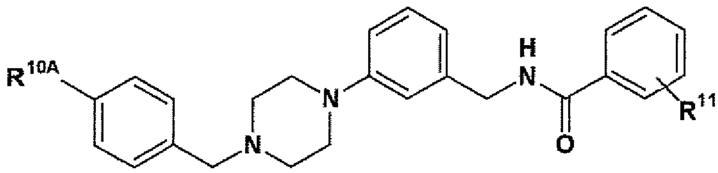

**(I-D-2)**

,

wherein all symbols have the same meanings as hereinbefore,

formula (I-E)

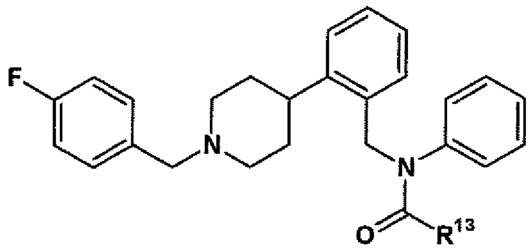

**(I-E)**

,

wherein $R^{13}$ is $-R^4$, $-NHR^5$, or $-OR^6$, and $R^4$, $R^5$ and $R^6$ have the same meanings as hereinbefore,

formula (I-E-1)

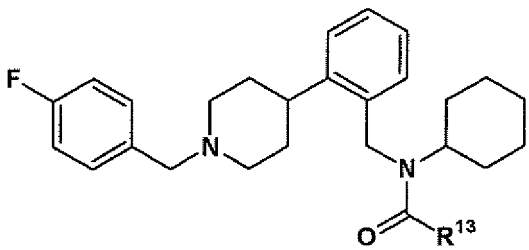

**(I-E-1)**

,

wherein $R^{13}$ has the same meaning as hereinbefore,

formula (I-E-2)

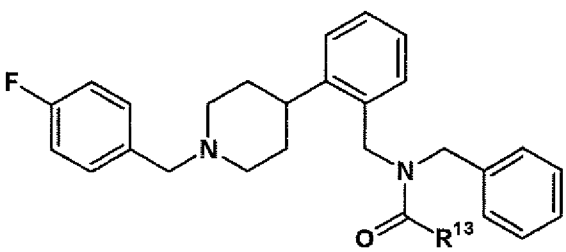

**(I-E-2)**

,

wherein $R^{13}$ has the same meaning as hereinbefore,

formula (I-E-3)

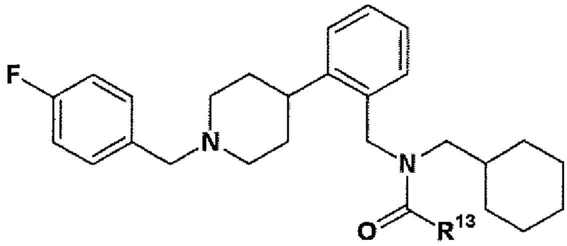

(I-E-3)

,

wherein $R^{13}$ has the same meaning as hereinbefore, *etc.*

Concretely preferable compounds in the present invention are, for example, the following compounds of (1) to (188), the compounds described in the examples, salts thereof, N-oxides thereof, solvates thereof or prodrugs thereof, *etc.*

(1) 4-chloro-N-({2-[1-(3,4-dimethoxybenzyl)piperidin-4-yl]pyridin-3-yl}methyl)benzamide,
(2) 3-chloro-N-({2-[1-(3,4-dimethoxybenzyl)piperidin-4-yl]pyridin-3-yl}methyl)-2-methylbenzamide,
(3) 4-chloro-N-({2-[1-(4-fluorobenzyl)piperidin-4-yl]pyridin-3-yl}methyl)benzamide,
(4) 3 -chloro-N-({2-[1-(4-fluorobenzyl)piperidin-4-yl]pyridin-3-yl}methyl)-2-methylbenzamide,
(5) 4-chloro-N-({2-[1-(3,4-dimethoxybenzyl)piperidin-4-yl]pyridin-4-yl}methyl)benzamide,
(6) 3 -chloro-N-({2-[1-(3,4-dimethoxybenzyl)piperidin-4-yl]pyridin-4-yl}methyl)-2-methylbenzamide,
(7) 4-chloro-N-({2-[1-(4-fluorobenzyl)piperidin-4-yl]pyridin-4-y1}methyl)benzamide,
(8) 3-chloro-N-({2-[1-(4-fluorobenzyl)piperidin-4-yl]pyridin-4-yl}methyl)-2-methylbenzamide,
(9) 4-chloro-N-({4-[1-(3,4-dimethoxybenzyl)piperidin-4-yl]-1,3-thiazol-5-yl}methyl)benzamide,
(10) 3-chloro-N-({4-[1-(3,4-dimethoxybenzyl)piperidin-4-yl]-1,3-thiazol-5-yl} methyl)-2-methylbenzamide,
(11) 4-chloro-N-({4-[1-(4-fluorobenzyl)piperidin-4-yl]-1,3-thiazol-5-yl} methyl)benzamide,
(12) 3-chloro-N-({4-[1-(4-fluorobenzyl)piperidin-4-yl]-1,3-thiazol-5-yl}methyl)-2-methylbenzamide,
(13) 4-chloro-N-({2-[1-(3,4-dimethoxybenzyl)piperidin-4-yl]-1,3-thiazol-5-yl}methyl)benzamide,
(14) 3-chloro-N-({2-[1-(3,4-dimethoxybenzyl)piperidin-4-yl]-1,3-thiazol-5-yl}methyl)-2-methylbenzamide,
(15) 4-chloro-N-({2-[1-(4-fluorobenzyl)piperidin-4-yl]-1,3-thiazol-5-yl}methyl)benzamide,
(16) 3-chloro-N-({2-[1-(4-fluorobenzyl)piperidin-4-yl]-1,3-thiazol-5-yl}methyl)-2-methylbenzamide,
(17) 4-chloro-N-({2-[4-(3,4-dimethoxybenzyl)piperazin-1-yl]pyridin-3-yl}methyl)benzamide,
(18) 3-chloro-N-({2-[4-(3,4-dimethoxybenzyl)piperazin-1-yl]pyridin-3-yl}methyl)-2-methylbenzamide,
(19) 4-chloro-N-({2-[4-(4-fluorobenzyl)piperazin-1-yl]pyridin-3-yl}methyl)benzamide,
(20) 3-chloro-N-({2-[4-(4-fluorobenzyl)piperazin-1-yl]pyridin-3-yl}methyl)-2-methylbenzamide,
(21) 4-chloro-N-({2-[4-(3,4-dimethoxybenzyl)piperazin-1-yl]pyridin-4-yl}methyl)benzamide,
(22) 3-chloro-N-({2-[4-(3,4-dimethoxybenzyl)piperazin-1-yl]pyridin-4-yl}methyl)-2-methylbenzamide,
(23) 4-chloro-N-({2-[4-(4-fluorobenzyl)piperazin-1-yl]pyridin-4-yl}methyl)benzamide,
(24) 3-chloro-N-({2-[4-(4-fluorobenzyl)piperazin-1-yl]pyridin-4-yl}methyl)-2-methylbenzamide,
(25) 4-chloro-N-({4-[4-(3,4-dimethoxybenzyl)piperazin-1-yl]-1,3-thiazol-5-yl}methyl)benzamide,
(26) 3-chloro-N-({4-[4-(3,4-dimethoxybenzyl)piperazin-1-yl]-1,3-thiazol-5-yl }methyl)-2-methylbenzamide,
(27) 4-chloro-N-({4-[4-(4-fluorobenzyl)piperazin-1-yl]-1,3-thiazol-5-yl}methyl)benzamide,
(28) 3-chloro-N-({4-[4-(4-fluorobenzyl)piperazin-1-yl]-1,3-thiazol-5-yl}methyl)-2-methylbenzamide,
(29) 4-chloro-N-({2-[4-(3,4-dimethoxybenzyl)piperazin-1-yl]-1,3-thiazol-5-yl}methyl)benzamide,
(30) 3-chloro-N-({2-[4-(3,4-dimethoxybenzyl)piperazin-1-yl]-1,3-thiazol-5-yl}methyl)-2-methylbenzamide,
(31) 4-chloro-N-({2-[4-(4-fluorobenzyl)piperazin-1-yl]-1,3-thiazol-5-yl}methyl)benzamide,
(32) 3-chloro-N-({2-[4-(4-fluorobenzyl)piperazin-1-yl]-1,3-thiazol-5-yl}methyl)-2-methylbenzamide,
(33) 4-chloro-N-{2-[4-(3,4-dimethoxybenzyl)-2-methylpiperazin-1-yl]benzyl}benzamide,
(34) 3-chloro-N-{2-[4-(3,4-dimethoxybenzyl)-2-methylpiperazin-1-yl]benzyl}-2-methylbenzamide,
(35) 4-chloro-N-{2-[4-(4-fluorobenzyl)-2-methylpiperazin-1-yl]benzyl}benzamide,
(36) 3-chloro-N-{2-[4-(4-fluorobenzyl)-2-methylpiperazin-1-yl]benzyl}-2-methylbenzamide,
(37) 4-chloro-N-{3-[4-(3,4-dimethoxybenzyl)-2-methylpiperazin-1-yl]benzyl}benzamide,
(38) 3-chloro-N-{3-[4-(3,4-dimethoxybenzyl)-2-methylpiperazin-1-yl]benzyl}-2-methylbenzamide,
(39) 4-chloro-N-{3-[4-(4-fluorobenzyl)-2-methylpiperazin-1-yl]benzyl}benzamide,
(40) 3-chloro-N-{3-[4-(4-fluorobenzyl)-2-methylpiperazin-1-yl]benzyl}-2-methylbenzamide,
(41) 4-chloro-N-{4-[4-(3,4-dimethoxybenzyl)-2-methylpiperazin-1-yl]benzyl}benzamide,

(42) 3-chloro-N-{4-[4-(3,4-dimethoxybenzyl)-2-methylpiperazin-1-yl]benzyl}-2-methylbenzamide,
(43) 4-chloro-N-{4-[4-(4-fluorobenzyl)-2-methylpiperazin-1-yl]benzyl}benzamide,
(44) 3-chloro-N-{4-[4-(4-fluorobenzyl)-2-methylpiperazin-1-yl]benzyl}-2-methylbenzamide,
(45) 4-chloro-N-({2-[4-(3,4-dimethoxybenzyl)-2-methylpiperazin-1-yl]pyridin-3-yl}methyl)benzamide,
(46) 3-chloro-N-({2-[4-(3,4-dimethoxybenzyl)-2-methylpiperazin-1-yl]pyridin-3-yl}methyl)-2-methylbenzamide,
(47) 4-chloro-N-({2-[4-(4-fluorobenzyl)-2-methylpiperazin-1-yl]pyridin-3-yl}methyl)benzamide,
(48) 3-chloro-N-({2-[4-(4-fluorobenzyl)-2-methylpiperazin-1-yl]pyridin-3-yl}methyl)-2-methylbenzamide,
(49) 4-chloro-N-({2-[4-(3,4-dimethoxybenzyl)-2-methylpiperazin-1-yl]pyridin-4-yl}methyl)benzamide,
(50) 3-chloro-N-({2-[4-(3,4-dimethoxybenzyl)-2-methylpiperazin-1-yl]pyridin-4-yl}methyl)-2-methylbenzamide,
(51) 4-chloro-N-({2-[4-(4-fluorobenzyl)-2-methylpiperazin-1-yl]pyridin-4-yl}methyl)benzamide,
(52) 3-chloro-N-({2-[4-(4-fluorobenzyl)-2-methylpiperazin-1-yl]pyridin-4-yl}methyl)-2-methylbenzamide,
(53) 4-chloro-N-({4-[4-(3,4-dimethoxybenzyl)-2-methylpiperazin-1-yl]-1,3-thiazol-5-yl}methyl)benzamide,
(54) 3-chloro-N-({4-[4-(3,4-dimethoxybenzyl)-2-methylpiperazin-1-yl]-1,3-thiazol-5-yl}methyl)-2-methylbenzamide,
(55) 4-chloro-N-({4-[4-(4-fluorobenzyl)-2-methylpiperazin-1-yl]-1,3-thiazol-5-yl}methyl)benzamide,
(56) 3-chloro-N-({4-[4-(4-fluorobenzyl)-2-methylpiperazin-1-yl]-1,3-thiazol-5-yl}methyl)-2-methylbenzamide,
(57) 4-chloro-N-({2-[4-(3,4-dimethoxybenzyl)-2-methylpiperazin-1-yl]-1,3-thiazol-5-yl}methyl)benzamide,
(58) 3-chloro-N-({2-[4-(3,4-dimethoxybenzyl)-2-methylpiperazin-1-yl]-1,3-thiazol-5-yl}methyl)-2-methylbenzamide,
(59) 4-chloro-N-({2-[4-(4-fluorobenzyl)-2-methylpiperazin-1-yl]-1,3-thiazol-5-yl}methyl)benzamide,
(60) 3-chloro-N-({2-[4-(4-fluorobenzyl)-2-methylpiperazin-1-yl]-1,3-thiazol-5-yl}methyl)-2-methylbenzamide,
(61) 4-chloro-N-{2-[4-(3,4-dimethoxybenzyl)-3-methylpiperazin-1-yl]benzyl}benzamide,
(62) 3-chloro-N-{2-[4-(3,4-dimethoxybenzyl)-3-methylpiperazin-1-yl]benzyl}-2-methylbenzamide,
(63) 4-chloro-N-{2-[4-(4-fluorobenzyl)-3-methylpiperazin-1-yl]benzyl}benzamide,
(64) 3-chloro-N-{2-[4-(4-fluorobenzyl)-3-methylpiperazin-1-yl]benzyl}-2-methylbenzamide,
(65) 4-chloro-N-{3-[4-(3,4-dimethoxybenzyl)-3-methylpiperazin-1-yl]benzyl}benzamide,
(66) 3-chloro-N-{3-[4-(3,4-dimethoxybenzyl)-3-methylpiperazin-1-yl]benzyl}-2-methylbenzamide,
(67) 4-chloro-N-{3-[4-(4-fluorobenzyl)-3-methylpiperazin-1-yl]benzyl}benzamide,
(68) 3-chloro-N-{3-[4-(4-fluorobenzyl)-3-methylpiperazin-1-yl]benzyl}-2-methylbenzamide,
(69) 4-chloro-N-{4-[4-(3,4-dimethoxybenzyl)-3-methylpiperazin-1-yl]benzyl}benzamide,
(70) 3-chloro-N-{4-[4-(3,4-dimethoxybenzyl)-3-methylpiperazin-1-yl]benzyl}-2-methylbenzamide,
(71) 4-chloro-N-{4-[4-(4-fluorobenzyl)-3-methylpiperazin-1-yl]benzyl}benzamide,
(72) 3-chloro-N-{4-[4-(4-fluorobenzyl)-3-methylpiperazin-1-yl]benzyl}-2-methylbenzamide,
(73) 4-chloro-N-({2-[4-(3,4-dimethoxybenzyl)-3-methylpiperazin-1-yl]pyridin-3-yl}methyl)benzamide,
(74) 3-chloro-N-({2-[4-(3,4-dimethoxybenzyl)-3-methylpiperazin-1-yl]pyridin-3-yl}methyl)-2-methylbenzamide,
(75) 4-chloro-N-({2-[4-(4-fluorobenzyl)-3-methylpiperazin-1-yl]pyridin-3-yl}methyl)benzamide,
(76) 3-chloro-N-({2-[4-(4-fluorobenzyl)-3-methylpiperazin-1-yl]pyridin-3-yl}methyl)-2-methylbenzamide,
(77) 4-chloro-N-({2-[4-(3,4-dimethoxybenzyl)-3-methylpiperazin-1-yl]pyridin-4-yl }methyl)benzamide,
(78) 3-chloro-N-({2-[4-(3,4-dimethoxybenzyl)-3-methylpiperazin-1-yl]pyridin-4-yl}methyl)-2-methylbenzamide,
(79) 4-chloro-N-({2-[4-(4-fluorobenzyl)-3-methylpiperazin-1-yl]pyridin-4-yl}methyl)benzamide,
(80) 3-chloro-N-({2-[4-(4-fluorobenzyl)-3-methylpiperazin-1-yl]pyridin-4-yl}methyl)-2-methylbenzamide,
(81) 4-chloro-N-({4-[4-(3,4-dimethoxybenzyl)-3-methylpiperazin-1-yl]-1,3-thiazol-5-yl}methyl)benzamide,
(82) 3-chloro-N-({4-[4-(3,4-dimethoxybenzyl)-3-methylpiperazin-1-yl]-1,3-thiazol-5-yl}methyl)-2-methylbenzamide,
(83) 4-chloro-N-({4-[4-(4-fluorobenzyl)-3-methylpiperazin-1-yl]-1,3-thiazol-5-yl}methyl)benzamide,
(84) 3-chloro-N-({4-[4-(4-fluorobenzyl)-3-methylpiperazin-1-yl]-1,3-thiazol-5-yl}methyl)-2-methylbenzamide,
(85) 4-chloro-N-({2-[4-(3,4-dimethoxybenzyl)-3-methylpiperazin-1-yl]-1,3-thiazol-5-yl}methyl)benzamide,
(86) 3-chloro-N-({2-[4-(3,4-dimethoxybenzyl)-3-methylpiperazin-1-yl]-1,3-thiazol-5-yl}methyl)-2-methylbenzamide,
(87) 4-chloro-N-({2-[4-(4-fluorobenzyl)-3-methylpiperazin-1-yl]-1,3-thiazol-5-yl}methyl)benzamide,
(88) 3-chloro-N-({2-[4-(4-fluorobenzyl)-3-methylpiperazin-1-yl]-1,3-thiazol-5-yl }methyl)-2-methylbenzamide,
(89) 4-chloro-N-({2-[1-(3,4-dimethoxybenzyl)-4-hydroxypiperidin-4-yl]pyridin-3-yl }methyl)benzamide,
(90) 3-chloro-N-({2-[1-(3,4-dimethoxybenzyl)-4-hydroxypiperidin-4-yl]pyridin-3-yl}methyl)-2-methylbenzamide,
(91) 4-chloro-N-({2-[1-(4-fluorobenzyl)-4-hydroxypiperidin-4-yl]pyridin-3-yl}methyl)benzamide,
(92) 3-chioro-N-({2-[1-(4-fluorobenzyl)-4-hydroxypiperidin-4-yl]pyridin-3-yl}methyl)-2-methylbenzamide,
(93) 4-chloro-N-({2-[1-(3,4-dimethoxybenzyl)-4-hydroxypiperidin-4-yl]pyridin-4-yl}methyl)benzamide,
(94) 3-chloro-N-({2-[1-(3,4-dimethoxybenzyl)-4-hydroxypiperidin-4-yl]pyridin-4-yl}methyl)-2-methylbenzamide,
(95) 4-chloro-N-({2-[1-(4-fluorobenzyl)-4-hydroxypiperidin-4-yl]pyridin-4-yl}methyl)benzamide,
(96) 3-chloro-N-({2-[1-(4-fluorobenzyl)-4-hydroxypiperidin-4-yl]pyridin-4-yl}methyl)-2-methylbenzamide,
(97) 4-chloro-N-({4-[1-(3,4-dimethoxybenzyl)-4-hydroxypiperidin-4-yl]-1,3-thiazol-5-yl}methyl)benzamide,
(98) 3-chloro-N-({4-[1-(3,4-dimethoxybenzyl)-4-hydroxypiperidin-4-yl]-1,3-thiazol-5-yl}methyl)-2-methylbenzamide,
(99) 4-chloro-N-({4-[1-(4-fluorobenzyl)-4-hydroxypiperidin-4-yl]-1,3-thiazol-5-yl}methyl)benzamide,

(100) 3-chloro-N-({4-[1-(4-fluorobenzyl)-4-hydroxypiperidin-4-yl]-1,3-thiazol-5-yl}methyl)-2-methylbenzamide,
(101) 4-chloro-N-({2-[1-(3,4-dimethoxybenzyl)-4-hydroxypiperidin-4-yl]-1,3-thiazol-5-yl}methyl)benzamide,
(102) 3-chloro-N-({2-[1-(3,4-dimethoxybenzyl)-4-hydroxypiperidin-4-yl]-1,3-thiazol-5-yl}methyl)-2-methylbenzamide,
(103) 4-chloro-N-({2-[1-(4-fluorobenzyl)-4-hydroxypiperidin-4-yl]-1,3-thiazol-5-yl}methyl)benzamide,
(104) 3-chloro-N-({2-[1-(4-fluorobenzyl)-4-hydroxypiperidin-4-yl]-1,3-thiazol-5-yl} methyl)-2-methylbenzamide,
(105) 4-chloro-N-{2-[4-cyano-1-(3,4-dimethoxybenzyl)piperidin-4-yl]benzyl}benzamide,
(106) 3-chloro-N-{2-[4-cyano-1-(3,4-dimethoxybenzyl)piperidin-4-yl]benzyl}-2-methylbenzamide,
(107) 4-chloro-N-{2-[4-cyano-1-(4-fluorobenzyl)piperidin-4-yl]benzyl}benzamide,
(108) 3 -chloro-N-{2-[4-cyano-1-(4-fluorobenzyl)piperidin-4-yl]benzyl}-2-methylbenzamide,
(109) 4-chloro-N-{3-[4-cyano-1-(3,4-dimethoxybenzyl)piperidin-4-yl]benzyl}benzamide,
(110) 3-chloro-N-{3-[4-cyano-1-(3,4-dimethoxybenzyl)piperidin-4-yl]benzyl}-2-methylbenzamide,
(111) 4-chloro-N-{3-[4-cyano-1-(4-fluorobenzyl)piperidin-4-yl]benzyl}benzamide,
(112) 3-chloro-N-{3-[4-cyano-1-(4-fluorobenzyl)piperidin-4-yl]benzyl}-2-methylbenzamide,
(113) 4-chloro-N-{4-[4-cyano-1-(3,4-dimethoxybenzyl)piperidin-4-yl]benzyl}benzamide,
(114) 3-chloro-N-{4-[4-cyano-1-(3,4-dimethoxybenzyl)piperidin-4-yl]benzyl}-2-methylbenzamide,
(115) 4-chloro-N-{4-[4-cyano-1-(4-fluorobenzyl)piperidin-4-yl]benzyl}benzamide,
(116) 3-chloro-N-{4-[4-cyano-1-(4-fluorobenzyl)piperidin-4-yl]benzyl}-2-methylbenzamide,
(117) 4-chloro-N-({2-[4-cyano-1-(3,4-dimethoxybenzyl)piperidin-4-yl]pyridin-3-yl}methyl)benzamide,
(118) 3-chloro-N-({2-[4-cyano-1-(3,4-dimethoxybenzyl)piperidin-4-yl]pyridin-3-yl }methyl)-2-methylbenzamide,
(119) 4-chloro-N-({2-[4-cyano-1-(4-fluorobenzyl)piperidin-4-yl]pyridin-3-yl} methyl)benzamide,
(120) 3-chloro-N-({2-[4-cyano-1-(4-fluorobenzyl)piperidin-4-yl]pyridin-3-yl}methyl)-2-methylbenzamide,
(121) 4-chloro-N-({2-[4-cyano-1-(3,4-dimethoxybenzyl)piperidin-4-yl]pyridin-4-yl} nnethyl)benzamide,
(122) 3-chloro-N-({2-[4-cyano-1-(3,4-dimethoxybenzyl)piperidin-4-yl]pyridin-4-yl}methyl)-2-methylbenzamide,
(123) 4-chloro-N-({2-[4-cyano-1-(4-fluorobenzyl)piperidin-4-yl]pyridin-4-yl} methyl)benzamide,
(124) 3-chloro-N-({2-[4-cyano-1-(4-fluorobenzyl)piperidin-4-yl]pyridin-4-yl}methyl)-2-methylbenzamide,
(125) 4-chloro-N-({4-[4-cyano-1-(3,4-dimethoxybenzyl)piperidin-4-yl]-1,3-thiazol-5-yl}methyl)benzamide,
(126) 3-chloro-N-({4-[4-cyano-1-(3,4-dimethoxybenzyl)piperidin-4-yl]-1,3-thiazol-5-yl}methyl)-2-methylbenzamide,
(127) 4-chloro-N-({4-[4-cyano-1-(4-fluorobenzyl)piperidin-4-yl]-1,3-thiazol-5-yl}methyl)benzamide,
(128) 3-chloro-N-({4-[4-cyano-1-(4-fluorobenzyl)piperidin-4-yl]-1,3-thiazol-5-yl}methyl)-2-methylbenzamide,
(129) 4-chloro-N-({2-[4-cyano-1-(3,4-dimethoxybenzyl)piperidin-4-yl]-1,3-thiazol-5-yl}methyl)benzamide,
(130) 3-chloro-N-({2-[4-cyano-1-(3,4-dimethoxybenzyl)piperidin-4-yl]-1,3-thiazol-5-yl}methyl)-2-methylbenzamide,
(131) 4-chloro-N-({2-[4-cyano-1-(4-fluorobenzyl)piperidin-4-yl]-1,3-thiazol-5-yl}methyl)benzamide,
(132) 3-chloro-N-({2-[4-cyano-1-(4-fluorobenzyl)piperidin-4-yl]-1,3-thiazol-5-yl}methyl)-2-methylbenzamide,
(133) 4-chloro-N-{2-[1-(3,4-dimethoxybenzyl)pyrrolidin-3-yl]benzyl}benzamide,
(134) 3-chloro-N-{2-[1-(3,4-dimethoxybenzyl)pyrrolidin-3-yl]benzyl}-2-methylbenzamide,
(135) 4-chloro-N-{2-[1-(4-fluorobenzyl)pyrrolidin-3-yl]benzyl}benzamide,
(136) 3-chloro-N-{2-[1-(4-fluorobenzyl)pyrrolidin-3-yl]benzyl}-2-methylbenzamide,
(137) 4-chloro-N- {3-[1-(3,4-dimethoxybenzyl)pyrrolidin-3-yl]benzyl}benzamide,
(138) 3-chloro-N-{3-[1-(3,4-dimethoxybenzyl)pyrrolidin-3-yl]benzyl}-2-methylbenzamide,
(139) 4-chloro-N-{3-[1-(4-fluorobenzyl)pyrrolidin-3-yl]benzyl}benzamide,
(140) 3 -chloro-N-{3-[1-(4-fluorobenzyl)pyrrolidin-3-yl]benzyl}-2-methylbenzamide,
(141) 4-chloro-N- {4-[1-(3,4-dimethoxybenzyl)pyrrolidin-3-yl]benzyl}benzamide,
(142) 3-chloro-N-{4-[1-(3,4-dimethoxybenzyl)pyrrolidin-3-yl]benzyl}-2-methylbenzamide,
(143) 4-chloro-N-{4-[1-(4-fluorobenzyl)pyrrolidin-3-yl]benzyl}benzamide,
(144) 3-chloro-N-{4-[1-(4-fluorobenzyl)pyrrolidin-3-yl]benzyl}-2-methylbenzamide,
(145) 4-chloro-N-({2-[1-(3,4-dimethoxybenzyl)pyrrolidin-3-yl]pyridin-3-yl}methyl)benzamide,
(146) 3-chloro-N-({2-[1-(3,4-dimethoxybenzyl)pyrrolidin-3-yl]pyridin-3-yl}methyl)-2-methylbenzamide,
(147) 4-chloro-N-({2-[1-(4-fluorobenzyl)pyrrolidin-3-yl]pyridin-3-yl} methyl)benzamide,
(148) 3-chloro-N-({2-[1-(4-fluorobenzyl)pyrrolidin-3-yl]pyridin-3-yl}methyl)-2-methylbenzamide,
(149) 4-chloro-N-({2-[1-(3,4-dimethoxybenzyl)pyrrolidin-3-yl]pyridin-4-yl}methyl)benzamide,
(150) 3-chloro-N-({2-[1-(3,4-dimethoxybenzyl)pyrrolidin-3-yl]pyridin-4-yl}methyl)-2-methylbenzamide,
(151) 4-chloro-N-({2-[1-(4-fluorobenzyl)pyrrolidin-3-yl]pyridin-4-yl}methyl)benzamide,
(152) 3-chloro-N-({2-[1-(4-fluorobenzyl)pyrrolidin-3-yl]pyridin-4-yl}methyl)-2-methylbenzamide,
(153) 4-chloro-N-({4-[1-(3,4-dimethoxybenzyl)pyrrolidin-3-yl]-1,3-thiazol-5-yl}methyl)benzamide,
(154) 3-chloro-N-({4-[1-(3,4-dimethoxybenzyl)pyrrolidin-3-yl]-1,3-thiazol-5-yl}methyl)-2-methylbenzamide,
(155) 4-chloro-N-({4-[1-(4-fluorobenzyl)pyrrolidin-3-yl]-1,3-thiazol-5-yl}methyl)benzamide,
(156) 3-chloro-N-({4-[1-(4-fluorobenzyl)pyrrolidin-3-yl]-1,3-thiazol-5-yl}methyl)-2-methylbenzamide,

(157) 4-chloro-N-({2-[1-(3,4-dimethoxybenzyl)pyrrolidin-3-yl]-1,3-thiazol-5-yl}methyl)benzamide,
(158) 3-chloro-N-({2-[1-(3,4-dimethoxybenzyl)pyrrolidin-3-yl]-1,3-thiazol-5-yl}methyl)-2-methylbenzamide,
(159) 4-chloro-N-({2-[1-(4-fluorobenzyl)pyrrolidin-3-yl]-1,3-thiazol-5-yl}methyl)benzamide,
(160) 3-chloro-N-({2-[1-(4-fluorobenzyl)pyrrolidin-3-yl]-1,3-thiazol-5-yl}methyl)-2-methylbenzamide,
(161) 4-chloro-N-{2-[4-(3,4-dimethoxybenzyl)-1,4-diazepan-1-yl]benzyl}benzamide,
(162) 3-chloro-N-{2-[4-(3,4-dimethoxybenzyl)-1,4-diazepan-1-yl]benzyl}-2-methylbenzamide,
(163) 4-chloro-N-{2-[4-(4-fluorobenzyl)-1,4-diazepan-1-yl]benzyl}benzamide,
(164) 3-chloro-N-{2-[4-(4-fluorobenzyl)-1,4-diazepan-1-yl]benzyl}-2-methylbenzamide,
(165) 4-chloro-N-{3-[4-(3,4-dimethoxybenzyl)-1,4-diazepan-1-yl]benzyl}benzamide,
(166) 3-chloro-N-{3-[4-(3,4-dimethoxybenzyl)-1,4-diazepan-1-yl]benzyl}-2-methylbenzamide,
(167) 4-chloro-N-{3-[4-(4-fluorobenzyl)-1,4-diazepan-1-yl]benzyl}benzamide,
(168) 3-chloro-N-{3-[4-(4-fluorobenzyl)-1,4-diazepan-1-yl]benzyl}-2-methylbenzamide,
(169) 4-chloro-N-{4-[4-(3,4-dimethoxybenzyl)-1,4-diazepan-1-yl]benzyl}benzamide,
(170) 3-chloro-N-{4-[4-(3,4-dimethoxybenzyl)-1,4-diazepan-1-yl]benzyl}-2-methylbenzamide,
(171) 4-chloro-N-{4-[4-(4-fluorobenzyl)-1,4-diazepan-1-yl]benzyl}benzamide,
(172) 3-chloro-N-{4-[4-(4-fluorobenzyl)-1,4-diazepan-1-yl]benzyl}-2-methylbenzamide,
(173) 4-chloro-N-({2-[4-(3,4-dimethoxybenzyl)-1,4-diazepan-1-yl]pyridin-3-yl}methyl)benzamide,
(174) 3-chloro-N-({2-[4-(3,4-dimethoxybenzyl)-1,4-diazepan-1-yl]pyridin-3-yl}methyl)-2-methylbenzamide,
(175) 4-chloro-N-({2-[4-(4-fluorobenzyl)-1,4-diazepan-1-yl]pyridin-3-yl}methyl)benzamide,
(176) 3-chloro-N-({2-[4-(4-fluorobenzyl)-1,4-diazepan-1-yl]pyridin-3-yl}methyl)-2-methylbenzamide,
(177) 4-chloro-N-({2-[4-(3,4-dimethoxybenzyl)-1,4-diazepan-1-yl]pyridin-4-yl}methyl)benzamide,
(178) 3-chloro-N-({2-[4-(3,4-dimethoxybenzyl)-1,4-diazepan-1-yl]pyridin-4-yl}methyl)-2-methylbenzamide,
(179) 4-chloro-N-({2-[4-(4-fluorobenzyl)-1,4-diazepan-1-yl]pyridin-4-yl}methyl)benzamide,
(180) 3-chloro-N-({2-[4-(4-fluorobenzyl)-1,4-diazepan-1-yl]pyridin-4-yl}methyl)-2-methylbenzamide,
(181) 4-chloro-N-({4-[4-(3,4-dimethoxybenzyl)-1,4-diazepan-1-yl]-1,3-thiazol-5-yl}methyl)benzamide,
(182) 3-chloro-N-({4-[4-(3,4-dimethoxybenzyl)-1,4-diazepan-1-yl]-1,3-thiazol-5-yl}methyl)-2-methylbenzamide,
(183) 4-chloro-N-({4-[4-(4-fluorobenzyl)-1,4-diazepan-1-yl]-1,3-thiazol-5-yl}methyl)benzamide,
(184) 3-chloro-N-({4-[4-(4-fluorobenzyl)-1,4-diazepan-1-yl]-1,3-thiazol-5-yl}methyl)-2-methylbenzamide,
(185) 4-chloro-N-({2-[4-(3,4-dimethoxybenzyl)-1,4-diazepan-1-yl]-1,3-thiazol-5-yl}methyl)benzamide,
(186) 3-chloro-N-({2-[4-(3,4-dimethoxybenzyl)-1,4-diazepan-1-yl]-1,3-thiazol-5-yl}methyl)-2-methylbenzamide,
(187) 4-chloro-N-({2-[4-(4-fluorobenzyl)-1,4-diazepan-1-yl]-1,3-thiazol-5-yl}methyl)benzamide or
(188) 3-chloro-N-({2-[4-(4-fluorobenzyl)-1,4-diazepan-1-yl]-1,3-thiazol-5-yl}methyl)-2-methylbenzamide;
and the compounds of the following (189)-(300), salts thereof, N-oxides thereof, solvates thereof or prodrugs thereof *etc*. are also preferable.
(189) N-{2-[1-(4-fluorobenzyl)piperidin-4-yl]benzyl}-N-phenylacetamide,
(190) N-{2-[1-(4-fluorobenzyl)piperidin-4-yl]benzyl}-N-phenylmethanesulfonamide,
(191) N'-ethyl-N- {2-[1-(4-fluorobenzyl)piperidin-4-yl]benzyl}-N-phenylurea,
(192) methyl 2-[1-(4-fluorobenzyl)piperidin-4-yl]benzyl(phenyl)carbamate,
(193) N-benzyl-N-{2-[1-(4-fluorobenzyl)piperidin-4-yl]benzyl}acetamide,
(194) N-benzyl-N-{2-[1-(4-fluorobenzyl)piperidin-4-yl]benzyl}methanesulfonamide,
(135) N-benzyl-N'-ethyl-N-{2-[1-(4-fluorobenzyl)piperidin-4-yl]benzyl}urea,
(196) methyl benzyl{2-[1-(4-fluorobenzyl)piperidin-4-yl]benzyl}carbamate,
(197) N-{2-[1-(4-fluorobenzyl)piperidin-4-yl]benzyl}-N-(2-phenylethyl)acetamide,
(198) N-{2-[1-(4-fluorobenzyl)piperidin-4-yl]benzyl}-N-(2-phenylethyl)methanesulfonamide,
(199) N'-ethyl-N-{2-[1-(4-fluorobenzyl)piperidin-4-yl]benzyl}-N-(2-phenylethyl)urea,
(200) methyl 2-[1-(4-fluorobenzyl)piperidin-4-yl]benzyl(2-phenylethyl)carbamate,
(201) N-cyclopentyl-N-{2-[1-(4-fluorobenzyl)piperidin-4-yl]benzyl}acetamide,
(202) N-cyclopentyl-N-{2-[1-(4-fluorobenzyl)piperidin-4-yl]benzyl}methanesulfonamide,
(203) N-cyclopentyl-N'-ethyl-N-{2-[1-(4-fluorobenzyl)piperidin-4-yl]benzyl}urea,
(204) methyl cyclopentyl{2-[1-(4-fluorobenzyl)piperidin-4-yl]benzyl}carbamate,
(205) N-cyclohexyl-N-{2-[1-(4-fluorobenzyl)piperidin-4-yl]benzyl}acetamide,
(206) N-cyclohexyl-N-{2-[1-(4-fluorobenzyl)piperidin-4-yl]benzyl}methanesulfonamide,
(207) N-cyclohexyl-N'-ethyl-N-{2-[1-(4-fluorobenzyl)piperidin-4-yl]benzyl}urea,
(208) methyl cyclohexyl{2-[1-(4-fluorobenzyl)piperidin-4-yl]benzyl}carbamate,
(209) N-(cyclohexylmethyl)-N-{2-[1-(4-fluorobenzyl)piperidin-4-yl]benzyl}acetamide,
(210) N-(cyclohexylmethyl)-N-{2-[1-(4-fluorobenzyl)piperidin-4-yl]benzyl}methanesulfonamide,
(211) N-(cyclohexylmethyl)-N'-ethyl-N-{2-[1-(4-fluorobenzyl)piperidin-4-yl]benzyl}urea,
(212) methyl cyclohexylmethyl{2-[1-(4-fluorobenzyl)piperidin-4-yl]benzyl}carbamate,

(213) N-{2-[1-(4-fluorobenzyl)piperidin-4-yl]benzyl}-N-(2-methylphenyl)acetamide,
(214) N-{2-[1-(4-fluorobenzyl)piperidin-4-yl]benzyl}-N-(2-methylphenyl)methanesulfonamide,
(215) N'-ethyl-N-{2-[1-(4-fluorobenzyl)piperidin-4-yl]benzyl}-N-(2-methylphenyl)urea,
(216) methyl 2-[1-(4-fluorobenzyl)piperidin-4-yl]benzyl(2-methylphenyl)carbamate,
(217) N-{2-[1-(4-fluorobenzyl)piperidin-4-yl]benzyl}-N-(3-methylphenyl)acetamide,
(218) N-{2-[1-(4-fluorobenzyl)piperidin-4-yl]benzyl}-N-(3-methylphenyl)methanesulfonamide,
(219) N'-ethyl-N-{2-[1-(4-fluorobenzyl)piperidin-4-yl]benzyl}-N-(3-methylphenyl)urea,
(220) methyl 2-[1-(4-fluorobenzyl)piperidin-4-yl]benzyl(3-methylphenyl)carbamate,
(221) N-{2-[1-(4-fluorobenzyl)piperidin-4-yl]benzyl}-N-(4-methylphenyl)acetamide,
(222) N-{2-[1-(4-fluorobenzyl)piperidin-4-yl]benzyl}-N-(4-methylphenyl)methanesulfonamide,
(223) N'-ethyl-N-{2-[1-(4-fluorobenzyl)piperidin-4-yl]benzyl}-N-(4-methylphenyl)urea,
(224) methyl 2-[1-(4-fluorobenzyl)piperidin-4-yl]benzyl(4-methylphenyl)carbamate,
(225) N-{2-[1-(4-fluorobenzyl)piperidin-4-yl]benzyl}-N-(2-methoxyphenyl)acetamide,
(226) N-{2-[1-(4-fluorobenzyl)piperidin-4-yl]benzyl}-N-(2-methoxyphenyl)methanesulfonamide,
(227) N'-ethyl-N-{2-[1-(4-fluorobenzyl)piperidin-4-yl]benzyl}-N-(2-methoxyphenyl)urea,
(228) methyl 2-[1-(4-fluorobenzyl)piperidin-4-yl]benzyl(2-methoxyphenyl)carbamate,
(229) N-{2-[1-(4-fluorobenzyl)piperidin-4-yl]benzyl}-N-(3-methoxyphenyl)acetamide,
(230) N-{2-[1-(4-fluorobenzyl)piperidin-4-yl]benzyl}-N-(3-methoxyphenyl)methanesulfonamide,
(231) N'-ethyl-N-{2-[1-(4-fluorobenzyl)piperidin-4-yl]benzyl}-N-(3-methoxyphenyl)urea,
(232) methyl 2-[1-(4-fluorobenzyl)piperidin-4-yl]benzyl(3-methoxyphenyl)carbamate,
(233) N-{2-[1-(4-fluorobenzyl)piperidin-4-yl]benzyl}-N-(4-methoxyphenyl)acetamide,
(234) N-{2-[1-(4-fluorobenzyl)piperidin-4-yl]benzyl}-N-(4-methoxyphenyl)methanesulfonamide,
(235) N'-ethyl-N-{2-[1-(4-fluorobenzyl)piperidin-4-yl]benzyl}-N-(4-methoxyphenyl)urea,
(236) methyl 2-[1-(4-fluorobenzyl)piperidin-4-yl]benzyl(4-methoxyphenyl)carbamate,
(237) N-(2-chlorophenyl)-N-{2-[1-(4-fluorobenzyl)piperidin-4-yl]benzyl}acetamide,
(238) N-(2-chlorophenyl)-N-{2-[1-(4-fluorobenzyl)piperidin-4-yl]benzyl}methanesulfonamide,
(239) N-(2-chlorophenyl)-N'-ethyl-N-{2-[1-(4-fluorobenzyl)piperidin-4-yl]benzyl}urea,
(240) methyl 2-chlorophenyl{2-[1-(4-fluorobenzyl)piperidin-4-yl]benzyl}carbamate,
(241) N-(3-chlorophenyl)-N-{2-[1-(4-fluorobenzyl)piperidin-4-yl]benzyl}acetamide,
(242) N-(3-chlorophenyl)-N-{2-[1-(4-fluorobenzyl)piperidin-4-yl]benzyl}methanesulfonamide,
(243) N-(3-chlorophenyl)-N'-ethyl-N-{2-[1-(4-fluorobenzyl)piperidin-4-yl]benzyl}urea,
(244) methyl 3-chlorophenyl{2-[1-(4-fluorobenzyl)piperidin-4-yl]benzyl}carbamate,
(245) N-(4-chlorophenyl)-N-{2-[1-(4-fluorobenzyl)piperidin-4-yl]benzyl}acetamide,
(246) N-(4-chlorophenyl)-N-{2-[1-(4-fluorobenzyl)piperidin-4-yl]benzyl}methanesulfonamide,
(247) N-(4-chlorophenyl)-N'-ethyl-N-{2-[1-(4-fluorobenzyl)piperidin-4-yl]benzyl}urea,
(248) methyl 4-chlorophenyl{2-[1-(4-fluorobenzyl)piperidin-4-yl]benzyl}carbamate,
(249) N-{2-[1-(4-fluorobenzyl)piperidin-4-yl]benzyl}-N-(2-methylbenzyl)acetamide,
(250) N-{2-[1-(4-fluorobenzyl)piperidin-4-yl]benzyl}-N-(2-methylbenzyl)methanesulfonamide,
(251) N'-ethyl-N-{2-[1-(4-fluorobenzyl)piperidin-4-yl]benzyl}-N-(2-methylbenzyl)urea,
(252) methyl 2-[1-(4-fluorobenzyl)piperidin-4-yl]benzyl(2-methylbenzyl)carbamate,
(253) N-{2-[1-(4-fluorobenzyl)piperidin-4-yl]benzyl}-N-(3-methylbenzyl)acetamide,
(254) N-{2-[1-(4-fluorobenzyl)piperidin-4-yl]benzyl}-N-(3-methylbenzyl)methanesulfonamide,
(255) N'-ethyl-N-{2-[1-(4-fluorobenzyl)piperidin-4-yl]benzyl}-N-(3-methylbenzyl)urea,
(256) methyl 2-[1-(4-fluorobenzyl)piperidin-4-yl]benzyl(3-methylbenzyl)carbamate,
(257) N-{2-[1-(4-fluorobenzyl)piperidin-4-yl]benzyl}-N-(4-methylbenzyl)acetamide,
(258) N-{2-[1-(4-fluorobenzyl)piperidin-4-yl]benzyl}-N-(4-methylbenzyl)methanesulfonamide,
(259) N'-ethyl-N-{2-[1-(4-fluorobenzyl)piperidin-4-yl]benzyl}-N-(4-methylbenzyl)urea,
(260) methyl 2-[1-(4-fluorobenzyl)piperidin-4-yl]benzyl(4-methylbenzyl)carbamate,
(261) N-{2-[1-(4-fluorobenzyl)piperidin-4-yl]benzyl}-N-(2-methoxybenzyl)acetamide,
(262) N-{2-[1-(4-fluorobenzyl)piperidin-4-yl]benzyl}-N-(2-methoxybenzyl)methanesulfonamide,
(263) N'-ethyl-N-{2-[1-(4-fluorobenzyl)piperidin-4-yl]benzyl}-N-(2-methoxybenzyl)urea,
(264) methyl 2-[1-(4-fluorobenzyl)piperidin-4-yl]benzyl(2-methoxybenzyl)carbamate,
(265) N-{2-[1-(4-fluorobenzyl)piperidin-4-yl]benzyl}-N-(3-methoxybenzyl)acetamide,
(266) N-{2-[1-(4-fluorobenzyl)piperidin-4-yl]benzyl}-N-(3-methoxybenzyl)methanesulfonamide,
(267) N'-ethyl-N-{2-[1-(4-fluorobenzyl)piperidin-4-yl]benzyl}-N-(3-methoxybenzyl)urea,
(268) methyl 2-[1-(4-fluorobenzyl)piperidin-4-yl]benzyl(3-methoxybenzyl)carbamate,
(269) N-{2-[1-(4-fluorobenzyl)piperidin-4-yl]benzyl}-N-(4-methoxybenzyl)acetamide,
(270) N-{2-[1-(4-fluorobenzyl)piperidin-4-yl]benzyl}-N-(4-methoxybenzyl)methanesulfonamide,

(271) N'-ethyl-N-{2-[1-(4-fluorobenzyl)piperidin-4-yl]benzyl}-N-(4-methoxybenzyl)urea,
(272) methyl 2-[1-(4-fluorobenzyl)piperidin-4-yl]benzyl(4-methoxybenzyl)carbamate,
(273) N-(2-chlorobenzyl)-N-{2-[1-(4-fluorobenzyl)piperidin-4-yl]benzyl}acetamide,
(274) N-(2-chlorobenzyl)-N-{2-[1-(4-fluorobenzyl)piperidin-4-yl]benzyl}methanesulfonamide,
(275) N-(2-chlorobenzyl)-N'-ethyl-N-{2-[1-(4-fluorobenzyl)piperidin-4-yl]benzyl}urea,
(276) methyl 2-chlorobenzyl{2-[1-(4-fluorobenzyl)piperidin-4-yl]benzyl}carbamate,
(277) N-(3-chlorobenzyl)-N-{2-[1-(4-fluorobenzyl)piperidin-4-yl]benzyl}acetamide,
(278) N-(3-chlorobenzyl)-N-{2-[1-(4-fluorobenzyl)piperidin-4-yl]benzyl}methanesulfonamide,
(279) N-(3-chlorobenzyl)-N'-ethyl-N-{2-[1-(4-fluorobenzyl)piperidin-4-yl]benzyl}urea,
(280) methyl 3-chlorobenzyl{2-[1-(4-fluorobenzyl)piperidin-4-yl]benzyl}carbamate,
(281) N-(4-chlorobenzyl)-N-{2-[1-(4-fluorobenzyl)piperidin-4-yl]benzyl}acetamide,
(282) N-(4-chlorobenzyl)-N-{2-[1-(4-fluorobenzyl)piperidin-4-yl]benzyl}methanesulfonamide,
(283) N-(4-chlorobenzyl)-N'-ethyl-N-{2-[1-(4-fluorobenzyl)piperidin-4-yl]benzyl}urea,
(284) methyl 4-chlorobenzyl{2-[1-(4-fluorobenzyl)piperidin-4-yl]benzyl }carbamate,
(285) N-{2-[1-(4-fluorobenzyl)piperidin-4-yl]benzyl}-N-(pyridin-2-ylmethyl)acetamide,
(286) N-{2-[1-(4-fluorobenzyl)piperidin-4-yl]benzyl}-N-(pyridin-2-ylmethyl)methanesulfonamide,
(287) N'-ethyl-N-{2-[1-(4-fluorobenzyl)piperidin-4-yl]benzyl}-N-(pyridin-2-ylmethyl)urea,
(288) methyl 2-[1-(4-fluorobenzyl)piperidin-4-yl]benzyl(pyridin-2-ylmethyl)carbamate,
(289) N-{2-[1-(4-fluorobenzyl)piperidin-4-yl]benzyl}-N-(pyridin-3-ylmethyl)acetamide,
(290) N-{2-[1-(4-fluorobenzyl)piperidin-4-yl]benzyl}-N-(pyridin-3-ylmethyl)methanesulfonamide,
(291) N'-ethyl-N-{2-[1-(4-fluorobenzyl)piperidin-4-yl]benzyl}-N-(pyridin-3-ylmethyl)urea,
(292) methyl 2-[1-(4-fluorobenzyl)piperidin-4-yl]benzyl(pyridin-3-ylmethyl)carbamate,
(293) N-{2-[1-(4-fluorobenzyl)piperidin-4-yl]benzyl}-N-(piperldin-4-ylmethyl)acetamide,
(294) N-{2-[1-(4-fluorobenzyl)piperidin-4-yl]benzyl}-N-(piperidin-4-ylmethyl)methanesulfonamide,
(295) N'-ethyl-N-{2-[1-(4-fluorobenzyl)piperidin-4-yl]benzyl}-N-(piperidin-4-ylmethyl)urea,
(296) methyl 2-[1-(4-fluorobenzyl)piperidin-4-yl]benzyl(piperidin-4-ylmethyl)carbamate,
(297) N-{2-[1-(4-fluorobenzyl)piperidin-4-yl]benzyl}-N-(2-phenoxyethyl)acetamide,
(298) N-{2-[1-(4-fluorobenzyl)piperidin-4-yl]benzyl}-N-(2-phenoxyethyl)methanesulfonamide,
(299) N'-ethyl-N-{2-[1-(4-fluorobenzyl)piperidin-4-yl]benzyl}-N-(2-phenoxyethyl)urea, and
(300) methyl 2-[1-(4-fluorobenzyl)piperidin-4-yl]benzyl(2-phenoxyethyl)carbamate.

In the present invention, all isomers are included unless specified. For example, alkyl, alkenyl, alkynyl, alkoxy, alkylthio, alkylene, alkenylene and alkynylene include straight and branched ones. Furthermore, the present invention includes isomers in double bonds, rings, fused rings (E, Z, cis, trans), isomers by the existence of asymmetric carbons *etc.* (R, S, α, β, enantiomer, diastereomer), optical isomers having optical rotation (D, L, d, 1), polars by chromatography separation (more polar, less polar), equilibrium compounds, rotational isomers, compounds of arbitrary ratios thereof and racemic mixture.

In the present invention, as is easily understood by those skilled in the art, the symbol 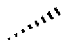 indicates that the substituent attached thereto is behind the sheet (α-position), the symbol 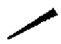 indicates that the substituent attached thereto is in front of the sheet (β-position), and 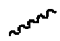 indicates that the substituent attached thereto is a mixture of those in β-position or α-position.

Salts:

The salts of the compound of formula (I) include all pharmaceutically acceptable ones. Pharmaceutically acceptable salts are preferably non-toxic and water-soluble ones. Appropriate salts are, e.g. salts of alkali metals (potassium, sodium, lithium, *etc.*), salts of alkaline earth metals (calcium, magnesium, *etc.*), ammonium salts (tetramethylammonium salt, tetrabutylammonium salt, *etc.),* salts of organic amines (triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, *etc.),* acid-addition salts [salts of inorganic acid (hydrochloride, hydrobromide, hydroiodide, sulfate, phoaphate, nitrate, *etc.*), salts of organic acids (acetate, trifluoroacetate, lactate, tartarate, oxalate, fumarate, maleate, benzoate, citrate, methanesulfonate, ethanesulfonate, benzenesulfonate, toluenesulfonate, isethionate , glucuronate, gluconate, *etc.*), *etc.*]. The salts of the compound of the present invention include, solvates thereof, solvates of alkali (earth) metals, ammonium salts, salts of organic amines or acid-addition salts of the

compound of the present invention. Preferable solvates are non-toxic and water-soluble ones. Appropriate solvates are, for example, solvates of water, alcohols (ethanol, *etc.*), *etc.* The compound of the present invention may be converted into pharmacologically acceptable salts by known methods.

Also, the salts include quartemary ammonium salts. Quarternary ammonium salts mean the compounds wherein the nitrogen atom of the compound of formula (I) is quarternarized by $R^0$.

R° is C1-8 alkyl, C1-8 alkyl substituted with phenyl.

The compounds of the present invention may be converted into N-oxides. The N-oxides mean the compounds wherein the nitrogen atom in the compound of formula (I) is oxidized.

The prodrugs of the compound of formula (I) (the compound (I)) mean the compounds which are converted into the compound (I) by an enzyme, gastric acid, *etc.* in the body. The prodrugs of the compound (I) are, when the compound (I) possesses an amino group, the amino group is acylated, alkylated, phosphorylated (e.g. the amino group of the compound (I) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated, t-butylated, etc.); when the compound (I) has a hydroxy group, the hydroxy group is acylated, alkylated, phosphorylated, borated (e.g. the hydroxy group of the compound (I) is acetylated, palmitoylated, propanoylated, pivaloylated, succinylated, fumarylated, alanylated, dimethylaminomethylcarbonylated, *etc.);* when the compound (I) has a carboxy group, the carboxy group is esterified, or amidated (e.g. the carboxy group of the compound (I) is converted into ethyl ester, ethoxycarbonyloxyethyl ester, pivaloyloxymethyl ester, ethoxycarbonyloxyethyl ester, phthalidy ester, (5-methyl-2-oxo-1,3-dioxolen-4-yl)methyl ester, cyclohexylcarbonylethyl ester, methyl amide, *etc.*), *etc.* These compounds may be prepared by known methods. The prodrug of the compound (I) may be a hydrate or a non-hydrate.

Method for the preparation of the compound of the present invention:

The compound of formula (I) may be prepared by known methods, for example, the following methods, the methods applying the following methods, or the methods described in Comprehensive Organic Transformations : A Guide to Functional Group Preparations, 2nd Edition (Richard C. Larock, John Wiley & Sons Inc, 1999), *etc.* or modifying and combining them. In each following method for the preparation, salts of the starting material may be used. The above salts of the compound (I) may be used.

In the formula (I), the compound wherein $R^1$ is attached to the ring A via a nitrogen atom, i.e. the compound (I-1) may be prepared, for example, according to the following method A.

Method A:

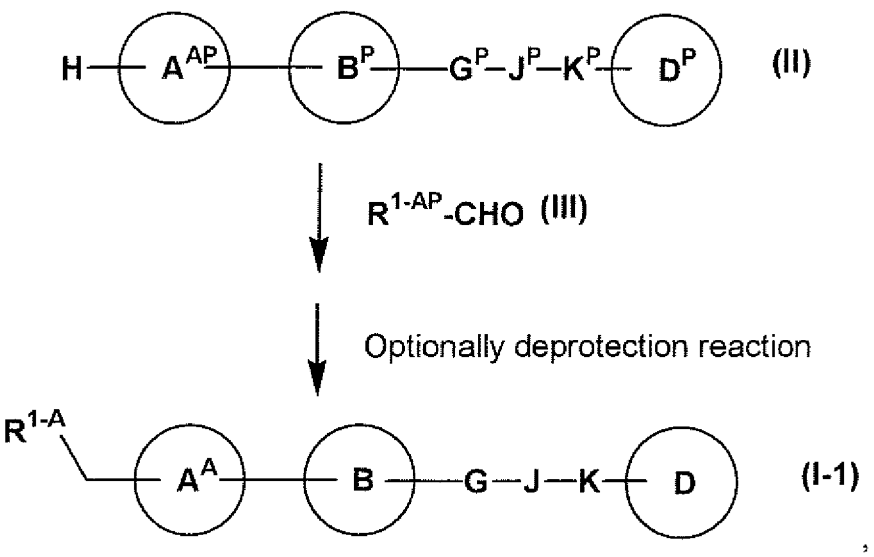


wherein ring $A^A$ has the same meaning as the ring A, with proviso that $R^1$ is attached to the ring A via the nitrogen atom and $R^{1-A}$ has the same meaning as $R^1$, but $R^{1-A}$ represents an aliphatic hydrocarbon group which has one less carbon atom from the aliphatic hydrocarbon optionally having substituent(s) represented by $R^1$, and ring $A^{AP}$, ring $B^P$, $G^P$, $J^P$, $K^P$ and ring $D^P$ have the same meanings as the ring $A^A$, ring B, G, J, K and ring D respectively, with proviso that when

the carboxy, hydroxy, amino and mercapto included in the groups represented by ring $A^{AP}$, ring $B^P$, $G^P$, $J^P$, $K^P$ and ring $D^P$ are protected if necessary, and the other symbols have the same meanings as hereinbefore.

The "aliphatic hydrocarbon" in the "optionally substituted aliphatic hydrocarbon" represented by $R^{1-A}$ is, for example, straight or branched C1-9 alkyl, straight or branched C2-9 alkenyl, straight or branched C2-9 alkynyl, *etc.* The "straight or branched C1-9 alkyl", "straight or branched C2-9 alkenyl", "straight or branched C2-9 alkynyl" represent the ones possessing C1-9 in the "straight or branched C2-10 alkyl", "straight or branched C2-10 alkenyl", "straight or branched C2-10 alkynyl" exemplified as the above $R^1$. The "substituent" in the "optionally substituted aliphatic hydrocarbon" represented by $R^{1-A}$ has the same meaning as the "substituent" as exemplified as the above $R^1$.

This method is carried out by subjecting to a reductive amination reaction the compound of formula (II) and the compound of formula (III), optionally followed by subjecting to a deprotection reaction of the protective groups.

This reductive amination reaction is carried out by known methods or according to known methods. For example, it is carried out in an organic solvent (e.g. dichloroethane, dichloromethane, N,N-dimethylformamide, acetic acid and a mixture thereof, *etc.*), in the presence of a reducing agent (sodium triacetoxyborohydride, sodium cyanoborhydride, *etc.*) at a temperature between 0 to 40°C.

The deprotection reaction of the protective groups may be carried out by known methods, for example, the methods described in T. W. Greene, Protective Groups in Organic Synthesis, Wiley, New York, 1999 or a method according to these methods. For example, the deprotection reaction of carboxy, hydroxy, amino or mercapto is well known, e.g. (1) alkali hydrolysis, (2) a deprotection under acidic condition, (3) a deprotection reaction by hydration, (4) a deprotection of silyl group, (5) a deprotection reaction using a metal, (6) a deprotection reaction using a metal complex, *etc.*

To explain these methods concretely,

(1) The deprotection reaction by alkali hydrolysis is carried out, for example, in an organic solvent (methanol, tetrahydrofuran, 1,4-dioxane, etc.) using a hydroxide of alkali metals (sodium hydroxide, potassium hydroxide, lithium hydroxide, *etc.*), hydroxide of alkaline earth metals (barium hydroxide, calcium hydroxide, *etc.*), carbonate (sodium carbonate, potassium carbonate, *etc.)* or a solution thereof or a mixture thereof at a temperature of 0 to 40°C;

(2) The deprotection reaction under acidic conditions is carried out, for example, in an organic solvent (dichloromethane, chloroform, 1,4-dioxane, ethyl acetate, anisole, *etc.),* in an organic acid (acetic acid, trifluoroacetic acid, methanesulfonic acid, p-toluenesulfonic acid, *etc.)* or an inorganic acid (hydrochloric acid, sulfuric acid, *etc.)* or a mixture thereof (hydrobromic acid/acetic acid, *etc.)* at a temperature of 0 to 100°C;

(3) The deprotection reaction by hydration is, for example, carried out in a solvent (ethers (tetrahydrofuran, 1,4-dioxane, dimethoxyethane, diethyl ether, *etc.*), alcohols (methanol, ethanol, *etc.*), benzenes (benzene, toluene, *etc.*), ketones (acetone, methyl ethyl ketone, *etc.*), nitriles (acetonitrile *etc.*), amides (dimethylformamide *etc.*), water, ethyl acetate, acetic acid or a mixture of more than two from above, etc.) in the presence of a catalyst (palladium-carbon, palladium black, palladium hydroxide, platinum oxide, Raney nickel, *etc.*) under the atmosphere of hydrogen of normal or suppressed pressure, or in the presence of ammonium formate at a temperature of 0 to 200°C;

(4) The deprotection of silyl group is, for example, carried out in a water-miscible organic solvent (tetrahydrofuran, acetonitrile, *etc.*) using tetrabutylammonium fluoride at a temperature of 0 to 40°C;

(5) The deprotection reaction by using a metal is carried out, for example, in an acidic solvent (acetic acid, a buffer of pH 4.2 to 7.2 or a mixture of the solution thereof and an organic solvent such as tetrahydrofuran etc.) in the presence of zinc powder at a temperature of 0 to 40°C optionally under sonication;

(6) The deprotection reaction by using a metal complex is carried out, for example, in an organic solvent (dichloromethane, dimethylformamide, tetrahydrofuran, ethyl acetate, acetonitrile, dioxane, ethanol, *etc.*), water or a mixture thereof, in the presence of a trap reagent (tributyltin hydride, triethylsilane, dimedone, morpholine, diethylamine, pyrrolidine, *etc.),* an organic acid (acetic acid, formic acid, 2-ethylhexane, *etc.*) and/or a salt of an organic acid (sodium 2-ethylhexanoate, potassium 2-ethylhexanoate, *etc.)* in the presence or absence of a phosphine reagent (triphenylphosphine etc.) using a metal complex (palladium tetrakistriphenylphosphine (0), palladium bis(triphenylphosphosphine) dichloride (II), palladium acetate (II), rhodium tris(triphenylphosphine) chloride (I), *etc.* at a temperature of 0 to 40°C.

As is easily understood by those skilled in the art, the desired compound of the present invention can be easily prepared.

The protective groups of carboxy include, for example, methyl, ethyl, allyl, t-butyl, trichloroethyl, benzyl (Bn), phenacyl, *etc.* The protective groups of hydroxy include, for example, methyl, trityl, methoxymethyl (MOM), 1-ethoxyethyl (EE), methoxyethoxymethyl (MEM), 2-tetrahydropyranyl (THP), trimethylsilyl (TMS), triethylsilyl (TES), t-butyldimethylsilyl (TBDMS), t-butyldiphenylsilyl (TBDPS), acetyl (Ac), pivaloyl, benzoyl, benzyl (Bn), p-methoxybenzyl, allyloxycarbonyl (Alloc), 2,2,2-trichloroethoxycarbonyl (Troc), *etc.* The protective groups of amino include, for example, benzyloxycarbonyl, t-butoxycarbonyl, allyloxycarbonyl (Alloc), 1-methyl-1-(4-biphenyl)ethoxycarbonyl (Bpoc), trifluoroacetyl, 9-fluorenylmethoxycarbonyl, benzyl (Bn), p-methoxybenzyl, benzyloxymethyl (BOM), 2-(trimethylsilyl)ethoxymethyl (SEM), *etc.* The protective groups of mercapto include, for example, benzyl, methoxybenzyl, methoxymethyl (MOM), 2-tetrahydropyranyl (THP), diphenylmethyl, acetyl (Ac). Protective groups for carboxy, hydroxy, amino or mercapto are not limited to the above groups, but those groups eliminated easily and selectively may be also used. For example, the ones described in T. W. Greene, Protective Groups in Organic Synthesis, Wiley, New York, 1999 are used.

In the formula (I), the compound wherein J is $-CONR^2-$, $-NR^2CO-$, -OCO-, or -COO-, i.e. the compound of formula (I-2) may be prepared, for example, by the following method B.

Method B

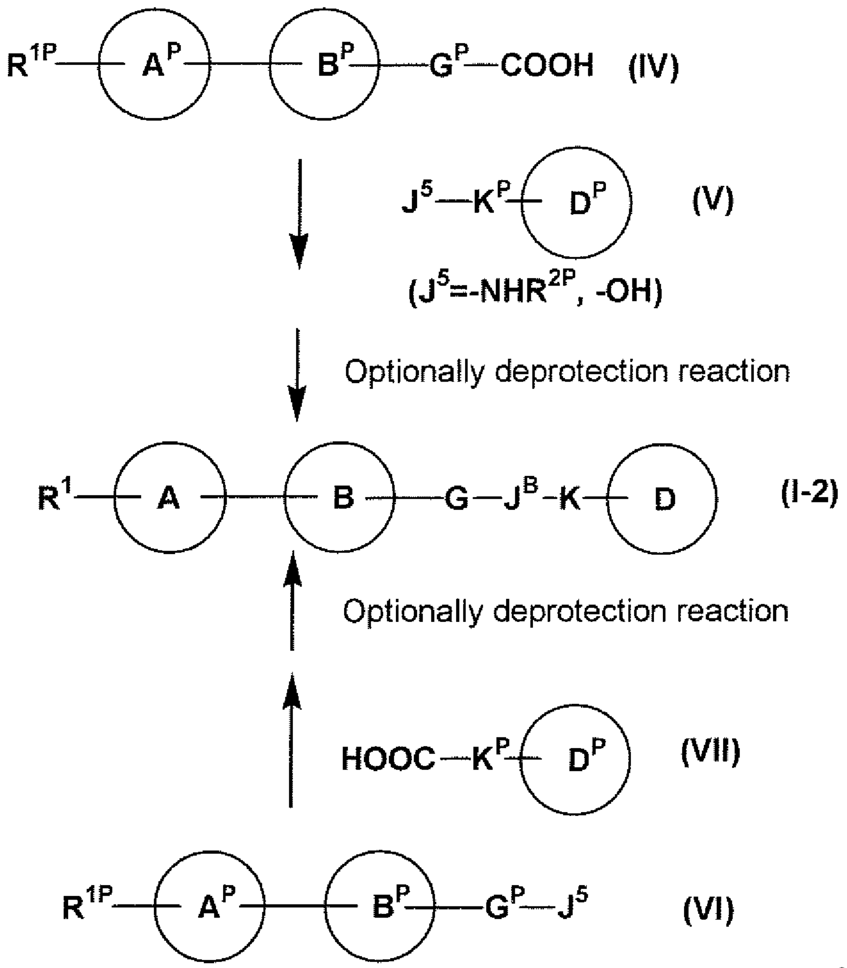


(IV) (V) (I-2) (VII) (VI)

wherein $R^{1P}$, $R^{2P}$ and ring $A^P$ have the same meanings as $R^1$, $R^2$ or ring $A^P$, and carboxy, hydroxy, amino and mercapto included in the group represented by $R^{1P}$, $R^{2P}$ and ring $A^P$ are protected when required, $J^B$ is $-CONR^2-$, $-NR^2CO-$, -OCO-, or -COO-, and the other symbols have the same meanings as hereinbefore.

This method is carried out by subjecting to an amidation reaction or esterification reaction the compound (IV) and the compound (V), or the compound (VI) and the compound (VII), optionally followed by subjecting to a deprotection reaction of protective groups.

The amidation or esterification reactions are carried out by known methods or by modifying known methods, for example,

(1) a method using an acid halide, (2) a method using a mixed anhydride,
(3) a method using a condensing agent, (4) a method using an activated ester, *etc.*

To explain these methods concretely,

(1) The method using an acid halide is, for example, carried out by subjecting to a reaction carboxylic acid in an organic solvent (chloroform, methylene chloride, diethyl ether, tetrahydrofuran, etc.) or without a solvent, and an acid-halogenating agent (oxalyl chloride, thionyl chloride, *etc.*) at a temperature of -20°C to refluxing temperature, and then subjecting to a reaction the resulting acid halide in the presence of a tertiary amine (pyridine, triethylamine, N,N-dimethylaniline, N,N-dimethylaminopyridine, diisopropylethylamine, *etc.)* with an amine in an organic solvent (chloroform, dichloromethane, diethyl ether, tetrahydrofuran, etc.) at a temperature of 0 to 40°C;

(2) The method using a mixed anhydride is, for example, carried out by subjecting to a reaction in an organic solvent (chloroform, dichloromethane, diethyl ether, tetrahydrofuran, etc.) or without a solvent, in the presence of a tertiary amine (pyridine, triethylamine, N,N-dimethylaniline, N,N-dimethylaminopyridine, diisopropylethylamine, *etc.)* a carboxylic acid with an acid halide (pivaloyl chloride, p-toluenesulfonyl chloride, methanesulfonyl chloride, *etc.*) or an acid derivative (chloroethyl formate, chloroisobutyl formate, *etc.)* at a temperature of 0 to 40°C, and then subjecting to a reaction the resulting mixed anhydride with an amine or an alcohol at a temperature of 0 to 40°C;

(3) The method using a condensing agent is carried out, for example, in an inert organic solvent (chloroform, dichloromethane, N,N-dimethylformamide, diethyl ether, tetrahydrofuran, etc.) or without a solvent, in the presence or absence of a base (pyridine, triethylamine, dimethylaniline, N,N-dimethylaniline, N,N-dimethylaminopyridine, *etc.*), using a condensing reagent (1,3-dicyclohexylcarbodiimide (DCC), 1-ethyl-3-[3-(dimethylamino)propyl]carbodiimide (EDC), 1,1'-carbonyldiimizazole (CDI), 2-chloro-1-methylpyridinium iodide, 1-propylphosphonic acid cyclic anhydride (PPA), *etc.)* in the presence or absence of 1-hydroxybenzotriazole (HOBt), by subjecting to a reaction a carboxylic acid and an amine or an alcohol at a temperature of 0 to 40°C;

(4) The method using an activated ester is, for example, carried out by subjecting to a reaction a carboxylic acid in an organic solvent (e.g. chloroform, dichloromethane, tetrahydrofuran, N,N-dimethylformamide, *etc.)* with a phenol derivative (p-nitrophenol, pentafluorophenol, etc.) or a succinimide derivative (N-hydroxysuccinimide *etc.)* using a condensing agent (1,3-dicyclohexylcarbodiimide (DCC), 1-ethyl-3-[3-(dimethylamino)propyl]carbodiimide (EDC), 1,3-diisopropylcarbonylimide, *etc.),* and the resulting activated ester is subjected to a reaction with an amine or an alcohol derivative in an organic solvent (tetrahydrofuran, dimethylformamide, *etc.*) in the presence or absence of a base (pyridine, triethylamine, *etc.*).

Each of these reactions (1), (2), (3) and (4) is desirably carried out in an inert gas (argon, nitrogen, *etc.)* under anhydrous conditions. In each reaction, reagents to be used may be supported by polystyrene *etc.*

The deprotection reactions of the protective groups may be carried out by the same methods as described hereinbefore.

In the formula (I), the compound wherein J is $-SO_2NR^2-$or $-NR^2SO_2-$, i.e. the compound of formula (I-3) may be prepared by the following method C.

Method C:

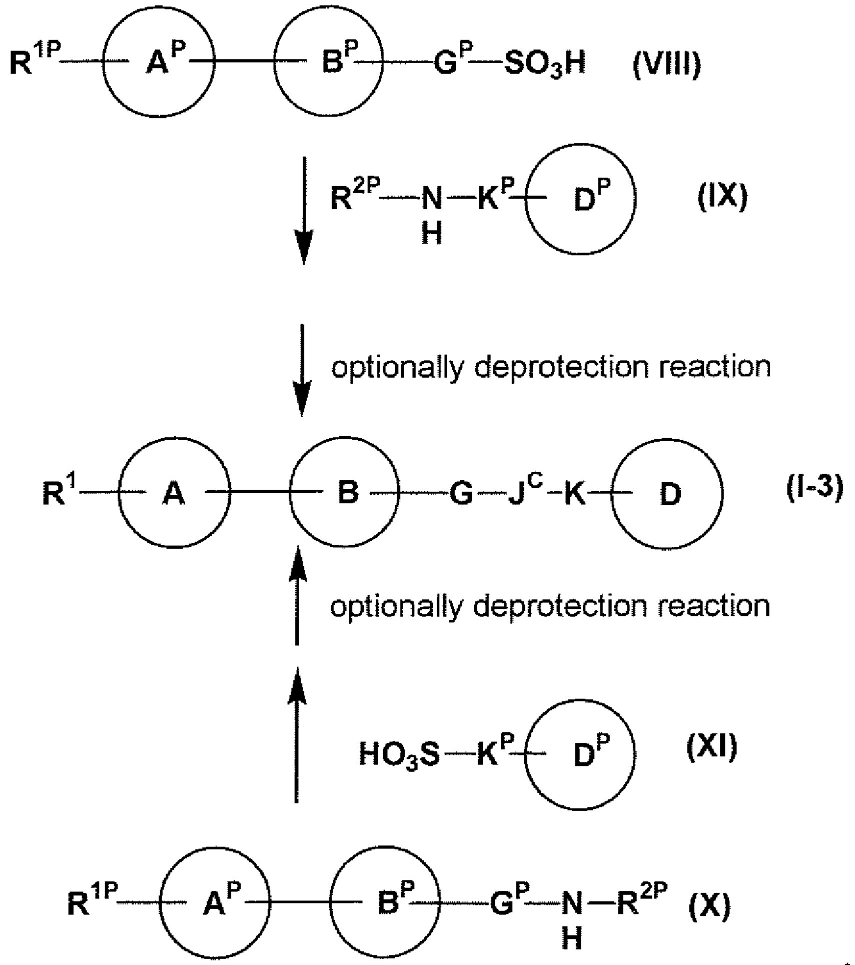


(VIII) (IX) (I-3) (XI) (X)

,

wherein $J^C$ is $-SO_2NR^2-$ or $-NR^2SO_2-$, and the other symbols have the same meanings as hereinbefore.

This method is carried out by subjecting to a sulfonamidation reaction the compound (VIII) and the compound (IX) or the compound (X) and the compound (XI), optionally followed by subjecting to a deprotection reaction of the protective groups.

This sulfonamidation reaction may be carried out by known methods or according to known methods. For example, it is carried out by subjecting to a reaction a sulfonic acid in an organic solvent (chloroform, dichloromethane, dichloroethane, diethyl ether, tetrahydrofuran, methyl t-butyl ether, *etc.*), or without a solvent, acid halide (oxalyl chloride, thionyl chloride, phosphorus pentachloride, phosphorus trichloride, *etc.*) at a temperature of -20°C to refluxing temperature, and then subjecting to a reaction the resulting sulfonyl halide in the presence of a base (diisopropylethylamine, pyridine, triethylamine, N,N-dimethylaniline, N,N-dimethylaminopyridine, *etc.)* in an organic solvent (chloroform, dichloromethane, dichloroethane, diethyl ether, tetrahydrofuran, *etc.*), with an amine at a temperature of 0 to 40°C.

In the formula (I), the compound wherein J is $-CSNR^2-$, $-NR^2CS-$, -O-CS-, or -CS-O-, i.e. the compound (I-4) may be prepared by the following method D.

Method D:

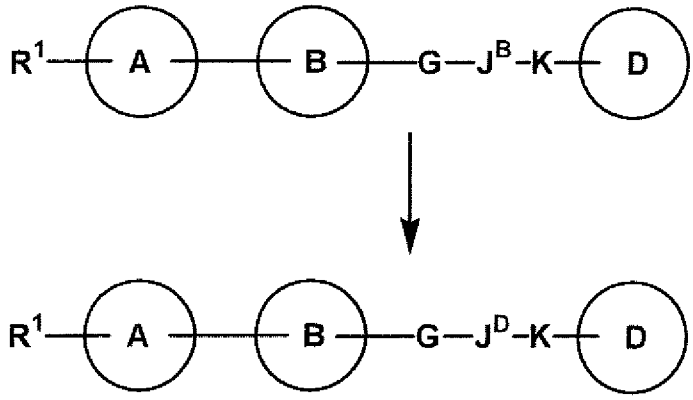

(I-2)

(I-4)

,

wherein $J^D$ is $-CSNR^2-$, $-NR^2CS-$, -O-CS-, or -CS-O-, and the other symbols have the same meanings as hereinbefore.

This method may be carried out by subjecting to a thiocarbonylation reaction the compound of formula (1-2), which was prepared by the previous method.

This thiocarbonylation reaction is carried out by known methods or according to known methods. For example, it is carried out in an organic solvent (e.g. toluene, diethyl ether, dichloromethane, chloroform, 1,4-dioxane, tetrahydrofuran, etc.) in the presence of thionating agent (Lawesson reagent (2,4-bis(4-methoxyphenyl)-1,3-dithia-2,4-diphosphetan-2,4-disulfide), phosphorus pentasulfide, *etc.*) at a temperature of 0 to 150°C.

In the compound of formula (I) of the present invention, the compound wherein at least one nitrogen atom represents a quarternary ammonium salt may be prepared by subjecting to a reaction the compound of formula (I) and the compound of formula (XII)

**$R^0$—Q** **(XII)**

wherein $R^0$ is C1-4 alkyl or C1-4 alkyl substituted with phenyl, Q is halogen atom.

This reaction is known, for example, it is carried out in an organic solvent (acetone, N,N-dimethylformamide, methyl ethyl ketone, *etc.)* at a temperature of 0 to 40°C.

In the compound of formula (I) of the present invention, the compound wherein at least one nitrogen atom represents N-oxide, may be prepared by subjecting to an oxidation reaction the compound of formula (I).

This oxidation reaction is known, for example, it is carried out in an appropriate organic solvent (dichloromethane, chloroform, benzene, hexane, t-butylalcohol, *etc.*) in the presence of excess oxidating agent (hydrogen peroxide, sodium periodate, acyl nitrite, sodium perborate, peracid (e.g. 3-chloroperbenzoate, peracetic acid, *etc.*), Oxone (a brand name of potassium peroxymonosulfate), potassium permanganate, chromate, *etc.*) at a temperature of 20 to 60°C.

The compounds of formula (II) to (XII) are known *per se,* or they may be prepared easily by combining the methods described in the examples, or known methods, for example, the methods described in *Comprehensive Organic Transformations : A Guide to Functional Group Preparations,* 2nd Edition (Richard C. Larock, John Wiley & Sons Inc, 1999), *etc.*

In each reaction of the present specification, polymer-supported reagents may be used which are supported with high molecular polymers (e.g. polystyrene, polyacrylamide, polypropylene, polyethyleglycol, *etc.*).

The compound of formula (II) may be prepared according to the methods described in the specification of WO01/60819.

In each reaction of the present specification, the reaction products may be purified by conventional techniques, for example, distillation under atmospheric or reduced pressure, high performance liquid chromatography, thin layer chromatography or column chromatography using silica gel or magnesium silicate, washing, recrystallization, *etc.* Purification may be carried out after each reaction, or after a series of reactions.

Pharmacological activity:

In order to carry out screening those compounds capable of inhibiting the binding of HIV to CCR5 which is a receptor on the CD4-positive cell, it is a more direct method to perform screening in an assay system where HIV is used. However, use of a large amount of HIV in the screening is not practical due to difficulty in handling. On the other hand,

since both the macrophage tropic (R5) HIV-I and the ligands, that is, RANTES, MIP-1α and MIP-1β, bind to CCR5, it can be presumed that there is a certain common character between CCR5 binding site of HIV and RANTES, MIP-1α or MIP-1β and RANTES, MIP-1a, MIP-1b and HIV binding site of CCR5.

Accordingly, in order to find those compounds capable of inhibiting adsorption of HIV to cells, which has an inhibitory mechanism different from the current anti-AIDS drugs (reverse transcription inhibitors and protease inhibitors, it is possible to use an assay system where an endogeneous CCR5 ligand is used; i.e. RANTES, MIP-1α, or MIP-1β in place of HIV. And of course, in order to carry out screening those compounds inhibiting the binding of CCR5 on the effector cells in inflammatory and immune diseases and RANTES, MIP-1α, MIP-1β, which are endogenous ligands, the assay system using these endogeneous ligands are available.

Specifically, e.g. since CCR5 is a G protein-coupled seven transmembrane type receptor, an assay system in which the effect of RANTES on the transient increase of Ca ion induced via CCR5 is measured is applicable for screening a compound which antagonizes binding of RANTES and CCR5.

Besides, as an assay system to measure CCR1 antagonism activity, an assay system in which the antagonism activity of each test compound is measured using a known method *(European Journal of Pharmacology,* 389, 41-49 (2000)), e.g. an evaluation method using THP-1 cells, is applicable.

Toxicity:

The compounds of the present invention have very low toxicity and therefore it is judged that they are safe enough for pharmaceutical use.

Application to pharmaceuticals:

Since the compound of formula (I) of the present invention antagonizes chemokine receptors (especially CCR1 and/or CCR5) in animals including human, especially human, it is used for prevention and/or treatment of those diseases induced by chemokine receptors, e.g. immune diseases such as various inflammatory diseases, autoimmune diseases, allergy; infections concerning inflammation or HIV infection (e.g. asthma, nephritis, nephropathy, hepatitits, arthritis, rheumatoid arthritis, rhinitis, conjunctivitis, ulcerative colitis, *etc*., organ rejection in transplantation, immunosuppression, psoriasis, multiple sclerosis, optic neuritis, polymyalgia rheumatica, uveitis, angiitis, human immunodeficiency virus infection (acquire immune deficiency syndrome), atopic dermatitits, urticaria, allergic bronchopulmonary aspergillosis, allergic eosinophilic gastroenteritis, osteoarthritis, ischemic reperfusion injury, acute respiratory distress syndrome, cytotoxic shock, diabetes, cancer metastasis, and atherosclerosis.

The compound of formula (I) of the present invention or a salt thereof has inhibitory effect against the infection of HIV-1, which has acquired tolerance to other drugs for preventing and/or treating HIV infection (especially the drugs for the preventing and/or treating AIDS). Therefore, the compound of formula (I) is applicable to HIV-infected patients to whom other drugs for preventing and/or treating HIV infection have become ineffective. In this case, the compound of the present invention may be administered by itself, or it may be administered together with the drug to which the infected HIV-1 mutant has acquired tolerance or other drugs.

The compound of formula (I), a salt thereof or a prodrug thereof may also be administered as a concomitant agent in combination with other agents for

1) supplementing and/or reinforcement of preventive and/or treating effect(s) of the compound,
2) improvement in kinetics and absorption of the compound and reduction of dose and/or
3) reduction of side effect of the compound.

Also, the concominant drug may be used for the purpose of

1) supplementing and/or reinforcement of preventive and/or treating effect(s) of the compound for the combination use,
2) improvement in kinetics and absorption of the compound for the combination and reduction of dose and/or
3) reduction of a side effect of the compound for the combination use.

Concomitant agents of the compound of formula (I) with other agents may be administered in a mode of compounded agent in which both components are compounded in a single preparation or in a mode of separate preparations. When administration is conducted using separate preparations, a simultaneous administration and administrations with time difference are included. In the case of administrations with time difference, the compound of formula (I) may be firstly administered and then the other drug may be administered, and vice versa. Each of the methods for the administration may be the same or different.

The diseases which the above concomitant drugs show a preventive and/or treating effect are not limited in particular, but whatever supplement and/or reinforce the preventive and/or treating effect of the compound of formula (I) would be acceptable.

Examples of the other drug for supplementing and/or reinforcing the preventive and/or treating effect of the compound of formula (I) against HIV infection or preventive or treating effect of AIDS include, for example, reverse transcriptase inhibitors, protease inhibitors, chemokine antagonists (e.g. CCR1 antagonists, CCR2 antagonists, CCR3 antagonists, CCR4 antagonists, CCR5 antagonists, CXCR4 antagonists, *etc.*), fusion inhibitors, HIV integrase inhibitors, antibody against antigen on the surface of HIV-1, HIV-1 vaccines, *etc.*

Other drugs for supplementing and/or reinforcing the preventive and/or treating effect of the compound of formula (I) for organ rejection in transplantation include, for example, immunosuppressants, *etc.*

Other drugs for supplementing and/or reinforcing the preventive and/or treating effect of the compound of formula (I) for multiple sclerosis include, for example, a steroidal drug, interferon, an immunosuppressant, a chemokine inhibitor, an aldose reductase inhibitor, a cannabinoid-2 receptor agonist, adrenocorticotropic hormone, *etc.*

Other drugs for supplementing and/or reinforcing the preventive and/or treating effect of the compound of formula (I) for multiple sclerosis include, for example, a metalloproteinase inhibitor, an immunosuppressant, a chemokine inhibitor, a nonsteroidal anti-inflammatory drug (NSAID), a steroidal drug, prostaglandins, a phosphodiesterase inhibitor, a cannabinoid-2 receptor agonist, a disease-modifying anti-rheumatoid drug , a T-cell inhibitor, a TNF-$\alpha$ inhibitor, an IL-6 inhibitor, an interferon $\gamma$ agonist, an IL-1 inhibitor or an NF-$\kappa$B inhibitor, *etc.*

Concominat drugs of the compound of the present invention and the drugs of the above mechanism may be selected from one kind or more. When selecting more than one kind of drug, it may be selected from the same mechanism or from different mechanisms.

Reverse transcriptase inhibitors are concretely (1) nucleoside/nucleotide reverse transcriptase inhibitors : zidovudine (brand name: Retrovir), didanosine (brand name: Videx), zalcitabine (brand name: HIVID), stavudine (brand name: Zerit), lamivudine (brand name: Epivir), abacavir (brand name: Ziagen), adefovir, adefovir dipivoxil, emtricitabine (brand name: Coviracil), PMPA (brand name: Tenofovir), *etc.* and (2) non-nucleoside reverse transcriptase inhibitors: nevirapine (brand name: Viramune), delavirdine (brand name: Rescriptor), efavirenz (brand name: Sustiva, Stocklin) or capravirine (AG1549), *etc.*

Protease inhibitors include, specifically, indinavir (brand name: Crixivan), ritonavir (brand name: Norvir), nelfinavir (brand name: Viracept), saquinavir (brand name: Invirase, Fortovase), amprenavir (brand name: Agenerase), lopinavir (brand name: Kaletra), tipanavir, *etc.*

Chemokine antagonists include, endogeneous ligands of chemokine receptors, derivatives thereof, non-peptide low-molecular compounds and antibodies to chemokine receptors.

Endogenous ligands of chemokine receptors include, specifically, MIP-1$\alpha$, MIP-1$\beta$, RANTES, SDF-1$\alpha$, SDF-1$\beta$, MCP-1, MCP-2, MCP-4, Eotaxin, MDC, *etc.*

Derivatives of endogenous ligands include, specifically, AOP-RANTES, Met-SDF-1$\alpha$, Met-SDF-1$\beta$, *etc.*

Antibodies of chemokine receptors include, specifically, Pro-140 *etc.*

CCR1 antagonists include, specifically, the compounds described in WO98/04554, WO98/38167, WO99/40061, WO00/14086, WO00/14089, WO01/72728, JP2002-179676, WO02/036581, WO03/013656, WO03/035627, WO03/035037 or BX-471, *etc.*

CCR2 antagonists include, specifically, the compounds described in WO99/07351, WO99/40913, WO00/46195, WO00/46196, WO00/46197, WO00/46198, WO00/46199, WO00/69432, WO00/69815 or *Bioorg. Med Chem. Lett.,* 10, 1803 (2000), *etc.*

CCR3 antagonists include, specifically, the compounds described in DE19837386, WO99/55324, WO99/55330, WO00/04003, WO00/27800, WO00/27835, WO00/27843, WO00/29377, WO00/31032, WO00/31033, WO00/34278, WO00/35449, WO00/35451, WO00/35452, WO00/35453, WO00/35454, WO00/35876, WO00/35877, WO00/41685, WO00/51607, WO00/51608, WO00/51609, WO00/51610, WO00/53172, WO00/53600, WO00/58305, WO00/59497, WO00/59498, WO00/59502, WO00/59503, WO00/62814, WO00/73327 or WO01/09088, *etc.*

CCR4 antagonists include, specifically, the compounds described in WO02/030357, WO02/030358, WO02/094264, WO03/051870 or WO03/059893, *etc.*

CCR5 antagonists include, specifically, the compounds described in WO99/17773, WO99/32100, WO00/06085, WO00/06146, WO00/10965, WO00/06153, WO00/21916, WO00/37455, EP1013276, WO00/38680, WO00/39125, WO00/40239, WO00/42045, WO00/53175, WO00/42852, WO00/66551, WO00/66558, WO00/66559, WO00/66141, WO00/68203, TP-A-2000-309598, WO00/51607, WO00/51608, WO00/51609, WO00/51610, WO00/56729, WO00/59497, WO00/59498, WO00/59502, WO00/59503, WO00/76933, WO98/25605, WO99/04794, WO99/38514, *Bioorg. Med Chem. Lett.*, 10, 1803 (2000), TAK-779, SCH-351125(SCH-C), SCH-417690(SCH-D), UK-427857, GW 873140A, TAK-220, *etc.*

CXCR4 antagonists include, specifically, AMD-3100, T-22, KRH-1120 or the compounds described in WO00/66112, *etc.*

Fusion inhibitors include, specifically, T-20 (pentafuside), T-1249, *etc.*

HIV integrase inhibitors include, Equisetin, Temacrazine, PL-2500, V-165, NSC-618929, L-870810, L-708906 analogue, S-1360, 1838, *etc.*

The above drugs for combination use are referred to as examples and therefore the present invention is not limited thereto.

For example, the following shows normal clinical doses of representative reverse transcriptase inhibitors and protease inhibitors, but the present invention is not limited thereto.

| | |
|---|---|
| zidovudine: | 100 mg capsule, 200 mg per dose, 3 times per day; 300 mg tablet, 300 mg per dose, twice per day; |
| didanosine: | 25-200 mg tablet, 125-200 mg per dose, twice per day; |
| zalcitabline: | 0.375-0.75 mg tablet, 0.75 mg per dose, 3 times per day; |
| Stavudine: | 15-40 mg capsule, 30-40 mg per dose, twice per day; |
| kamivudine: | 150 mg tablet, 150 mg per dose, twice per day; |
| abacavir: | 300 mg tablet, 300 mg per dose, twice per day; |
| nevirapine: | 200 mg tablet, 200 mg per dose, once per day for 14 days and then twice per day; |
| delavirdine: | 100 mg tablet, 400 mg per dose, three times per day; |
| efavirenz: | 50-200 mg capsule, 600 mg per dose, once per day; |
| indinavir: | 200-400 mg capsule; 800 mg per dose, three times per day; |
| ritonavir: | 100 mg capsule, 600 mg per dose, twice per day; |
| nelfinavir: | 250 mg tablet, 750 mg per dose, three times per day; |
| saquinavir: | 200 mg capsule, 100 or 200 mg per dose, three times per day; |
| amprenavir: | 50-150 mg tablet, 100 or 200 mg per dose, twice per day. |

Immunosuppressants include, for example, cyclosporin (calcineurin inhibitor), or tacrolimus (FK506), sirolimus (rapamycin, TOR inhibitor), corticosteroid (non-specific antiinflammatory agent), azatiopurine (DNA synthesis inhibitor), mycophenolate mofetil (purine de novo synthesis inhibitor), methotrexate, ascomycin, leflunomide, bucillamine, salazosulfapyrizine, *etc.*

Steroidal drugs include, for example, as external medicines, clobetasol propionate, diflorasone acetate, fluocinonide, mometasone furoate, betamethasone dipropionate, betamethasone butyrate propionate, betamethasone valerate, difluprednate, budesonide, diflucortolone valerate, amcinonide, halcinonide, dexamethasone, dexamethasone propionate, dexamethasone valerate, dexamethasone acetate, hydrocortisone acetate, hydrocortisone lactate, hydrocortisone butyrate propionate,deprodone propionate, prednisolone valerate acetate, fluocinolone acetonid, beclomethasone dipropionate, triamcinolone acetonid, flumethasone pivalate, alclometasone dipropionate, beclomethasone lactate, prednisolone, beclomethasone dipropionate, fludroxycortide, *etc.*

Internal medicines and injections include, for example, cortisone acetate, hydrocortisone, sodium hydrocortisone phosphate, sodium hydrocortisone succinate, fludrocortisone acetate, prednisolone, prednisolone acetate, sodium prednisolone succinate, butyl prednisolone acetate, prednisolone sodium phosphate, halopredone acetate, methyl prednisolone, methyl prednisolone acetate, sodium methyl prednisolone succinate, triamcinolone, triamcinolone acetate, triamcinolone acetonid, dexamethasone, dexamethasone acetate, sodium dexamethasone phosphate, dexamethasone palmitate, paramethasone acetate, betamethasone, *etc.*

Inhalant medicines include, for example, beclometasone dipropionate, fluticasone propionate, budesonide, flunisolide, triamcinolone, ST-126P, ciclesonide, dexamethasone paromitionate, mometasone furoate, prasterone sulfonate, deflazacort, methylprednisolone, sleptanate, methylprednisolone sodium succinate, *etc.*

Non-steroidal anti-inflammatory drugs include, for example, sasapyrine, sodium salicylate, aspirin, aspirin dialuminate, diflunisal, indomethacin, suprofen, ufenamate ,dimethylisopropylazulene, bufexamac, felbinac, dichlofenac, tolmetin sodium, clinoril, fenbufen, nabumetone, proglumetacin, indometacin famesil, acemetacin, proglumetacin maleate, amfenac sodium, mofezolac, etodolac, ibuprofen, ibuprofenpiconol, naproxen, flurbiprofen, flurbiprofen axetil, ketoprofen, phenoprofen calcium, tiaprafenic acid, oxaprozin, pranoprofen, loxoprofen sodium, alrninoprofen, zaltoprofen, mefenamic acid, aluminum mefenamate, tolfenamic acid, floctafenine, ketophenylbutazon, oxyfenbutazon, piroxicam, tenoxicam, ampiroxicam, napageln ointment, epirizole, tiaramide hydrochloride, tinoridine hydrochloride, emorfazone, sulpyrine, migrenin, saridon, cedes G, amipylo-N, sorbon, pyrine cold preparation, acetoaminofen, fenacetin, dimetotiazine mesilate, simetride, non-pyrine cold preparation, *etc.*

Prostaglandins (abbreviated as PG hereafter) include, PG receptor agonist, PG receptor antagonist, *etc.* PG receptors include, PGE receptor ($EP_1$, $EP_2$, $EP_3$, $EP_4$), PGD receptor (DP, CRTH2), PGF receptor (FP), PGI receptor (IP), TX receptor (TP), *etc.*

Phosphodiesterase inhibitors include, for example, rolipram, cilomilast (brand name: Ariflo), Bay19-8004, NIK-616, Roflumilast (BY-217), cipamfylline (BRL-61063), atizoram (CP-80633), SCH-351591, YM-976, V-11294A, PD-168787, D-4396, IC-485, *etc.*

Disease-modifying antirheumatic drugs (slow-acting antirheumatic drugs) include, for example, aureus thioglucose, aureus thiomalate sodium, auranofin, actarit, D-penicillamine preparations, lobenzarit disodium, bucillamine, hydroxychloroquine sulphate, salazosulfapyridine, *etc.*

Antiinflammatory enzyme drugs include, for examples, lysozyme chloride, bromelain, pronase, serrapeptase, streptokinase-streptodornase combination drug, *etc.*

Cartilage-protecting agents include, for example, sodium hyaluronate, glucosamine, chondroitin sulfate, glycosaminoglycan sulfate, *etc.*

There is no limitation for the ratio by weight of the compound of formula (I) to other agents. With regard to other agents, two or more members of any agent may be administered in combination. Such other agents which supplement and/or reinforce the preventive and/or treating effect of the compound include not only those which have been found on the basis of the above-mentioned mechanism but also those which will be found in the future.

The compound of formula (I), a salt thereof, a combination of the compound or a salt thereof and other drugs are generally administered systemically or topically and orally or parenterally when used for the above objects.

The dosages are determined depending on age, body weight, symptom, therapeutic effect, administration route, duration of the treatment and the like. Generally, 1 μg to 1000 mg per adult is orally administered once to several times per day, or 100 ng to 100 mg, per adult is parenterally administered once to several times per day, or continuously administered from vein for 1 to 24 hours per day. Since the dosage changes depending on various conditions as described above, there are cases in which doses lower than or greater than the above ranges may be used.

When the compound of formula (I), a salt thereof, a combination of the compound or a salt thereof and other drugs may be administered in the form of solid compositions, liquid compositions and other compositions for oral administration, and injections, external medicine, suppositories, eye lotions, inhalants and the like for parenteral administration.

Solid compositions for oral administration include tablets, pills, capsules, dispersible powders, granules and the like. Capsules include hard capsules and soft capsules. In such solid compositions, one or more active compound(s) are mixed with one or more of excipients (lactose, mannitol, glucose, microcrystalline cellulose, starch, *etc.*), binding agent (hydroxypropylcellulose, polyvinylpyrrolidone, magnesium aluminometasilicate, *etc.*), disintegrating agent (calcium glycolate cellulose, *etc.),* lubricating agents (magnesium stearate *etc.*), stabilizer, agents to assist resolution (glutamic acid, aspartic acid, *etc.), etc.* and are formulated by known methods. If necessary, the solid compositions may be coated with film of gastric- or enteric- coating agents (e.g. sugar, gelatin, hydroxypropyl cellulose and hydroxypropyl cellulose phthalate, *etc.*), or be coated with two or more films. Furthermore, capsules of absorbable materials such as gelatin are included. Liquid compositions for oral administration include pharmaceutically acceptable aqueous solutions, suspensions, emulsions, syrups, elixirs and the like. In such liquid compositions, one or more active compound(s) are dissolved, suspended or emulsified in an inert diluent commonly used (e.g., purified water, ethanol or a mixture thereof, *etc.*). Liquid compositions may further contain wetting agents, suspending agents, emulsifying agents, sweetening agents, flavoring agents, aromatic agents, preserving agents, buffering agents, *etc.*

Formulations of external medicines for parenteral administration include, for example, ointments, gels, creams, poultices, adhesive preparations, liniments, air sprays, inhalants, sprays, eye drops, nasal preparations, *etc.* These contain one or more of active substance and they may be formulated by known methods or usually used prescriptions. Ointments may be prepared by known or usually used prescriptions, for example, by levigating or fusing one or more of active substance as a base. An ointment base is selected from known or usually used ones, for example, higher fatty acids or higher fatty acid esters (adipic acid, myristic acid, palmitic acid, stearic acid, oleic acid, adipic acid ester, myristic acid ester, palmitic acid ester, stearic acid ester, oleic acid ester, *etc.),* wax (bees wax, whale wax, ceresin wax, *etc.*), surfactants (polyoxyethylenealkylether phosphate *etc.*), higher alcohols (cetanol, stearylalcohol, cetostearylalcohol, *etc.),* silicon oil (dimethylpolysiloxane *etc.*), hydrocarbons (hydrophilic petrolatum, white petrolatum, purified lanoline, liquid paraffin, *etc.),* glycols (ethyleneglycol, diethyleneglycol, propyleneglycol, polyethyleneglycol, macrogol, *etc.),* salad oil (castor oil, olive oil, sesame oil, terepin oil, *etc.),* animal oil (mink oil, vitellus oil, squalane, squalene, *etc.*), water, absorption promoter, irritation preventives. Also, ointments may further contain moisturizing agents, preserving agents, stabilizing agents, antioxidants, aromatic agents, *etc.*

Gels may be prepared by known methods or usually used prescriptions, for example, one or more of active substance is fused in a base. Gel bases are selected from known or usually used ones, for example, one or more selected from lower alcohol (ethanol, isopropylalcohol, *etc.*), gelling agents (carboxymethylcellulose, hydroxyethylcellulose, hydroxypropylcellulose, ethylcellulose, *etc.*), neutralizing agents (triethanolamine, diisopropanolamine, *etc.),* surfactants (polyethyleneglycol monostearate *etc.*), gums, water, absorption promoters, irritation preventives. Gels may further contain preserving agents, antioxidants, aromatic agents, *etc.*

Creams may be prepared by known methods or usually used prescriptions. For example, they may be prepared by fusing or emulsifying one or more of active substance in a base. Cream bases are selected from known or usually used ones, for example, one or more selected from higher fatty acid esters, lower alcohols, hydrocarbons, multiple alcohols (propyleneglycol, 1,3-butyleneglycol, *etc.*), higher alcohols (2-hexyldecanol, cetanol, *etc.*), emulsifying agents

(polyoxyethylenealkyl ether, fatty acid ester, *etc.*), water, absorption promoters, irritation preventives, *etc*. Creams may further contain preserving agents, antioxidants, aromatic agents, *etc*.

Poultices may be prepared by known methods or usually used prescriptions. For example, one or more of active substance is fused in a base, and the resulting paste mixture is beat out and coated over support medium. The poultices base is selected from known or usually used ones. For example, one or more selected from thickening agents (polyacrylic acid, polyvinylpyrrolidone, Arabia gum, starch, methylcellulose, *etc.*), wetting agents (urea, glycerin, propyleneglycol, *etc.*), bulking agents (kaolin, zinc oxide, talc, calcium, magnesium, *etc.*), water, solubilizing agents, agents to assist adhesion and irritation preventives. Poultices may further contain preserving agents, antioxidants, aromatic agents, *etc*.

Adhesive preparations may be prepared by known methods or usually used prescriptions. For example, one or more active substance is fused in a base, and it is beat out and coated over support medium. The bases for adhesive preparations are selected from known or usually used ones, for example, one or more selected from macromolecule base, grease, higher fatty acid, agents to assist adhesion, irritation preventives. And they may further include preserving agents, antioxidants, aromatic agents, *etc.*

Liniments may be prepared by known methods or usually used prescriptions. For example, liniments may be prepared by dissolving, suspending or emulsifying one or more of active substance in one or more selected from water, alcohol (ethanol, polyethyleneglycol, *etc.),* higher fatty acid, glycerin, soaps, emulsifying agents, suspending agents, *etc*. Liniments may further contain preserving agents, antioxidants, aromatic agents, *etc.*

Air sprays, inhalants and sprays contain, stabilizing agents such as sodium hydrogen sulfate, buffering agents to give isotonicity, i.e. isotonic solutions such as sodium chloride, sodium citrate or citric acid, except inert diluents. The processes for preparing sprays are, for example, described in U.S. Patents 2,868,691 and 3,095,355.

Injections for parenteral administration include all injections; e.g. muscle injection, intravenous injection, intravenous drip, *etc.*

Injections for parenteral administration include solutions, suspensions, emulsions and solid composition for injection to be dissolved before use. Injections may be used by dissolving, suspending or emulsifying one or more active substance in a solvent. Solvents contain, for example, distilled water for injection, physiological saline, vegetable oil, alcohols (e.g. propyleneglycol, polyethyleneglycol, ethanol, *etc.*), and combinations thereof. The injections may further contain stabilizing agents, solubilizing agents (e.g. glutamic acid, aspartic acid, polysorbate 80 (registered trademark), suspending agents, emulsifying agents, soothing agents, buffering agents, preserving agents, *etc*. They are sterilized in the final step or prepared by aseptic manipulation. Otherwise, sterile solid compositions, for example, freeze-dried products are manufactured and they may be sterilized or dissolved in sterile purified water or other solvents before use.

The inhalants for parenteral administration include aerosols, powders for inhalation, and liquids for inhalation, and the liquids for inhalation may be in the form which is dissolved or suspended in water or an appropriate medium before use. These inhalations can be prepared according to known methods. For example, liquids for inhalation can be prepared, if necessary, by appropriately selecting from preserving agents (e.g. benzalkonium chloride, paraben, *etc.*), coloring agents, buffering agents (e.g. sodium phosphate, sodium acetate, *etc.*), isotonizing agents (e.g. sodium chloride, concentrated glycerin, *etc.*), thickening agents (e.g. carboxyvinyl polymer, *etc.),* absorption promoters, and the like. Powders for inhalation can be prepared, if necessary, by appropriately selecting from lubricating agents (e.g. stearic acid, salts thereof, *etc.*), binding agents (e.g. starch, dextrin, *etc.*), excipients (e.g. lactose, cellulose, *etc.*), coloring agents, preserving agents (e.g. benzalkonium chloride, paraben, *etc.*), absorption promoters, and the like.

When the liquids for inhalation are administered, sprayers (e.g., atomizer, nebulizer) is usually used. When the powders for inhalation are administered, inhalation administration apparatus for powder agents is usually used.

Other compositions for parenteral administration include suppositories for intrarectal administration, pessaries for intravaginal administration and the like containing one or more of active substance, which can be prescribed by known methods.

Effect of the invention:

The compound of formula (I) of the present invention, a salt thereof or a prodrug thereof antagonizes chemokine receptors (particularly, CCR1 and/or CCR5) and therefore, it is useful for the prevention and/or treatment of those diseases induced by chemokine receptors, e.g. various types of inflammation, autoimmune diseases, allergic diseases, *etc.*; infection concerning inflammation or HIV infections (e.g. asthma, nephritis, nephropathy, hepatitis, arthritis, rheumatoid arthritis, rhinitis, conjunctivitis, ulcerative colitis, *etc.*), organ transplantation rejection, immunosuppression, psoriasis, multiple sclerosis, optic neuritis, polymyalgia rheumatica syndrome, uveitis, vasculitis, human immunodeficiency virus infection (acquired immunodeficiency syndrome *etc.*), atopic dermatitis, urticaria, allergic bronchopulmonary aspergillosis, allergic eosinophilic gastroenteritis, osteoarthritis, ischemic reperfusion injury, acute respiratory distress syndrome, shock accompanying bacteria infection, diabetes, cancer metastasis, atherosclerosis, *etc.*).

Best Mode For Carrying Out The Invention

The present invention is illustrated by the following reference examples and examples, but it is not limited thereto.

The nomenclature in the present invention was carried out according to ACD/Name (Brand Name; Advanced Chemistry Development Inc.), which generates nomenclature of IUPAC rules.

For example, the compound represented by

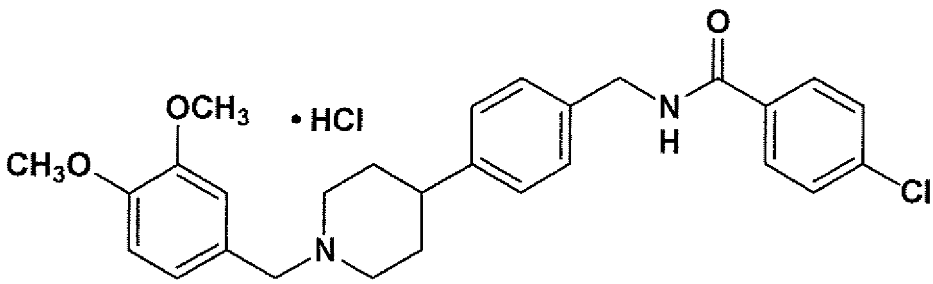

was named 4-chloro-N-{4-[1-(3,4-dimethoxybenzyl)piperidin-4-yl]benzyl}benzamide hydrochloride.

The solvents in the parentheses show the developing or eluting solvents and the ratios of the solvents used are by volume in chromatographic separations or TLC.

The solvents in the parentheses in NMR show the solvents for measurement.

The measurement of HPLC was carried out by the following conditions unless specified.

| | |
|---|---|
| Column: | Xterra (registered trademark) MS $C_{18}$ 5 μm, 4.6 × 50 mm I.D. |
| Rate of flow: | 3 ml / min |
| Solvent | solution A: 0.1% aqueous solution of trifluoroacetic acid solution B: 0.1% solution of trifluoroacetic acid-in acetonitrile |

For 0.5 minute after starting measurement, the ratio of solution A and solution B was fixed at 95/5. Then the ratio of liquid A and liquid B was shifted to 0/100 over a period of 2.5 minutes linearly. Then the ratio of liquid A and liquid B was fixed at 0/100 for 0.5 minute. Then the ratio of liquid A and liquid B was shifted to 95/5 over a period of 0.01 minute linearly.

Reference Example 1

4-[4-(azidomethyl)phenyl]piperidine hydrochloride

Using t-butyl 4-oxopiperidin-1-carboxylate, lithium diisopropylaldehyde and N,N-bis(trifluoromethylsulfonyl)anilide were subjected to a reaction, t-butyl 4-{[(trifluoromethyl)sulfonyl]oxo}-3,6-dihydropyridin-1(2H)-carboxylate was given. In the presence of tetrakis(triphenylphosphine)palladium(0), thus given compound and (4-formylphenyl)boronic acid were subjected to a reaction, and t-butyl 4-(4-formylphenyl)-3,6-dihydropyridin-1(2H)-carboxylate was given. The given compound was reduced in the presence of palladium-carbon by hydrogen gas to give t-butyl 4-[4-(hydroxymethyl)phenyl]piperidin-1-carboxylate. In carbon tetrachloride, the resulting compound was subjected to a reaction with triphenylphosphine, t-butyl 4-[4-(chloromethyl)phenyl]piperidin-1-carboxylate was given. By subjecting to a reaction the resulting compound and sodium azide t-butyl 4-[4-(azidomethyl)phenyl]piperidin-1-carboxylate was given. The given compound was subjected to a deprotection reaction by 4N hydrochloric acid-ethyl acetate to give the title compound having the following physical data.

TLC:Rf 0.20 (chloroform : methanol : 28% ammonia water = 8 : 1 :0.1).

Reference Example 2

4-[4-(azidomethyl)phenyl]-1-(3,4-dimethoxybenzyl)piperidine

To a solution of the compound prepared in reference example 1 (3.78 g) in 1,2-dichloroethane (150 ml) were added 3,4-dimethoxybenzaldehyde (2.49 g) and diisopropylethylamine (2,87 ml). To the mixture was added sodium triacetoxyborohydride (6.31 g). The reaction mixture was stirred overnight at room temperature. To the mixture was

added water and the resulting mixture was extracted with ethyl acetate. The extract was dried over anhydrous sodium sulfate and concentrated to give the title compound (5.64 g) having the following physical data.
TLC:Rf 0.45 (dichloromethane : methanol = 10 : 1).

Reference Example 3

{4-[1-(3,4-imethoxybenzyl)piperidin-4-yl]benzyl}amine

To a solution of the compound prepared in reference example 2 (74 mg) in tetrahydrofuran (4 ml) was added polystyrene-supported triphenylphosphine (38 mg). The reaction mixture was stirred for 6 hours at room temperature. To the reaction mixture was added water (0.11 ml) and the resulting mixture was stirred overnight at room temperature. The reaction mixture was filtered. The filtrate was concentrated to give the title compound (63 mg) having the following physical data.
TLC:Rf 0.12 (dichloromethane : methanol = 10 : 1).

Example 1

4-chloro-N-{4-[1-(3,4-dimethoxybenzyl)piperidin-4-yl]benzyl}benzamide hydrochloride

To a solution of the compound prepared in reference example 3 (63 mg) and pyridine (0.018 ml) in tetrahydrofuran (2 ml) was added 4-chlorobenzoyl chloride (0.028 ml). The reaction mixture was stirred for 1 hour at room temperature. To the reaction mixture was added a saturated aqueous solution of ammonium chloride and extracted with ethyl acetate. The extract was washed with a saturated aqueous solution of sodium bicarbonate, water, a saturated aqueous solution of sodium chloride and dried over anhydrous magnesium sulfate. The filtrate was concentrated and the resulting residue was purified by column chromatography on silica gel (ethyl acetate : methanol = 10 : 1). To a solution of the resulting compound in ethyl acetate was added 4N solution of hydrogen chloride in ethyl acetate. The mixture was concentrated to give the compound of the present invention (38 mg) having the following physical data.
TLC:Rf0.23 (dichloromethane : methanol = 10 : 1);
NMR($CD_3OD$):$\delta$ 7.83-7.80 (m, 2H), 7.48-7.44 (m, 2H), 7.37-7.29 (m, 2H), 7.23-7.17 (m, 3H), 7.10-7.02 (m, 2H), 4.52 (s, 2H), 4.27 (s, 2H), 3.88 (s, 3H), 3.85 (s, 3H), 3.60-3.50 (m, 2H), 3.20-3.00 (m, 2H), 2.85 (m, 1H), 2.04-1.98 (m, 4H).

Example 1(1) and 1(2)

By the same procedure as described in reference example 2 → reference example 3 → example 1 using a corresponding azide compound in place of 4-[4-(azidomethyl)phenyl]piperidine hydrochloride, the following compounds of the present invention were given.

Example 1(1)

4-chloro-N-{3-[4-(3,4-dimethoxybenzyl)piperazin-1-yl]benzyl}benzamide dihydrochloride

TLC:Rf 0.32 (dichloromethane : methanol = 10 : 1);
NMR($CD_3OD$): $\delta$ 7.84-7.81 (m, 2H), 7.48-7.45 (m, 2H), 7.29-7.18 (m, 2H), 7.12-7.05 (m, 3H), 7.04-6.94 (m, 2H), 4.52 (s, 2H), 4.34 (s, 2H), 3.88 (s, 3H), 3.86 (s, 3H), 3.90-3.80 (m, 2H), 3.60-3.50 (m, 2H), 3.40-3.20 (m, 2H), 3.20-3.00 (m, 2H).

Example 1(2)

4-chloro-N-{4-[1-(3,4-dimethoxybenzyl)-4-hydroxypiperidin-4-yl]benzyl}benzamide hydrochloride

TLC:Rf0.20 (dichloromethane : methanol = 10 : 1);
NMR($CD_3OD$): $\delta$ 7.81 (d, J = 9.0 Hz, 2H), 7.48-7.02 (m, 9H), 4.54 (s, 2H), 4.31 (s, 2H), 3.88 (s, 3H), 3.86 (s, 3H), 3.50-3.20 (m, 4H), 2.40-2.10 (m, 2H), 2.00-1.80 (m, 2H).

Example 2

N- {3 -[1-(4-methoxybenzyl)piperidin-4-yl]benzyl}-4-(methylthio)benzamide

{3-[1-(4-methoxybenzyl)piperidin-4-yl]benzyl}amine (given by the same procedure as described in reference

example 2 → reference example 3 using 4-[3-(azidomethyl)phenyl]piperidine hydrochloride and 4-methoxybenzaldehyde, and 4-(methylthio)benzoic acid) was subjected to a reaction using polystyrene-supported carbodiimide in the presence of 1-hydroxybenzotriazole, and purified by polystyrene-supported trisamine, further purified by high performance liquid chromatography, the compound of the present invention was given.
HPLC retention time (min.):3.47;
NMR($CD_3OD$): δ 7.76 (d, J=6.6 Hz, 2H), 7.80-7.21 (m, 6H), 7.16-7.10 (m, 2H), 6.87 (d, J=6.6 Hz, 2H), 4.52 (s, 2H), 3.77 (s, 3H), 3.51 (s, 2H), 3.02-2.98 (m, 2H), 2.58-2.43 (m, 1H), 2.49 (s, 3H), 2.20-2.05 (m, 2H), 1.80-1.75 (m, 4H).

<u>Example 2(1) - 2(240)</u>

By the same procedure as described in example 2 using a corresponding amine derivative in place of {3-[1-(4-methoxybenzyl)piperidin-4-yl]benzyl}amine and a corresponding carboxylic acid in place of 4-(methylthio)benzoic acid, the following compounds of the present invention were given.

<u>Example 2(1)</u>

N-{3-[1-(3,4-dimethoxybenzyl)piperidin-4-yl]benzyl}-4-(methylthio)benzamide

HPLC retention time (min.):3.44;
NMR($CDCl_3$): δ 7.69 (d, J=7.8 Hz, 2H), 7.31-7.16 (m, 6H), 6.92 (s, 1H), 6.92-6.82 (m, 2H), 6.34 (m, 1H), 4.61 (d, J=6.0 Hz, 2H), 3.90 (s, 3H), 3.87 (s, 3H), 3.49 (s, 2H), 3.02-2.98 (m, 2H), 2.55-2.25 (m, 1H), 2.50 (s, 3H), 2.10-2.05 (m, 2H), 1.83-1.79 (m, 4H).

<u>Example 2(2)</u>

N-(3-{1-[4-(methylsulfonyl)benzyl]piperidin-4-yl}benzyl)-4-(methylthio)benzamide

HPLC retention time (min.):3.34;
NMR($CDCl_3$): δ 7.89 (d, J=8.1 Hz, 2 H), 7.70 (d, J=8.1 Hz, 2H), 7.56 (d, J=8.1 Hz, 2H), 7.32-7.16 (m, 6H), 6.38 (m, 1H), 4.62 (d, J=5.4 Hz, 2H), 3.61 (s, 2H), 3.05 (s, 3H), 2.98-2.94 (m, 2H), 2.60-2.45 (m, 1H), 2.50 (s, 3H), 2.17-2.08 (m ,2H), 1.83-1.76 (m, 4H).

<u>Example 2(3)</u>

N-{3-[1-(4-chlorobenzyl)piperidin-4-yl]benzyl}-4-(methylthio)benzamide

HPLC retention time (min.):3.55;
NMR($CD_3OD$): δ 7.77 (d, J=8.4 Hz, 2H), 7.33-7.21 (m, 8H), 7.17 (s, 1H), 7.13 (t, J=7.8 Hz, 1H), 4.53 (s, 2H), 3.57 (s, 2H), 3.02-2.98 (m, 2H), 2.60-2.45 (m, 1H), 2.50 (s, 3H), 2.25-2.15 (m, 2H), 1.85.-1.74 (m, 4H).

<u>Example 2(4)</u>

4-fluoro-N-{3-[1-(4-methoxybenzyl)piperidin-4-yl]benzyl}benzamide

HPLC retention time (min.):3.40;
NMR($CD_3OD$): δ 7.91-7.86 (m, 2H), 7.23-7.09 (m, 8H), 6.89 (d, J=8.4 Hz, 2H), 4.53 (s, 2H), 3.76 (s, 3H), 3.51 (s, 2H), 3.02-2.98 (m, 2H), 2.60-2.45 (m, 1H), 2.18-2.09 (m, 2H), 1.79-1.71 (m, 4H).

<u>Example 2(5)</u>

4-fluoro-N-(3-{1-[4-(methylsulfonyl)benzyl]piperidin-4-yl}benzyl)benzamide

HPLC retention time (min.):3.25;
NMR($CDCl_3$): δ 7.89 (d, J=8.1 Hz, 2H), 7.82-7.79 (m, 2H), 7.57 (d, J=8.1 Hz, 2H), 7.28 (m, 2H), 7.21-7.17 (m, 3H), 7.09 (t, J=8.1 Hz, 1H), 6.42 (m, 1H), 4.61 (d, J=5.7 Hz, 2H), 3.62 (s, 2H), 3.05 (s, 3H), 2.98-2.94 (m, 2H), 2.60-2.45 (m, 1H), 2.17-2.08 (m, 2H), 1.82-1.70 (m, 4H).

Example 2(6)

N-{3-[1-(4-chlorobenzyl)piperidin-4-yl]benzyl}-4-fluorobenzamide

HPLC retention time (min.):3.47;
NMR($CDCl_3$): $\delta$ 7.81-7.73 (m, 2H), 7.31-7.26 (m, 4H), 7.20-7.15 (m, 5H), 7.10 (t, J=8.1 Hz, 1H), 6.32 (m, 1H), 4.61 (d, J=5.7 Hz, 2H), 3.52 (s, 2H), 3.00-2.96 (m, 2H), 2.60-2.40 (m, 1H), 2.15-2.04 (m, 2H), 1.80-1.70 (m, 4H).

Example 2(7)

N-{{3-[1-(3,4-dimethoxybenzyl)piperidin-4-yl]benzyl}-4-fluorobenzamide

HPLC retention time (min.):3.36;
NMR($CDCl_3$): $\delta$ 7.82-7.77 (m, 2H), 7.31-7.26 (m, 2H), 7.21-7.17 (m, 3H), 7.13 (t, J=8.4 Hz, 1H), 6.93 (s, 1H), 6.93-6.82 (m, 2H), 6.33 (m, 1H), 4.61 (d, J=5.4 Hz, 2H), 3.90 (s, 3H), 3.87 (s, 3H), 3.53 (s, 2H), 3.06-3.02 (m, 2H), 2.60-2.45 (m, 1H), 2.18-2.10 (m, 2H), 1.90-1.70 (m, 4H).

Example 2(8)

2,4-dimethoxy-N-{3-[1-{2-methyl-4-phenylpentyl)piperidin-4-yl]benzyl}benzamide

HPLC retention time (min.):3.73; Mass data:515 $(M + H)^+$.

Example 2(9)

4-(acetylamino)-N-{3-[1-(2-methyl-4-phenylpentyl)piperidin-4-yl]benzyl}benzamide

HPLC retention time (min.):3.50; Mass data:512 $(M + H)^+$.

Example 2(10)

N-(3-{1-[(3-methyl-2-thienyl)methyl]piperidin-4-yl}benzyl)-3-(trifluoromethyl)benzamide

HPLC retention time (min.):3.57; Mass data:473 $(M + H)^+$.

Example 2(11)

3-chloro-N-(3-{1-[(3-methyl-2-thienyl)methyl]piperidin-4-yl}benzyl)benzamide

HPLC retention time (min.):3.51; Mass data:441, 439 $(M + H)^+$.

Example 2(12)

2,4-dimethoxy-N-(3-{1-[(3-methyl-2-thienyl)methyl]piperidin-4-yl}benzyl)benzamide

HPLC retention time (min.):3.46; Mass data:465 $(M + H)^+$.

Example 2(13)

N-{3-[1-(2-chlorobenzyl)piperidin-4-yl]benzyl}-2,4-dimethoxybenzamide

HPLC retention time (min.):3.49; Mass data:481, 479 $(M + H)^+$.

Example 2(14)

4-(acetylamino)-N-{3-[1-(2-chlorobenzyl)piperidin-4-yl]benzyl}benzamide

HPLC retention time (min.):3.23; Mass data:478, 476 $(M + H)^+$.

Example 2(15)

4-(acetylamino)-N-{3-[1-(4-phenoxybenzyl)piperidin-4-yl]benzyl}benzamide

HPLC retention time (min.):3.49; Mass data:534 $(M + H)^+$.

Example 2(16)

N-{3 -[1-(3-chloro-4-methoxybenzyl)piperidin-4-yl]benzyl}-3-(trifluoromethyl)benzamide

HPLC retention time (min.):3.66; Mass data:517 $(M + H)^+$.

Example 2(17)

3-chloro-N-{3-[1-(3-chloro-4-methoxybenzyl)piperidin-4-yl]benzyl}benzamide

HPLC retention time (min.):3.58; Mass data:485, 483 $(M + H)^+$.

Example 2(18)

N-{3-[1-(3-chloro-4-methoxybenzyl)piperidin-4-yl]benzyl}-3,4-dimethylbenzamide

HPLC retention time (min.):3.61; Mass data:479, 477 $(M + H)^+$.

Example 2(19)

N-{3-[1-(3-chloro-4-methoxybenzyl)piperidin-4-yl]benzyl}-4-(methylthio)benzamide

HPLC retention time (min.):3.56; Mass data:497, 495 $(M + H)^+$.

Example 2(20)

4-(acetylamino)-N-{3-[1-(3-chloro-4-methoxybenzyl)piperidin-4-yl]benzyl}benzamide

HPLC retention time (min.):3.30; Mass data:508, 506 $(M + H)^+$.

Example 2(21)

N-{3-[1-(4-chlorobenzyl)piperidin-4-yl]benzyl}-3-(trifluoromethyl)benzamide

HPLC retention time (min.):3.64; Mass data:487 $(M + H)^+$.

Example 2(22) 3-chloro-N-{3-[1-(4-chlorobenzyl)piperidin-4-yl]benzyl}benzamide

HPLC retention time (min.):3.59; Mass data:455, 453 $(M + H)^+$.

Example 2(23)

N-{3-[1-(4-chlorobenzyl)piperidin-4-yl]benzyl}-2,4-dimethoxybenzamide

HPLC retention time (min.):3.52; Mass data:481, 479 $(M + H)^+$.

Example 2(24)

4-(acetylamino)-N-{3-[1-(4-chlorobenzyl)piperidin-4-yl]benzyl}benzamide

HPLC retention time (min.):3.29; Mass data:478, 476 $(M + H)^+$.

Example 2(25)

4-methoxy-N-(3-{1-[(3-methyl-2-thienyl)methyl]piperidin-4-yl}benzyl)benzenesulfonamide

HPLC retention time (min.):3.47; Mass data:471 $(M + H)^+$.

Example 2(26)

5-chloro-N-(3-{1-[(3-methyl-2-thienyl)methyl]piperidin-4-yl}benzyl)thiophene-2-sulfonamide

HPLC retention time (min.):3.58; Mass data:483, 481 $(M + H)^+$.

Example 2(27) 4-chloro-N-[3-(1-hexylpiperidin-4-yl)benzyl]benzenesulfonamide

HPLC retention time (min.):3.67; Mass data:451, 499 $(M + H)^+$,

Example 2(28)

N-(3-{1-[4-(diethylamino)benzyl]piperidin-4-yl}benzyl)-4-methoxybenzenesulfonamide

HPLC retention time (min.):3.18; Mass data:522 $(M + H)^+$, 162.

Example 2(29)

N-(4-{[(3-{1-[4-(diethylamino)benzyl]piperidin-4-yl}benzyl)amino]sulfonyl}phenyl)acetamide

HPLC retention time (min.):3.03; Mass data: 549 $(M + H)^+$, 162.

Example 2(30)

N-(3-{1-[4-(diethylamino)benzyl]piperidin-4-yl}benzyl)-1-methyl-1H-imidazol-4-sulfonamide

HPLC retention time (min.):2.91; Mass data:496 $(M + H)^+$, 162.

Example 2(31)

N- {3-[1-(2-chlorabenzyl)piperidin-4-yl]benzyl}-4-methoxybenzenesulfonamide

HPLC retention time (min.):3.47; Mass data:487, 485 $(M + H)^+$.

Example 2(32)

4-chloro-N-{3-[1-(2-chlorobenzyl)piperidin-4-yl]benzyl}benzenesulfonamide

HPLC retention time (min.):3.56; Mass data:491, 489 $(M + H)^+$.

Example 2(33)

N-{4-[({3-[1-(2-chlorobenzyl)piperidin-4-yl]benzyl}amino)sulfonyl]phenyl}acetamide

HPLC retention time (min.):3.33; Mass data:514, 512 $(M + H)^+$.

Example 2(34)

N-{3-[1-(2,2-dimethylpropyl)piperidin-4-yl]benzyl}-4-propylbenzenesulfonamide

HPLC retention time (min.):3.67; Mass data:443 $(M + H)^+$.

Example 2(35)

N-{4-[({3-[1-(3-chloro-4-methoxybenzyl)piperidin-4-yl]benzyl }amino)sulfonyl]phenyl}acetamide

HPLC retention time (min.):3.37; Mass data:542 $(M + H)^+$.

Example 2(36)

N-[3-(1-hexylpiperidin-4-yl)benzyl]quinoline-8-sulfonamide

HPLC retention time (min.):3.51; Mass data:466 $(M + H)^+$.

Example 2(37)

N-(3-{1-[4-(diethylamino)benzyl]piperidin-4-yl}benzyl)quinoline-8-sulfonamide

HPLC retention time (mm.):3.12; Mass data: 543 $(M + H)^+$, 162.

Example 2(38)

N-{3-[1-(4-chlorobenzyl)piperidin-4-yl]benzyl}quinoline-8-sulfonamide

HPLC retention time (min.):3.48; Mass data: 508, 506 $(M + H)^+$.

Example 2(39)

N-[3-(1-hexylpiperidin-4-yl)benzyl]-1-methyl-1H-imidazole-4-sulfonamide

HPLC retention time (min.):3.24; Mass data:419 $(M + H)^+$.

Example 2(40)

N-{3-[1-(4-chlorobenzyl)piperidin-4-yl]benzyl}-2-phenoxyacetamide

HPLC retention time (min.):3.53; Mass data:449 $(M + H)^+$.

Example 2(41)

N-{3-[1-(4-chlorobenzyl)piperidin-4-yl]benzyl}-4-methylbenzamide

HPLC retention time (min.):3.51; Mass data:433 $(M + H)^+$.

Example 2(42)

N-{3-[1-(4-chlorobenzyl)piperidin-4-yl]benzyl}benzamide

HPLC retention time (min.):3.44; Mass data:419 $(M + H)^+$.

Example 2(43)

N-{3-[1-(4-chlorobenzyl)piperidin-4-yl]benzyl}-2-furanamide

HPLC retention time (min.):3.33; Mass data:409 $(M + H)^+$.

Example 2(44)

2-(benzyloxy)-N-{3-[1-(4-chlorobenzyl)piperidin-4-yl]benzyl}acetamide

HPLC retention time (min.):3.53; Mass data:465, 463 $(M + H)^+$.

Example 2(45)

N-{3-[1-(4-chlorobenzyl)piperidin-4-yl]benzyl}-1-benzofuran-2-carboxamide

HPLC retention time (min.):3.56; Mass data:459 $(M + H)^+$.

Example 2(46)

N-(3-{1-[4-(dimethylamino)benzyl]piperidin-4-yl}benzyl)-4-(methylthio)benzamide

HPLC retention time (min.):3.25; Mass data:474 $(M + H)^+$, 134.

Example 2(47)

4-(methylthio)-N-{3-[1-(4-pyrrolidin-1-ylbenzyl)piperidin-4-yl]benzyl}benzamide

HPLC retention time (min.):3.62; Mass data; 500 $(M + H)^+$, 160.

Example 2(48)

N-[3-(1-{4-[3-(dimethylamino)propoxy]benzyl}piperidin-4-yl)benzyl]-4-(methylthio)benzamide

HPLC retention time (min.):3.22; Mass data:532 $(M + H)^+$.

Example 2(49)

N-{3-[1-(4-fluorobenzyl)piperidin-4-yl]benzyl}-4-(methylthio)benzamide

HPLC retention time (min.):3.49; Mass data:449 $(M + H)^+$.

Example 2(50)

N-{3-[1-(2,4-dimethoxybenzyl)piperidin-4-yl]benzyl}-4-(methylthio)benzamide

HPLC retention time (min.):3.55; Mass data:491 $(M + H)^+$.

Example 2(51)

N-{3-[1-(2,5-dimethoxybenzyl)piperidin-4-yl]benzyl}-4-(methylthio)benzamide

HPLC retention time (min.):3.53; Mass data:491 $(M + H)^+$.

Example 2(52)

N-{3-[1-(2,6-dimethoxybenzyl)piperidin-4-yl]benzyl }-4-(methylthio)benzamide

HPLC retention time (min.):3.56; Mass data:491 $(M + H)^+$.

Example 2(53)

4-(methylthio)-N-{3-[1-(2,4,6-trimethoxybenzyl)piperidin-4-yl]benzyl}benzamide

HPLC retention time (min.):3.62; Mass data:521 $(M + H)^+$.

Example 2(54)

4-(methylthio)-N-{3-[1-(3,4,5-trimethoxybenzyl)piperidin-4-yl]benzyl}benzamide

HPLC retention time (min.):3.47; Mass data:521 $(M + H)^+$.

Example 2(55)

N-{3-[1-(4-methylbenzyl)piperidin-4-yl]benzyl}-4-(methylthio)benzamide

HPLC retention time (min.):3.55; Mass data:445 $(M + H)^+$.

Example 2(56)

N-{3-[1-(4-cyanobenzyl)piperidin-4-yl]benzyl}-4-(methylthio)benzamide

HPLC retention time (min.):3.42; Mass data:456 $(M + H)^+$.

Example 2(57)

4-(methylthio)-N-(3-{1-[4-(trifluoromethoxy)benzyl]piperidin-4-yl}benzyl)benzamide

HPLC retention time (min.):3.64; Mass data:515 $(M + H)^+$.

Example 2(58)

N-{3-[1-(3-methoxybenzyl)piperidin-4-yl]benzyl}-4-(methylthio)benzamide

HPLC retention time (min.):3.49; Mass data:461 $(M + H)^+$.

Example 2(59)

N-{3-[1-(2-methoxybenzyl)piperidin-4-yl]benzyl}-4-(methylthio)benzamide

HPLC retention time (min.):3.53; Mass data:461 $(M + H)^+$.

Example 2(60)

4-(methylthio)-N-(3-{1-[3-(methylthio)propyl]piperidin-4-yl}benzyl)benzamide

HPLC retention time (min.):3.40; Mass data:429 $(M + H)^+$.

Example 2(61) N-[3-(1-benzylpiperidin-4-yl)benzyl]-4-(methylthio)benzamide

HPLC retention time (min.):3.45; Mass data:431 $(M + H)^+$.

Example 2(62)

N-[3-(1-{(2E)-3-[4-(dimethylamino)phenyl]prop-2-en-1-yl}piperidin-4-yl)benzyl]-4-(methylthio)benzamide

HPLC retention time (min.):3.25; Mass data:500 $(M + H)^+$, 341, 160.

Example 2(63)

N-{3-[1-(2,3-dihydro-1,4-benzodioxin-6-ylmethyl)piperidin-4-yl]benzyl}-4-(methylthio)benzamide

HPLC retention time (min.):3.49; Mass data:489 $(M + H)^+$.

Example 2(64)

N-(3-{1-[(1-methyl-1H-pyrrol-2-yl)methyl]piperidin-4-yl}benzyl)-4-(methylthio)benzamide

HPLC retention time (min.):3.44; Mass data:434 $(M + H)^+$.

Example 2(65)

N-{3-[4-(4-methylbenzyl)piperazin-1-yl]benzyl}benzamide

HPLC retention time (min.):3.34; Mass data:400 $(M + H)^+$.

Example 2(66)

3-methyl-N-{3-[4-(4-methylbenzyl)piperazin-1-yl]benzyl}benzamide

HPLC retention time (min.):3.42; Mass data:414 $(M + H)^+$.

Example 2(67)

4-fluoro-N-{3-[4-(4-methylbenzyl)piperazin-1-yl]benzyl}benzamide

HPLC retention time (min.):3.38; Mass data:418 $(M + H)^+$.

Example 2(68)

N-{3-[4-(4-methoxybenzyl)piperazin-1-yl]benzyl}benzamide

HPLC retention time (min.):3.27; Mass data:416 $(M + H)^+$.

Example 2(69)

N-{3-[4-(4-methoxybenzyl)piperazin-1-yl]benzyl}-4-methylbenzamide

HPLC retention time (min.):3.37; Mass data:430 $(M + H)^+$.

Example 2(70)

N-{3-[4-(4-methoxybenzyl)piperazin-1-yl]benzyl}-3-methylbenzamide

HPLC retention time (min.):3.37; Mass data:430 $(M + H)^+$,

Example 2(71)

N-{3-[4-(4-methoxybenzyl)piperazin-1-yl]benzyl}-2-methylbenzamide

HPLC retention time (min.):3.31; Mass data:430 $(M + H)^+$.

Example 2(72)

4-fluoro-N-{3-[4-(4-methoxybenzyl)piperazin-1-yl]benzyl}benzamide

HPLC retention time (min.):3.34; Mass data:434 $(M + H)^+$.

Example 2(73)

N-(3-{4-[4-(dimethylamino)benzyl]piperazin-1-yl}benzyl)benzamide

HPLC retention time (min.):3.04; Mass data:429 $(M + H)^+$, 134.

Example 2(74)

N-(3-{4-[4-(dimethylamino)benzyl]piperazin-1-yl}benzyl)-4-methylbenzamide

HPLC retention time (min.):3.13; Mass data:443 $(M + H)^+$, 134.

Example 2(75)

N-(3-{4-[4-(dimethylamino)benzyl]piperazin-1-yl}benzyl)-3-methylbenzamide

HPLC retention time (min.):3.15; Mass data:443 $(M + H)^+$, 134.

Example 2(76)

N-(3-{4-[4-(dimethylamino)benzyl]piperazin-1-yl}benzyl)-2-methylbenzamide

HPLC retention time (min.):3.08; Mass data:443 $(M + H)^+$, 134.

Example 2(77)

N-(3-{4-[4-(dimethylamino)benzyl]piperazin-1-yl}benzyl)-4-fluorobenzamide

HPLC retention time (min.):3.11; Mass data:447 $(M + H)^+$, 134.

Example 2(78)

N-(3-{4-[4-(dimethylamino)benzyl]piperazin-1-yl}benzyl)-4-methoxybenzamide

HPLC retention time (min.):3.08; Mass data:459 $(M + H)^+$, 134.

Example 2(79)

N-{3-[4-(2,4-dimethoxybenzyl)piperazin-1-yl]benzyl}benzamide

HPLC retention time (min.):3.34; Mass data:446 $(M + H)^+$, 151.

Example 2(80)

N-{3-[4-(2,4-dimethoxybenzyl)piperazin-1-yl]benzyl}-4-methylbenzamide

HPLC retention time (min.):3.42; Mass data:460 $(M + H)^+$.

Example 2(81)

N-{3-[4-(2,4-dimethoxybenzyl)piperazin-1-yl]benzyl}-2,5-dimethylbenzamide

HPLC retention time (min.):3.46; Mass data:474 $(M + H)^+$.

Example 2(82)

N-{3-[4-(2,4-dimethoxybenzyl)piperazin-1-yl]benzyl}-3-methylbenzamide

HPLC retention time (min.):3.44; Mass data:460 $(M + H)^+$.

Example 2(83)

N-{3-[4-(2,4-dimethoxybenzyl)piperazin-1-yl]benzyl}-2-methylbenzamide

HPLC retention time (min.):3.37; Mass data:460 $(M + H)^+$.

Example 2(84)

N-{3-[4-(2,4-dimethoxybenzyl)piperazin-1-yl]benzyl}-4-fluorobenzamide

HPLC retention time (min.):3.39; Mass data:464 $(M + H)^+$, 151.

Example 2(85)

N-{3-[4-(2,4-dimethoxybenzyl)piperazin-1-yl]benzyl}-4-methoxybenzamide

HPLC retention time (min.):3.36; Mass data:476 $(M + H)^+$.

Example 2(86)

N-[3-(4-{(2E)-3-[4-(dimethylamino)phenyl]prop-2-en-1-yl}piperazin-1-yl)benzyl]benzamide

HPLC retention time (min.):3.06; Mass data:455 $(M + H)^+$, 160.

Example 2(87)

N-[3-(4-{(2E)-3-[4-(dimethylamino)phenyl]prop-2-en-1-yl}piperazin-1-yl)benzyl]-4-methylbenzamide

HPLC retention time (min.):3.14; Mass data:469 $(M + H)^+$, 164.

Example 2(88)

N-[3-(4-{(2E)-3-[4-(dimethylamino)phenyl]prop-2-en-1-yl}piperazin-1-yl)benzyl]-2,4-dimethylbenzamide

HPLC retention time (min.):3.18; Mass data:483 $(M + H)^+$, 160.

Example 2(89)

N-[3-(4-{(2E)-3-[4-(dimethylamino)phenyl]prop-2-en-1-yl}piperazin-1-yl)benzyl]-2,5-dimethylbenzamide

HPLC retention time (min.):3.18; Mass data:483 $(M + H)^+$, 160.

Example 2(90)

N-[3-(4-{(2E)-3-[4-(dimethylamino)phenyl]prop-2-en-1-yl}piperazin-1-yl)benzyl]-3-methylbenzamide

HPLC retention time (min.):3.15; Mass data:469 $(M + H)^+$, 160.

Example 2(91)

N-[3-(4-{(2E)-3-[4-(dimethylamino)phenyl]prop-2-en-1-yl}piperazin-1-yl)benzyl]-2-methylbenzamide

HPLC retention time (min.):3.09; Mass data:469 $(M + H)^+$, 164, 160.

Example 2(92)

N-[3-(4-{(2E)-3-[4-(dimethylamino)phenyl]prop-2-en-1-yl}piperazin-1-yl)benzyl]-4-fluorobenzamide

HPLC retention time (min.):3.12; Mass data:473 $(M + H)^+$, 164, 160.

Example 2(93)

4-(acetylamino)-N-[3-(4-{(2E)-3-[4-(dimethylamino)phenyl]prop-2-en-1-yl}piperazin-1-yl)benzyl]benzamide

HPLC retention time (min.):2.94; Mass data:512 $(M + H)^+$, 353, 160.

Example 2(94)

N-[3-(4-{(2E)-3-[4-(dimethylamino)phenyl]prop-2-en-1-yl}piperazin-1-yl)benzyl]isonicotinamide

HPLC retention time (min.):2.78; Mass data:456 $(M + H)^+$, 297, 160.

Example 2(95)

N-[3-(4-{(2E)-3-[4-(dimethylamino)phenyl]prop-2-en-1-yl}piperazin-1-yl)benzyl]-4-methoxybenzamide

HPLC retention time (min.):3.09; Mass data:485 $(M + H)^+$, 326, 160.

Example 2(96)

N-{3-[4-(4-chlorobenzyl)piperazin-1-yl]benzyl}benzamide

HPLC retention time (min.):3.36; Mass data:420 $(M + H)^+$.

Example 2(97)

N-{3-[4-(4-chlorobenzyl)piperazin-1-yl]benzyl}-2-methylbenzamide

HPLC retention time (min.):3.38; Mass data:434 $(M + H)^+$.

Example 2(98)

N-{3-[4-(4-chlorobenzyl)piperazin-1-yl]benzyl}-4-fluorobenzamide

HPLC retention time (min.):3.39; Mass data:438 $(M + H)^+$.

Example 2(99)

N-{3-[4-(3-chloro-4-methoxybenzyl)piperazin-1-yl]benzyl}-2-methylbenzamide

HPLC retention time (min.):3.40; Mass data:464 $(M + H)^+$.

Example 2(100)

N-{3-[4-(3-chloro-4-methoxybenzyl)piperazin-1-yl]benzyl}-4-fluorobenzamide

HPLC retention time (min.):3.00; Mass data:468 $(M + H)^+$.

Example 2(101)

N-{3-[4-(3,4-dimethoxybenzyl)piperazin-1-yl]benzyl}benzamide

HPLC retention time (min.):3.23; Mass data:446 $(M + H)^+$.

Example 2(102)

N-{3-[4-(3,4-dimethoxybenzyl)piperazin-1-yl]benzyl}-4-methylbenzamide

HPLC retention time (min.):3.31; Mass data:460 $(M + H)^+$.

Example 2(103)

N-{3-[4-(3,4-dimethoxybenzyl)piperazin-1-yl]benzyl}-2,4-dimethylbenzamide

HPLC retention time (min.):3.36; Mass data:474 $(M + H)^+$, 178.

Example 2(104)

N-{3-[4-(3,4-dimethoxybenzyl)piperazin-1-yl]benzyl}-2,5-dimethylbenzamide

HPLC retention time (min.):3.36; Mass data:474 $(M + H)^+$.

Example 2(105)

N-{3-[4-(3,4-dimethoxybenzyl)piperazin-1-yl]benzyl}-3-methylbenzamide

HPLC retention time (min.):3.32; Mass data:460 $(M + H)^+$.

Example 2(106)

N-{3-[4-(3,4-dimethoxybenzyl)piperazin-1-yl]benzyl}-2-methylbenzamide

HPLC retention time (min.):3.26; Mass data:460 $(M + H)^+$.

Example 2(107)

N-{3-[4-(3,4-dimethoxybenzyl)piperazin-1-yl]benzyl}-4-fluorobenzamide

HPLC retention time (min.):3.29; Mass data:464 $(M + H)^+$.

Example 2(108)

N-{3-[4-(3,4-dimethoxybenzyl)piperazin-1-yl]benzyl}isonicotinamide

HPLC retention time (min.):2.89; Mass data:447 $(M + H)^+$.

Example 2(109)

N-{3-[4-(3,4-dimethoxybenzyl)piperazin-1-yl]benzyl}-2-(4-methylphenyl)acetamide

HPLC retention time (min.):3.31; Mass data:474 $(M + H)^+$.

Example 2(110)

N-{3-[4-(3,4-dimethoxybenzyl)piperazin-1-yl]benzyl}-4-methoxybenzamide

HPLC retention time (min.):3.24; Mass data:476 $(M + H)^+$.

Example 2(111) N-{4-[1-(4-methylbenzyl)piperidin-4-yl]benzyl}benzamide

HPLC retention time (min.):3.38; Mass data:399 $(M + H)^+$.

Example 2(112)

4-methyl-N-{4-[1-(4-methylbenzyl)piperidin-4-yl]benzyl}benzamide

HPLC retention time (min.):3.47; Mass data:413 $(M + H)^+$.

Example 2(113)

2,4-dimethyl-N-{4-[1-(4-methylbenzyl)piperidin-4-yl]benzyl}benzamide

HPLC retention time (min.):3.5; Mass data:427 $(M+ H)^+$.

Example 2(114)

3-methyl-N-{4-[1-(4-methylbenzyl)piperidin-4-yl]benzyl}benzamide

HPLC retention time (min.):3.48; Mass data:413 $(M + H)^+$.

Example 2(115)

4-fluoro-N-{4-[1-(4-methylbenzyl)piperidin-4-yl]benzyl}benzamide

HPLC retention time (min.):3.44; Mass data:417 $(M + H)^+$.

Example 2(116)

4-methoxy-N-{4-[1-(4-methylbenzyl)piperidin-4-yl]benzyl}benzamide

HPLC retention time (min.):3.41; Mass data:429 $(M + H)^+$.

Example 2(117)

N-{4-[1-(4-methoxybenzyl)piperidin-4-yl]benzyl}-4-methylbenzamide

HPLC retention time (min.):3.42; Mass data:429 $(M + H)^+$.

Example 2(118)

4-methoxy-N-{4-[1-(4-methoxybenzyl)piperidin-4-yl]benzyl}benzamide

HPLC retention time (min.):3.35; Mass data:445 $(M + H)^+$.

Example 2(119)

4-methoxy-N-(4-{1-[4-(methylsulfonyl)benzyl]piperidin-4-yl}benzyl)benzamide

HPLC retention time (min.):3.22; Mass data:493 $(M + H)^+$.

Example 2(120)

N-(4-{1-[4-(dimethylamino)benzyl]piperidin-4-yl}benzyl)benzamide

HPLC retention time (min.):3.07; Mass data:428 $(M + H)^+$, 134.

Example 2(121)

N-(4-{1-[4-(dimethylamino)benzyl]piperidin-4-yl}benzyl)-4-methylbenzamide

HPLC retention time (min.):3.17; Mass data:442 $(M + H)^+$, 164.

Example 2(122)

N-(4-{1-[4-(dimethylamino)benzyl]piperidin-4-yl}benzyl)-2,4-dimethylbenzamide

HPLC retention time (min.):3.21; Mass data:456 $(M + H)^+$, 134.

Example 2(123)

N-(4-{1-[4-(dimethylamino)benzyl]piperidin-4-yl}benzyl)-2,5-dimethylbenzamide

HPLC retention time (min.):3.20; Mass data:456 $(M + H)^+$, 134.

Example 2(124)

N-(4-{1-[4-(dimethylamino)benzyl]piperidin-4-yl}benzyl)-3-methylbenzamide

HPLC retention time (min.):3.17; Mass data:442 $(M + H)^+$, 134.

Example 2(125)

N-(4-{1-[4-(dimethylamino)benzyl]piperidin-4-yl}benzyl)-2-methylbenzamide

HPLC retention time (min.):3.09; Mass data:442 $(M + H)^+$, 134.

Example 2(126)

N-(4-{1-[4-(dimethylamino)benzyl]piperidin-4-yl}benzyl)-4-fluorobenzamide

HPLC retention time (min.):3.12; Mass data:446 $(M + H)^+$, 134.

Example 2(127)

4-(acetylamino)-N-(4-{1-[4-(dimethylamino)benzyl]piperidin-4-yl}benzyl)benzamide

HPLC retention time (min.):2.94; Mass data:485 $(M + H)^+$, 134.

Example 2(128)

N-(4-{1-[4-(dimethylamino)benzyl]piperidin-4-yl}benzyl)-4-methoxybenzamide

HPLC retention time (min.):3.10; Mass data:458 $(M + H)^+$, 134.

Example 2(129) N-{4-[1-(2,4-dimethoxybenzyl)piperidin-4-yl]benzyl}benzamide

HPLC retention time (min.):3.40; Mass data:445 $(M + H)^+$.

Example 2(130)

N-{4-[1-(2,4-dimethoxybenzyl)piperidin-4-yl]benzyl}-4-methylbenzamide

HPLC retention time (min.):3.48; Mass data:459 $(M + H)^+$.

Example 2(131)

N-{4-[1-(2,4-dimethoxybenzyl)piperidin-4-yl]benzyl}-2,4-dimethylbenzamide

HPLC retention time (min.):3.51; Mass data:473 $(M + H)^+$.

Example 2(132)

N-{4-[1-(2,4-dimethoxybenzyl)piperidin-4-yl]benzyl}-2,5-dimethylbenzamide

HPLC retention time (min.):3.51; Mass data:473 $(M + H)^+$.

Example 2(133)

N-{4-[1-(2,4-dimethoxybenzyl)piperidin-4-yl]benzyl}-3-methylbenzamide

HPLC retention time (min.):3.47; Mass data:459 $(M + H)^+$.

Example 2(134)

N-{4-[1-(2,4-dimethoxybenzyl)piperidin-4-yl]benzyl}-2-rnethylbenzamide

HPLC retention time (min.):3.42; Mass data:459 $(M + H)^+$.

Example 2(135)

N-{4-[1-(2,4-dimethoxybenzyl)piperidin-4-yl]benzyl}-4-fluorobenzamide

HPLC retention time (min.):3.44; Mass data:463 $(M + H)^+$, 151.

Example 2(136)

N-{4-[1-(2,4-dimethoxybenzyl)piperidin-4-yl]benzyl}-4-methoxybenzamide

HPLC retention time (min.):3.41; Mass data:475 $(M + H)^+$.

Example 2(137) N-{4-[1-(4-cyanobenzyl)piperidin-4-yl]benzyl}-3-methylbenzamide

HPLC retention time (min.):3.35; Mass data:424 $(M + H)^+$.

Example 2(138)

N-{4-[1-(4-cyanobenzyl)piperidin-4-yl]benzyl}-4-methoxybenzamide

HPLC retention time (min.):3.29; Mass data:440 $(M + H)^+$.

Example 2(139)

N-{4-[1-(4-chlorobenzyl)piperidin-4-yl]benzyl}isonicotinamide

HPLC retention time (min.):3.05; Mass data:420 $(M + H)^+$.

Example 2(140)

N-{4-[1-(3,4-dimethoxybenzyl)piperidin-4-yl]benzyl}benzamide

HPLC retention time (min.):3.26; Mass data:445 $(M + H)^+$.

Example 2(141)

N-{4-[1-(3,4-dimethoxybenzyl)piperidin-4-yl]benzyl}-4-methylbenzamide

HPLC retention time (min.):3.36; Mass data:459 $(M + H)^+$.

Example 2(142)

N-{4-[1-(3,4-dimethoxybenzyl)piperidin-4-yl]benzyl}-2,4-dimethylbenzamide

HPLC retention time (min.):3.39; Mass data:473 $(M + H)^+$,

Example 2(143)

N-{4-[1-(3,4-dimethoxybenzyl)piperidin-4-yl]benzyl}-2,5-dimethylbenzamide

HPLC retention time (min.):3.39; Mass data:473 $(M + H)^+$.

Example 2(144)

N-{4-[1-(3,4-dimethoxybenzyl)piperidin-4-yl]benzyl}-3-methylbenzamide

HPLC retention time (min.):3.36; Mass data:459 $(M + H)^+$.

Example 2(145)

N- {4-[1-(3,4-dimethoxybenzyl)piperidin-4-yl]benzyl}-2-methylbenzamide

HPLC retention time (min.):3.32; Mass data:459 $(M + H)^+$.

Example 2(146)

N-{4-[1-(3,4-dimethoxybenzyl)piperidin-4-yl]benzyl}-4-fluorobenzamide

HPLC retention time (min.):3.32; Mass data:463 $(M + H)^+$.

Example 2(147)

N-{4-[1-(3,4-dimethoxybenzyl)piperidin-4-yl]benzyl}-4-methoxybenzamide

HPLC retention time (min.):3.29; Mass data:475 $(M + H)^+$.

Example 2(148)

N-{3-[1-(4-methoxybenzyl)-1,2,3,6-tetrahydropyridin-4-yl]benzyl}benzamide

HPLC retention time (min.):3.36; Mass data:413 $(M + H)^+$.

Example 2(149)

N-{3-[1-(4-methoxybenzyl)-1,2,3,6-tetrahydropyridin-4-yl]benzyl}-3-methylbenzamide

HPLC retention time (min.):3.44; Mass data:427 $(M + H)^+$.

Example 2(150)

4-fluoro-N-{3-[1-(4-methoxybenzyl)-1,2,3,6-tetrahydropyridin-4-yl]benzyl}benzamide

HPLC retention time (min.):3.41; Mass data:431 $(M + H)^+$.

Example 2(151)

4-methoxy-N-{3-[1-(4-methoxybenzyl)-1,2,3,6-tetrahydropyridin-4-yl]benzyl}benzamide

HPLC retention time (min.):3.37; Mass data:443 $(M + H)^+$.

Example 2(152)

N-(3-{1-[4-(methylsulfonyl)benzyl]-1,2,3,6-tetrahydropyridin-4-yl}benzyl)benzamide

HPLC retention time (min.):3.20; Mass data:461 $(M + H)^+$.

Example 2(153)

3-methyl-N-(3-{1-[4-(methylsulfonyl)benzyl]-1,2,3,6-tetrahydropyridin-4-yl}benzyl)benzamide

HPLC retention time (min.):3.30; Mass data:475 $(M + H)^+$.

Example 2(154)

2-methyl-N-(3-{1-[4-(methylsulfonyl)benzyl]-1,2,3,6-tetrahydropyridin-4-yl}benzyl)benzamide

HPLC retention time (min.):3.25; Mass data:475 $(M + H)^+$.

Example 2(155)

4-fluoro-N-(3-{1-[4-(methylsulfonyl)benzyl]-1,2,3,6-tetrahydropyridin-4-yl}benzyl)benzamide

HPLC retention time (min.):3.25; Mass data:479 $(M + H)^+$.

Example 2(156)

N-(3-{1-[4-(dimethylamino)benzyl]-1,2,3,6-tetrahydropyridin-4-yl}benzyl)benzamide

HPLC retention time (min.):3.11; Mass data:426 $(M + H)^+$, 134.

Example 2(157)

N-(3-{1-[4-(dimethylamino)benzyl]-1,2,3,6-tetrahydropyridin-4-yl}benzyl)-4-methylbenzamide

HPLC retention time (min.):3.20; Mass data:440 $(M + H)^+$, 134.

Example 2(158)

N-(3-{1-[4-(dimethylamino)benzyl]-1,2,3,6-tetrahydropyridin-4-yl}benzyl)-3-methylbenzamide

HPLC retention time (min.):3.20; Mass data:440 $(M + H)^+$, 134.

Example 2(159)

N-(3-{1-[4-(dimethylamino)benzyl]-1,2,3,6-tetrahydropyridin-4-yl}benzyl)-2-methylbenzamide

HPLC retention time (min.):3.15; Mass data:440 $(M + H)^+$, 134.

Example 2(160)

N-(3-{1-[4-(dimethylamino)benzyl]-1,2,3,6-tetrahydropyridin-4-yl}benzyl)-4-fluorobenzamide

HPLC retention time (min.):3.14; Mass data:444 $(M + H)^+$, 134.

Example 2(161)

N-(3-{1-[4-(dimethylamino)benzyl]-1,2,3,6-tetrahydropyridin-4-yl} benzyl)isonicotinamide

HPLC retention time (min.):2.81; Mass data:427 $(M + H)^+$, 134.

Example 2(162)

N-(3-{1-[4-(dimethylamino)benzyl]-1,2,3,6-tetrahydropyridin-4-yl}benzyl)-4-methoxybenzamide

HPLC retention time (min.):3.14; Mass data:456 $(M + H)^+$, 134.

Example 2(163)

N- {3-[1-(2,4-dimethoxybenzyl)-1,2,3,6-tetrahydropyridin-4-yl]benzyl}benzamide

HPLC retention time (min.):3.42; Mass data:443 $(M + H)^+$, 151.

Example 2(164)

N-{3-[1-(2,4-dimethoxybenzyl)-1,2,3,6-tetrahydropyridin-4-yl]benzyl}-4-methylbenzamide

HPLC retention time (min.):3.50; Mass data:457 $(M + H)^+$.

Example 2(165)

N- {3-[1-(2,4-dimethoxybenzyl)-1,2,3,6-tetrahydropyridin-4-yl]benzyl}-2,5-dimethylbenzamide

HPLC retention time (min.):3.53; Mass data:471 $(M + H)^+$.

Example 2(166)

N-{3-[1-(2,4-dimethoxybenzyl)-1,2,3,6-tetrahydropyridin-4-yl]benzyl}-3-methylbenzamide

HPLC retention time (min.):3.50; Mass data:457 $(M + H)^+$, 151.

Example 2(167)

N-{3-[1-(2,4-dimethoxybenzyl)-1,2,3,6-tetrahydropyridin-4-yl]benzyl}-2-methylbenzamide

HPLC retention time (min.):3.44; Mass data:457 $(M + H)^+$.

Example 2(168)

N-{3-[1-(2,4-dimethoxybenzyl)-1,2,3,6-tetrahydropyridin-4-yl]benzyl}-4-fluorobenzamide

HPLC retention time (min.):3.46; Mass data:461 $(M + H)^+$.

Example 2(169)

N-{3-[1-(2,4-dimethoxybenzyl)-1,2,3,6-tetrahydropyridin-4-yl]benzyl}-4-methoxybenzamide

HPLC retention time (min.):3.43; Mass data:473 $(M + H)^+$, 151.

Example 2(170)

N-{3-[1-(3-chloro-4-methoxybenzyl)-1,2,3,6-tetrahydropyridin-4-yl]benzyl}-4-fluorobenzamide

HPLC retention time (min.):3.46; Mass data:465 $(M + H)^+$.

Example 2(171)

N-{3-[1-(3,4-dimethoxybenzyl)-1,2,3,6-tetrahydropyridin-4-yl]benzyl}benzamide

HPLC retention time (min.):3.28; Mass data:443 $(M + H)^+$.

Example 2(172)

N-{3-[1-(3,4-dimethoxybenzyl)-1,2,3,6-tetrahydropyridin-4-yl]benzyl}-4-methylbenzamide

HPLC retention time (min.):3.36; Mass data:457 $(M + H)^+$.

Example 2(173)

N- {3-[1-(3,4-dimethoxybenzyl)-1,2,3,6-tetrahydropyridin-4-yl]benzyl}-2,4-dimethylbenzamide

HPLC retention time (min.):3.41; Mass data:471 $(M + H)^+$.

Example 2(174)

N-{3-[1-(3,4-dimethoxybenzyl)-1,2,3,6-tetrahydropyridin-4-yl]benzyl}-2,5-dimethylbenzamide

HPLC retention time (min.):3.40; Mass data:471 $(M + H)^+$.

Example 2(175)

N-{3-[1-(3,4-dimethoxybenzyl)-1,2,3,6-tetrahydropyridin-4-yl]benzyl}-3-methylbenzamide

HPLC retention time (min.):3.37; Mass data:457 $(M + H)^+$.

Example 2(176)

N-{3-[1-(3,4-dimethoxybenzyl)-1,2,3,6-tetrahydropyridin-4-yl]benzyl}-2-methylbenzamide

HPLC retention time (min.):3.31; Mass data:457 $(M + H)^+$.

Example 2(177)

N-{3-[1-(3,4-dimethoxybenzyl)-1,2,3,6-tetrahydropyridin-4-yl]benzyl}-4-fluorobenzamide

HPLC retention time (min.):3.32; Mass data:461 $(M + H)^+$.

Example 2(178)

4-(acetylamino)-N-{3-[1-(3,4-dimethoxybenzyl)-1,2,3,6-tetrahydropyridin-4-yl]benzyl}benzamide

HPLC retention time (min.):3.12; Mass data:500 $(M + H)^+$.

Example 2(179)

N-{3-[1-(3,4-dimethoxybenzyl)-1,2,3,6-tetrahydropyridin-4-yl]benzyl}isonicotinamide

HPLC retention time (min.):2.94; Mass data:444 $(M + H)^+$, 151.

Example 2(180)

N-{3-[1-(3,4-dimethoxybenzyl)-1,2,3,6-tetrahydropyridin-4-yl]benzyl}-2-(4-methylphenyl)acetamide

HPLC retention time (min.):3.36; Mass data:471 $(M + H)^+$.

Example 2(181)

N-{3-[1-(3,4-dimethoxybenzyl)-1,2,3,6-tetrahydropyridin-4-yl]benzyl}-4-methoxybenzamide

HPLC retention time (min.):3.31; Mass data:473 $(M + H)^+$.

Example 2(182)

N-{3-[4-(4-methoxybenzyl)piperazin-1-yl]benzyl}-4-(methylthio)benzamide

HPLC retention time (min.):3.41; Mass data:462 $(M + H)^+$.

Example 2(183)

N-(3-{4-[4-(dimethylamino)benzyl]piperazin-1-yl}benzyl)-4-(methylthio)benzamide

HPLC retention time (min.):3.19; Mass data:475 $(M + H)^+$, 134.

Example 2(184)

N-{3-[4-(2,4-dimethoxybenzyl)piperazin-1-yl]benzyl}-4-(methylthio)benzamide

HPLC retention time (min.):3.46; Mass data:492 $(M + H)^+$.

Example 2(185)

N-[3-(4-{(2E)-3-[4-(dimethylamino)phenyl]prop-2-en-1-yl}piperazin-1-yl)benzyl]-4-(methylthio)benzamide

HPLC retention time (min.):3.18; Mass data:501 $(M + H)^+$, 160.

Example 2(186)

N-{3-[4-(3,4-dimethoxybenzyl)piperazin-1-yl]benzyl}-4-(methylthio)benzamide

HPLC retention time (min.):3.35; Mass data:492 $(M + H)^+$.

Example 2(187)

N-{4-[1-(2,4-dimethoxybenzyl)piperidin-4-yl]benzyl}-4-(methylthio)benzamide

HPLC retention time (min.):3.52; Mass data:491 $(M + H)^+$.

Example 2(188)

N-{4-[1-(3,4-dimethoxybenzyl)piperidin-4-yl]benzyl}-4-(methylthio)benzamide

HPLC retention time (min.):3.39; Mass data:491 $(M + H)^+$.

Example 2(189)

N-(3-{1-[4-(dimethylamino)benzyl]-1,2,3,6-tetrahydropyridin-4-yl}benzyl)-4-(methylthio)benzamide

HPLC retention time (min.):3.22; Mass data:472 $(M + H)^+$, 134.

Example 2(190)

N-{3-[1-(2,4-dimethoxybenzyl)-1,2,3,6-tetrahydropyridin-4-yl]benzyl}-4-(methylthio)benzamide

HPLC retention time (min.):3.51; Mass data:489 $(M + H)^+$.

Example 2(191)

N-{3-[1-(3,4-dimethoxybenzyl)-1,2,3,6-tetrahydropyridin-4-yl]benzyl}-4-(methylthio)benzamide

HPLC retention time (min.): 3.40; Mass data:489 $(M + H)^+$.

Example 2(192)

N-{3-[4-(4-chlorobenzyl)piperazin-1-yl]benzyl}-2-(methylthio)benzamide

HPLC retention time (min.):3.40; Mass data:931 $(2M + H)^+$, 466 $(M + H)^+$.

Example 2(193)

N-{3-[4-(4-chlorobenzyl)piperazin-1-yl]benzyl}-2,6-dimethoxybenzamide

HPLC retention time (min.):3.33; Mass data:959 $(2M + H)^+$, 480 $(M + H)^+$.

Example 2(194)

2-chloro-N-{3-[4-(4-chlorobenzyl)piperazin-1-yl]benzyl}benzamide

HPLC retention time (min.):3.40; Mass data:907 $(2M + H)^+$, 454 $(M + H)^+$.

Example 2(195)

N-{3-[4-(4-chlorobenzyl)piperazin-1-yl]benzyl}quinoline-4-carboxamide

HPLC retention time (min.): 3.11; Mass data:941 $(2M + H)^+$, 471 $(M + H)^+$, 236.

Example 2(196)

N-{3-[4-(3,4-dimethoxybenzyl)piperazin-1-yl]benzyl}-2-fluorobenzamide

HPLC retention time (min.): 3.29; Mass data:927 $(2M + H)^+$, 464 $(M + H)^+$.

Example 2(197)

N-{3-[4-(3,4-dimethoxybenzyl)piperazin-1-yl]benzyl}-3-methoxybenzamide

HPLC retention time (min.): 3.29; Mass data:951 $(2M + H)^+$, 476 $(M + H)^+$.

Example 2(198)

N-{3-[4-(3,4-dimethoxybenzyl)piperazin-1-yl]benzyl}-3-(trifluoromethyl)benzamide

HPLC retention time (min.): 3.47; Mass data:514 $(M + H)^+$.

Example 2(199)

N-{3-[4-(3,4-dimethoxybenzyl)piperazin-1-yl]benzyl}-2-(trifluoromethyl)benzamide

HPLC retention time (min.): 3.33; Mass data:514 $(M + H)^+$.

Example 2(200)

3-(acetylamino)-N-{3-[4-(3,4-dimethoxybenzyl)piperazin-1-yl]benzyl}benzamide

HPLC retention time (min.): 3.12; Mass data:503 $(M + H)^+$.

Example 2(201)

N-{3-[4-(3,4-dimethoxybenzyl)piperazin-1-yl]benzyl}-2-(methylthio)benzamide

HPLC retention time (min.):3.29; Mass data:983 $(2M + H)^+$, 492 $(M + H)^+$.

Example 2(202)

N-{3-[4-(3,4-dimethoxybenzyl)piperazin-1-yl]benzyl}-4-ethylbenzamide

HPLC retention time (min.):3.44; Mass data:947 $(2M + H)^+$, 474 $(M + H)^+$.

Example 2(203)

N-{3-[4-(3,4-dimethoxybenzyl)piperazin-1-yl]benzyl}-2,6-dimethoxybenzamide

HPLC retention time (min.):3.22; Mass data:506 $(M + H)^+$.

Example 2(204)

2-chloro-N-{3-[4-(3,4-dimethoxybenzyl)piperazin-1-yl]benzyl}benzamide

HPLC retention time (min.):3.27; Mass data:959 $(2M + H)^+$, 480 $(M + H)^+$.

Example 2(205)

3-chloro-N-{3-[4-(3,4-dimethoxybenzyl)piperazin-1-yl]benzyl}benzamide

HPLC retention time (min.):3.38; Mass data:959 $(2M + H)^+$, 480 $(M + H)^+$.

Example 2(206)

4-chloro-N-{3-[4-(3,4-dimethoxybenzyl)piperazin-1-yl]benzyl}-3-methylbenzamide

HPLC retention time (min.):3.49; Mass data:987 $(2M + H)^+$, 494 $(M + H)^+$.

Example 2(207)

3-chloro-N-{3-[4-(3,4-dimethoxybenzyl)piperazin-1-yl]benzyl}-4-methylbenzamide

HPLC retention time (min.):3.45; Mass data:987 $(2M + H)^+$, 494 $(M + H)^+$.

Example 2(208)

4-cyano-N-{3-[4-(3,4-dimethoxybenzyl)piperazin-1-yl]benzyl}benzamide

HPLC retention time (min.):3.27; Mass data:941 $(2M + H)^+$, 471 $(M + H)^+$.

Example 2(209)

N-{3-[4-(3,4-dimethoxybenzyl)piperazin-1-yl]benzyl}-3,4-dimethylbenzamide

HPLC retention time (min.):3.42; Mass data:947 $(2M + H)^+$, 474 $(M + H)^+$.

Example 2(210)

6-chloro-N-{3-[4-(3,4-dimethoxybenzyl)piperazin-1-yl]benzyl}nicotinamide

HPLC retention time (min.):3.23; Mass data:961 $(2M + H)^+$, 481 $(M + H)^+$.

Example 2(211)

N-{3-[4-(3,4-dimethoxybenzyl)piperazin-1-yl]benzyl}quinoline-4-carboxamide

HPLC retention time (min.):3.03; Mass data:993 $(2M + H)^+$, 497 $(M + H)^+$, 347, 151.

Example 2(212)

4-t-butyl-N-{3-[4-(3,4-dimethoxybenzyl)piperazin-1-yl]benzyl}benzamide

HPLC retention time (min.):3.55; Mass data:502 $(M + H)^+$.

Example 2(213)

N-{3-[4-(3,4-dimethoxybenzyl)piperazin-1-yl]benzyl}-2-furanamide

HPLC retention time (min.):3.16; Mass data:871 $(2M + H)^+$, 436 $(M + H)^+$.

Example 2(214)

N- {3-[4-(3,4-dimethoxybenzyl)piperazin-1-yl]benzyl}-3-methoxy-4-methylbenzamide

HPLC retention time (min.):3.40; Mass data:979 $(2M + H)^+$, 490 $(M + H)^+$.

Example 2(215)

N-{3-[4-(3,4-dimethoxybenzyl)piperazine-1-yl]benzyl}-4-(dimethylamino)benzamide

HPLC retention time (min.):3.16; Mass data:977 $(2M + H)^+$, 489 $(M + H)^+$.

Example 2(216)

2-chloro-N-{4-[1-(4-chlorobenzyl)piperidin-4-yl]benzyl}benzamide

HPLC retention time (min.):3.42; Mass data:905 $(2M + H)^+$, 453 $(M + H)^+$.

Example 2(217)

2-chloro-N-{4-[1-(4-chlorobenzyl)piperidin-4-yl]benzyl}isonicotinamide

HPLC retention time (min.):3.40; Mass data:907 $(2M + H)^+$, 454 $(M + H)^+$.

Example 2(218)

N- {4-[1-(4-chlorobenzyl)piperidin-4-yl]beuzyl}quinoline-4-carboxamide

HPLC retention time (min.):3.16; Mass data:939 $(2M + H)^+$, 470 $(M + H)^+$, 235.5.

Example 2(219)

N-{4-[1-(3,4-dimethoxybenzyl)piperidin-4-yl]benzyl}-3-(trifluoromethyl)benzamide

HPLC retention time (min.):3.49; Mass data:513 $(M + H)^+$.

Example 2(220)

N-{4-[1-(3,4-dimethoxybenzyl)piperidin-4-yl]benzyl}-2-(trifluoromethyl)benzamide

HPLC retention time (min.):3.38; Mass data:513 $(M + H)^+$.

Example 2(221)

N-{4-[1-(3,4-dimethoxybenzyl)piperidin-4-yl]benzyl}-2-(methylthio)benzamide

HPLC retention time (min.):3.33; Mass data:981 $(2M + H)^+$, 491 $(M + H)^+$.

Example 2(222)

N-{4-[1-(3,4-dimethoxybenzyl)piperidin-4-yl]benzyl}-4-ethylbenzamide

HPLC retention time (min.):3.45; Mass data:945 $(2M + H)^+$, 473 $(M + H)^+$.

Example 2(223)

N-{4-[1-(3,4-dimethoxybenzyl)piperidin-4-yl]benzyl}-2,4-dimethoxybenzamide

HPLC retention time (min.):3.36; Mass data:505 $(M + H)^+$, 355, 151.

Example 2(224)

N- {4-[1-(3,4-dimethoxybenzyl)piperidin-4-yl]benzyl}-6-methylpyridin-2-carboxamide

HPLC retention time (min.):3.27; Mass data:919 $(2M + H)^+$, 460 $(M + H)^+$, 310, 151.

Example 2(225)

2-chloro-N-{4-[1-(3,4-dimethoxybenzyl)piperidin-4-yl]benzyl}benzamide

HPLC retention time (min.):3.31, Mass data:957 $(2M + H)^+$, 479 $(M + H)^+$.

Example 2(226)

3-chloro-N-{4-[1-(3,4-dimethoxybenzyl)piperidin-4-yl]benzyl}benzamide

HPLC retention time (min.):3.42; Mass data:957 $(2M + H)^+$, 479 $(M + H)^+$.

Example 2(227)

4-chloro-N-{4-[1-(3,4-dimethoxybenzyl)piperidin-4-yl]benzyl}-3-methylbenzamide

HPLC retention time (min.):3.51; Mass data:985 $(2M + H)^+$, 493 $(M + H)^+$.

Example 2(228)

N-{4-[1-(3,4-dimethoxybenzyl)piperidin-4-yl]benzyl}-3,5-dimethylbenzamide

HPLC retention time (min.):3.47; Mass data:945 $(2M + H)^+$, 473 $(M + H)^+$.

Example 2(229)

N-{4-[1-(3,4-dimethoxybenzyl)piperidin-4-yl]benzyl}-2,3-dimethylbenzamide

HPLC retention time (min.):3.38; Mass data:945 $(2M + H)^+$, 473 $(M + H)^+$.

Example 2(230)

N-{4-[1-(3,4-dimethoxybenzyl)piperidin-4-yl]benzyl}-2,3-dimethoxybenzamide

HPLC retention time (min.):3.36; Mass data:505 $(M + H)^+$.

Example 2(231)

N-{4-[1-(3,4-dimethoxybenzyl)piperidin-4-yl]benzyl}-3,4-dimethoxybenzamide

HPLC retention time (min.):3.25; Mass data:505 $(M + H)^+$.

Example 2(232)

N-{4-[1-(3,4-dimethoxybenzyl)piperidin-4-yl]benzyl}quinoline-3-carboxamide

HPLC retention time (min.):3.12; Mass data:991 $(2M + H)^+$, 496 $(M + H)^+$, 346, 151.

Example 2(233)

2-chloro-N-{4-[1-(3,4-dimethoxybenzyl)piperidin-4-yl]benzyl}isonicotinamide

HPLC retention time (min.):3.27; Mass data:959 $(2M + H)^+$, 480 $(M + H)^+$.

Example 2(234)

2-chloro-N-{4-[1-(3,4-dimethoxybenzyl)piperidin-4-yl]benzyl}-6-methylisonicotinamide

HPLC retention time (min.):3.31; Mass data:987 $(2M + H)^+$, 494 $(M + H)^+$.

Example 2(235)

4-t-butyl-N-{4-[1-(3,4-dimethoxybenzyl)piperidin-4-yl]benzyl}benzamide

HPLC retention time (min.):3.58; Mass data:501 $(M + H)^+$.

Example 2(236)

N-{4-[1-(3,4-dimethoxybenzyl)piperidin-4-yl]benzyl}-2-furanamide

HPLC retention time (min.):3.18; Mass data:869 $(2M + H)^+$, 434 $(M + H)^+$.

Example 2(237)

N-{4-[1-(3,4-dimethoxybenzyl)piperidin-4-yl]benzyl}-3-methoxy-4-methylbenzamide

HPLC retention time (min.):3.42; Mass data:977 $(2M + H)^+$, 489 $(M + H)^+$.

Example 2(238)

N-{4-[1-(3,4-dimethoxybenzyl)piperidin-4-yl]benzyl}-4-methoxy-2-methylbenzamide

HPLC retention time (min.):3.33; Mass data:977 $(2M + H)^+$, 489 $(M + H)^+$.

Example 2(239)

N-{4-[1-(3,4-dimethoxybenzyl)piperidin-4-yl]benzyl}-4-(dimethylamino)benzamide

HPLC retention time (min.):3.16; Mass data:975 $(2M + H)^+$, 488 $(M + H)^+$.

Example 2(240)

N-{4-[1-(3,4-dimethoxybenzyl)piperidin-4-yl]benzyl}-3-fluoro-4-methylbenzamide

HPLC retention time (min.):3.44; Mass data:953 $(2M + H)^+$, 477 $(M + H)^+$.

Example 3

5-chloro-2-{4-[1-(3,4-dimethoxybenzyl)piperidin-4-yl]benzyl}-1H-isoindole-1,3(2H)-dione hydrochloride

To a solution of the compound prepared in reference example 3 (318 mg) in toluene (20 ml) was added 5-chloro-2-benzofuran-1,3-dione (186 mg). The reaction mixture was stirred for 6 days at 120°C. To the reaction mixture was added IN hydrochloric acid and the resulting mixture was extracted with dichloromethane. The extract was dried over anhydrous sodium sulfate and concentrated. The resulting residue was purified by column chromatography on silica gel (dichloromethane : methanol = 9 : 1) to give the compound of the present invention (12 mg) having the following physical data.
TLC:Rf 0.48 (dichloromethane : methanol = 10 : 1);
NMR($CD_3OD$): δ 7.86-7.81 (m, 2H), 7.33 (d, J=8.0 Hz, 2H), 7.23 (d, J=8.0 Hz, 2H), 7.14 (s, 1H), 7.06-7.01 (m, 3H), 4.78 (s, 2H), 4.26 (s, 2H), 3.88 (s, 3H), 3.85 (s, 3H), 3.58-3.54 (m, 2H), 3.10-3.00 (m, 2H), 2.90 (m, 1H), 2.10-1.90 (m, 4H).

Example 4(1) - 4(41)

By the same procedure as described in example 2 using a corresponding amine derivative in place of {3-[1-(4-methoxybenzyl)piperidin-4-yl]benzyl}amine and a corresponding carboxylic acid in place of 4-(methylthio)benzoic acid, the compounds of the present invention were given.

Example 4(1)

N-{3-[1-(4-chlorobenzyl)piperidin-4-yl]benzyl}-2-naphthamide

HPLC retention time (min.):3.64; Mass data:470 $(M + H)^+$.

Example 4(2)

N-[3-(4-benzylpiperazin-1-yl)benzyl]-2-methylbenzamide retention time (min.):3.29; Mass data:400 $(M + H)^+$.

Example 4(3)

N- {3-[4-(4-fluorobenzyl)piperazin-1-yl]benzyl}-2-methylbenzamide

HPLC retention time (min.):3.31; Mass data:418 $(M + H)^+$.

Example 4(4)

N-[3-(1-benzyl-1,2,3,6-tetrahydropyridin-4-yl)benzyl]-2-methylbenzamide

HPLC retention time (min.):3.35; Mass data:397 $(M + H)^+$.

Example 4(5)

N-{2-[1-(4-fluorobenzyl)piperidin-4-yl]benzyl}-2,4-dimethylbenzamide

HPLC retention time (min.):3.49; Mass data:431 $(M + H)^+$.

Example 4(6)

N-{2-[1-(4-fluorobenzyl)piperidin-4-yl]benzyl}-2,5-dimethylbenzamide

HPLC retention time (min.):3.47; Mass data:431 $(M + H)^+$.

Example 4(7)

N-{2-[1-(4-fluorobenzyl)piperidin-4-yl]benzyl}-3-methylbenzamide

HPLC retention time (min.):3.46; Mass data:417 $(M + H)^+$.

Example 4(8)

N-{2-[1-(4-fluorobenzyl)piperidin-4-yl]benzyl}-2-methylbenzamide

HPLC retention time (min.):3.39; Mass data:417 $(M + H)^+$,

Example 4(9)

N-{2-[1-(4-chlorobenzyl)piperidin-4-yl]benzyl}benzamide

HPLC retention time (min.):3.43; Mass data:419 $(M + H)^+$.

Example 4(10)

N-{2-[1-(4-chlorobenzyl)piperidin-4-yl]benzyl}-2-methylbenzamide

HPLC retention time (min.):3.47; Mass data:433 $(M + H)^+$.

Example 4(11)

2-chloro-N-{2-[1-(4-fluorobenzyl)piperidin-4-yl]benzyl}benzamide

HPLC retention time (min.):3.36; Mass data:437 $(M + H)^+$.

Example 4(12)

2,3-dichloro-N-{2-[1-(4-fluorobenzyl)piperidin-4-yl]benzyl}benzamide

HPLC retention time (min.):3.45; Mass data:471 $(M + H)^+$.

Example 4(13)

2,5-dichloro-N-{2-[1-(4-fluorobenzyl)piperidin-4-yl]benzyl}benzamide

HPLC retention time (min.):3.47; Mass data:475, 473, 471 $(M + H)^+$.

Example 4(14)

2-chloro-6-fluoro-N-{2-[1-(4-fluorobenzyl)piperidin-4-yl]benzyl}benzamide

HPLC retention time (min.):3.36; Mass data:457, 455 $(M + H)^+$.

Example 4(15)

N-{2-[1-(4-fluorobenzyl)piperidin-4-yl]benzyl}-2,6-dimethoxybenzamide

HPLC retention time (min.):3.29; Mass data:463 $(M + H)^+$.

Example 4(16)

N-{2-[1-(4-fluorobenzyl)piperidin-4-yl]benzyl}-1,1'-biphenyl-2-carboxamide

HPLC retention time (min.):3.51; Mass data:479 $(M + H)^+$.

Example 4(17)

N-{2-[1-(4-fluorobenzyl)piperidin-4-yl]benzyl}-2-(trifluoromethyl)benzamide

HPLC retention time (min.):3.42; Mass data:471 $(M + H)^+$.

Example 4(18)

N-{2-[1-(4-fluorobenzyl)piperidin-4-yl]benzyl}-2,3-dimethylbenzamide

HPLC retention time (min.):3.42; Mass data:431 $(M + H)^+$.

Example 4(19)

N-{2-[1-(4-fluorobenzyl)piperidin-4-yl]benzyl}-3-methylthiophene-2-carboxamide

HPLC retention time (min.):3.36; Mass data:423 $(M + H)^+$.

Example 4(20)

N-{2-[1-(4-fluorobenzyl)piperidin-4-yl]benzyl}-1H-indole-4-carboxamide

HPLC retention time (min.):3.29; Mass data:442 $(M + H)^+$.

Example 4(21)

5-chloro-N-{2-[1-(4-fluorobenzyl)piperidin-4-yl]benzyl}-2-methoxybenzamide

HPLC retention time (min.):3.51; Mass data:469, 467 $(M + H)^+$.

Example 4(22)

N-{2-[1-(4-fluorobenzyl)piperidin-4-yl]benzyl}-2-(methylsulfanyl)benzamide

HPLC retention time (min.):3.38; Mass data:449 $(M + H)^+$.

Example 4(23)

5-fluoro-N- {2-[1-(4-fluorobenzyl)piperidin-4-yl]benzyl}-2-methylbenzamide

HPLC retention time (min.):3.40; Mass data:435 $(M + H)^+$.

Example 4(24)

3-chloro-2-fluoro-N-{2-[1-(4-fluorobenzyl)piperidin-4-yl]benzyl}benzamide

HPLC retention time (min.):3.45; Mass data:457, 455 $(M + H)^+$.

Example 4(25)

2-ethyl-N-{2-[1-(4-fluorobenzyl)piperidin-4-yl]benzyl}benzamide

HPLC retention time (min.):3.44; Mass data:431 $(M + H)^+$.

Example 4(26)

3-chloro-N-{2-[1-(4-fluorobenzyl)piperidin-4-yl]benzyl}-2-methylbenzamide

HPLC retention time (min.):3.47; Mass data:453, 451 $(M + H)^+$.

Example 4(27)

3-fluoro-N-{2-[1-(4-fluorobenzyl)piperidin-4-yl]benzyl}-2-methylbenzamide

HPLC retention time (min.):3.40; Mass data:435 $(M + H)^+$.

Example 4(28)

N-{2-[1-(4-fluorobenzyl)piperidin-4-yl]benzyl}-2,4,5-trimethylbenzamide

HPLC retention time (min.):3.53; Mass data:445 $(M + H)^+$.

Example 4(29)

N-{2-[1-(4-fluorobenzyl)piperidin-4-yl]benzyl}-2,3-dihydro-1,4-benzodioxine-5-carboxamide

HPLC retention time (min.):3.36; Mass data:461 $(M + H)^+$.

Example 4(30)

5-chloro-2-fluoro-N-{2-[1-(4-fluorobenzyl)piperidin-4-yl]benzyl}benzamide

HPLC retention time (min.):3.45; Mass data:457, 455 $(M + H)^+$.

Example 4(31)

2-fluoro-N-{2-[1-(4-fluorobenzyl)piperidin-4-yl]benzyl}-6-methoxybenzamide

HPLC retention time (min.):3.33; Mass data:451 $(M + H)^+$.

Example 4(32)

5-fluoro-N-{2-[1-(4-fluorobenzyl)piperidin-4-yl]benzyl}-2-methoxybenzamide

HPLC retention time (min.):3.44; Mass data:451 $(M + H)^+$.

Example 4(33)

2,3-dichloro-N-{2-[4-(4-fluorobenzyl)piperazin-1-yl]benzyl}benzamide

HPLC retention time (min.):3.44; Mass data:476, 474, 472 $(M + H)^+$.

Example 4(34)

N-{2-[4-(4-fluorobenzyl)piperazin-1-yl]benzyl}-2,6-dimethoxybenzamide

HPLC retention time (min.):3.27; Mass data:464 $(M + H)^+$.

Example 4(35)

N-{2-[4-(4-fluorobenzyl)piperazin-1-yl]benzyl}-2-(trifluoromethyl)benzamide

HPLC retention time (min.):3.40; Mass data:472 $(M + H)^+$.

Example 4(36)

N-{2-[4-(4-fluorobenzyl)piperazin-1-yl]benzyl}-2,5-dimethylbenzamide

HPLC retention time (min.):3.42; Mass data:432 $(M + H)^+$.

Example 4(37)

N-{2-[4-(4-fluorobenzyl)piperazin-1-yl]benzyl}-1-methyl-1H-pyrrole-2-carboxamide

HPLC retention time (min.):3.31; Mass data:407 $(M + H)^+$.

Example 4(38)

N-{2-[4-(4-fluorobenzyl)piperazin-1-yl]benzyl}-3-methylthiophene-2-carboxamide

HPLC retention time (min.):3.34; Mass data:424 $(M + H)^+$.

Example 4(39)

N-{2-[4-(4-fluorobenzyl)piperazin-1-yl]benzyl}-2-(methylsulfanyl)benzamide

HPLC retention time (min.):3.34; Mass data:450 $(M + H)^+$.

Example 4(40)

2-ethyl-N-{2-[4-(4-fluorobeuzyl)piperazin-1-yl]benzyl}benzamide

HPLC retention time (min.):3.42; Mass data:432 $(M + H)^+$.

Example 4(41)

2-fluoro-N-{2-[4-(4-fluorobenzyl)piperazin-1-yl]benzyl}-6-methoxybenzamide

HPLC retention time (min.):3.31; Mass data:452 $(M + H)^+$.

Example 4(42)

N-{2-[1-(4-fluorobenzyl)piperidin-4-yl]benzyl}-N-phenylacetamide

HPLC retention time (min.):3.38; Mass data:417 $(M + H)^+$.

Example 4(43)

N'-ethyl-N-{2-[1-(4-fluorobenzyl)piperidin-4-yl]benzyl}-N-phenylurea

HPLC retention time (min.):3.42; Mass data:446 $(M + H)^+$.

Example 4(44)

methyl 2-[1-(4-fluorobenzyl)piperidin-4-yl]benzyl(phenyl)carbamate

HPLC retention time (min.):3.43; Mass data:433 $(M + H)^+$.

Example 4(45)

N-benzyl-N-{2-[1-(4-fluorobenzyl)piperidin-4-yl]benzyl}acetamide

HPLC retention time (min.):3.38; Mass data:431 $(M + H)^+$.

Example 4(46)

N-benzyl-N'-ethyl-N- {2-[1-(4-fluorobenzyl)piperidin-4-yl]benzyl}urea

HPLC retention time (min.):3.44; Mass data:460 $(M + H)^+$.

Example 4(47)

methyl benzyl{2-[1-(4-fluorobenzyl)piperidin-4-yl]benzyl}carbamate
HPLC retention time (min.):3.55; Mass data:447 $(M + H)^+$.

Biochemical Examples

It was confirmed that the compound of formula (I) of the present invention had a CCR5 antagonistic activity, for example, by the following experiments.
The whole procedures were carried out by preparing gene high expressing cells under basic genetic engineering

methods by utilizing conventional methods. Also, as to the methods for the measurement in the present invention, improvement of measurement accuracy and/or modification of measurement sensitibity were adopted in order to evaluate the compound of the present invention. Detailed methods for the experiment are illustrated below.

(1) Isolation of human CCR5 genes

Human placental cDNA was prepared using Marathon cDNA amplification kit (Clontech). PCR primers hCCR5XbaI-F1: 5'-AGCTAGTCTA GATCCGTTCC CCTACAAGAA ACTCTCC-3' (SEQ ID NO:1) and hCCR5XbaI-R1: 5'-AGCTAGTCTA GAGTGCACAA CTCTGACTGG GTCACCA-3' (SEQ ID NO:2) were designed based on the sequence of GenBank U54994.

Using the human placental cDNA as the template and using Ex Taq (Takara), PCR (at 95°C 2 minutes → [at 95°C for 30 seconds, at 60°C for 45 seconds, at 72°C for 1 minute] × 35 times) was carried out. Thus amplified PCR adduct was subjected to 1% agarose gel electrophoresis, purified using QIAquick Gel Extraction Kit (QIAGEN), and then digested with a restriction enzyme VbaI. The digested fragments were ligated to an expression vector pEF-BOS-bsr using DNA Ligation Kit Ver.2 (Takara) and transformed into Escherichia coli DH5α. By preparing the resulting plasmid pEF-BOS-bsr/hCCR5, its DNA sequence was verified.

(2) Culturing of CHO cells

CHO-dhfr(-) was cultured using Ham's F-12 (containing fetal bovine serum (10 %), penicillin (50 U/ml) and streptomycin (50 mg/ml)). Also, the transduced cells were cultured by adding blastcidin (5 mg/ml) to the above medium.

(3) Transduction into CHO cells

The plasmid pEF-BOS-bsr/hCCR5 was transduced into the CHO-dhfr(-) cells using DMRIE-C reagent (Gibco BRL). After 48 hours, the medium was replaced with a medium containing 5 mg/ml of blasticidin to carry out the selection, resulting in establishing stable over-expressing cells.

(4) Inhibition test on the binding of RANTES to CCR5 (activity of RANTES to induce transient increase of Ca ion)

Thus established human CCR5 stable over-expressing CHO cells (CCR5/CHO cells) were suspended in Ham's F-12 medium containing FBS (10%) and dispensed in 3.0 × $10^6$ cells/well portions into a 96 well plate. One day after culturing at 37°C, the culture supernatant was discarded, and Ham's F-12 medium (containing Fura-2AM (5 mM), Probenecid (2.5 mM) and HEPES (20 mM, pH 7.4) was dispensed in 80 ml/well portions to carry out 1 hour of incubation at 37°C under shaded condition. After washing twice with 1 x Hanks/HEPES (20 mM, pH 7.4) solution, the same solution was dispensed in 100 μl/well portions. Each of the test compounds was added to the thus Fura-2AM-incorporated CCR5/CHO cell, and 3 minutes thereafter, a recombinant human RANTES (PeproTach) diluted with 1 x Hanks / HEPES (20 mM; pH 7.4) solution was added thereto to a final concentration of 10 nM. Transient increase in the intracellular $Ca^{2+}$ concentration induced by the human RANTES was measured using a $Ca^{2+}$ detector for 96 well use (Hamamatsu Photonics), and inhibition ration (%) of the test compound was calculated by the following calculation formula.

$$\text{Inhibition ratio} = (Ec - Ea) / Ec \times 100$$

Ec : measured value of $Ca^{2+}$ transient increase by RANTES
Ea : measured value of $Ca^{2+}$ transient increase by RANTES when a test compound was added

It was confirmed that the compound of formula (I) of the present invention had a CCR1 antagonistic activity by Ca assay method using THP-1 cells (see *European Journal of Pharmacology*, 389, 41-49 (2000)).

As a result, the compound of the present invention showed more than 80% inhibition at 30 μM. For example, the compound of example 1 showed an $IC_{50}$ value of 4.16 μM.

Formulation Example 1

4-Chloro-N-{4-[1-(3,4-dimethoxybenzyl)piperidin-4-yl]benzyl}benzamide hydrochloride (5.0 kg), carboxymethylcellulose calcium (disintegrating agent, 0.2 kg), magnesium stearate (lubricating agent, 0.1 kg) and microcrystalline cellulose (4.7 kg) were admixed by a conventional method and punched out to give 100,000 tablets each containing 50

mg of active ingredient.

Formulation Example 2

4-Chloro-N-{4-[1-(3,4-dimethoxybenzyl)piperidin-4-yl]benzyl}benzamide hydrochloride (2.0 kg), mannitol (20 kg) and distilled water (500 L) were admixed by a conventional method, and the resulting solution was sterilized by a conventional method, placed 5 ml portions into ampoules and freeze-dried to give 100,000 ampoules each containing 20 mg of active ingredient.

Industrial Applicability

The compound of formula (I), a salt thereof or a prodrug thereof of the present invention antagonizes chemokine receptors (especially CCR1 and/or CCR5) and therefore it is useful as a medicament for the prevention and/or treatment of various diseases.

**Claims**

1. A compound of formula (I):

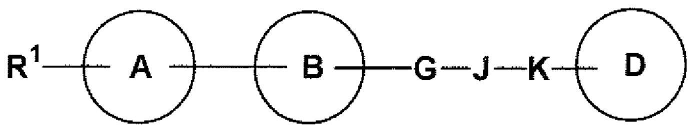

(I)

wherein $R^1$ represents aliphatic hydrocarbon optionally having substituent(s),
ring A represents a cyclic group comprising at least one nitrogen atom optionally having further substituent(s) besides $R^1$,
ring B represents a cyclic group optionally having substituent(s) and is attached to ring A via a bond,
G represents a bond or a spacer comprising 1-4 atoms in the main chain,
J represents a spacer having a hydrogen-bond accepting group optionally having substituent(s),
K represents a bond or a spacer comprising 1-4 atoms in the main chain, and
ring D represents a cyclic group optionally having substituent(s), which may form a ring together with a substituent on J,
a salt thereof, an N-oxide thereof, a solvate thereof or a prodrug thereof

2. The compound according to claim 1, wherein the hydrogen-bond accepting group in J is carbonyl, thiocarbonyl, imino, sulfonyl or sulfinyl, a salt thereof, an N-oxide thereof, a solvate thereof or a prodrug thereof.

3. The compound according to claim 1,
wherein J is -CO-, -CONR$^2$-, -NR$^2$CO-, -OCO-, -COO-, -CS-, -CSNR$^{2-}$-NR$^2$CS-, -O-CS-, -CS-O-, -$SO_2$-, -$SO_2$NR$^2$-, -NR$^2$$SO_2$-, -O-$SO_2$-, -$SO_2$-O-, -S(O)-, -S(O)NR$^2$-, -NR$^2$S(O)-, -O-S(O)-, -S(O)-O-, or -C(=NR$^3$)-,
wherein $R^2$ represents a hydrogen atom, optionally substituted aliphatic hydrocarbon or an optionally substituted cyclic group and $R^3$ represents a hydrogen atom, cyano, optionally protected hydroxy, optionally substituted amino, optionally substituted aliphatic hydrocarbon or an optionally substituted cyclic group,
a salt thereof, an N-oxide thereof a solvate thereof or a prodrug thereof

4. The compound according to claim 1,
wherein J is -N(COR$^4$)-, -N(CONHR$^5$)-, -N(COOR$^6$)-, or -N($SO_2$R$^7$)-,
wherein $R^4$, $R^5$, $R^6$ and $R^7$ each represents a hydrogen atom, optionally substituted aliphatic hydrocarbon or an optionally substituted cyclic group,
a salt thereof, an N-oxide thereof, a solvate thereof or a prodrug thereof.

5. The compound according to claim 1, wherein the cyclic group represented by ring D is a C3-15 mono-, bi- or tri-cyclic aromatic carbocyclic ring which may be partially or completely saturated, or a 3-15 membered mono-, bi- or tri-cyclic aromatic heterocyclic ring comprising 1-5 of heteroatom selected from oxygen, nitrogen and sulfur which may be partially or completely saturated,
a salt thereof, an N-oxide thereof, a solvate thereof or a prodrug thereof

6. The compound according to claim 1, wherein the cyclic group represented by ring D is a C3-15 mono-, bi- or tri-cyclic aromatic carbocyclic ring, or a 3-15 membered mono-, bi- or tri-cyclic aromatic heterocyclic ring containing 1-5 of heteroatom,

a salt thereof, an N-oxide thereof, a solvate thereof or a prodrug thereof.

7. The compound according to claim 1, wherein
wherein

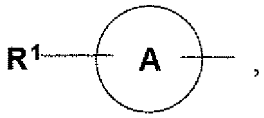

wherein all symbols have the same meanings as in claim 1, is

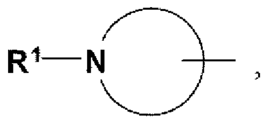

wherein

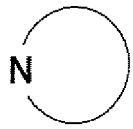

is a cyclic ring comprising at least one nitrogen atom and optionally having substituent(s) and $R^1$ has the same meaning as in claim 1,
a salt thereof, an N-oxide thereof, a solvate thereof or a prodrug thereof.

8. The compound according to claim 7,
wherein

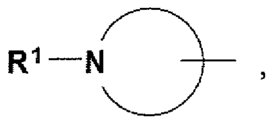

wherein all symbols have the same meanings as in claim 1,
is piperidine, piperazine, pyrrolidine, 1,4-diazepane, 1,2,3,6-tetrahydropyridine or 8-azabicyclo[3.2.1]octane ring optionally having substituent(s),
a salt thereof, an N-oxide thereof, a solvate thereof or a prodrug thereof.

9. A pharmaceutical composition comprising a compound of formula (I)

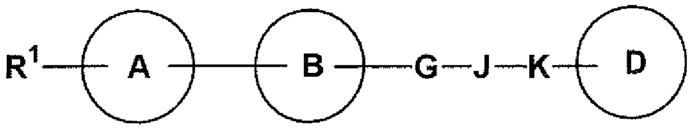

(I)

wherein $R^1$ represents aliphatic hydrocarbon optionally having substituent(s),
ring A represents a cyclic group comprising at least one nitrogen atom optionally having further substituent(s) besides $R^1$,
ring B represents a cyclic group optionally having substituent(s) and is attached to ring A via a bond,
G represents a bond or a spacer comprising 1-4 atoms in the main chain,
J represents a spacer having a hydrogen-bond accepting group optionally having substituent(s),

K represents a bond or a spacer comprising 1-4 atoms in the main chain, and
ring D represents a cyclic group optionally having substituent(s), which may form a ring together with a substituent on J,
a salt thereof, an N-oxide thereof, a solvate thereof or a prodrug thereof.

**10.** The composition according to claim 9, which is a chemokine receptor antagonist.

**11.** The composition according to claim 10, wherein the chemokine receptor is CCR1.

**12.** The composition according to claim 10, wherein the chemokine receptor is CCR5.

**13.** The composition according to claim 10, which is a medicament for the prevention and/or treatment of human immunodeficiency virus infectious disease, acquired immunodeficiency syndrome and/or organ rejection in transplantation.

**14.** The composition according to claim 10, which is a medicament for the prevention and/or treatment of multiple sclerosis and/or arthritis.

**15.** A method for the prevention and/or treatment of diseases induced by a chemokine receptor in a mammal, which comprises administering to an mammal an effective amount of the compound according to claim 1, a salt thereof, an N-oxide thereof, a solvate thereof or a prodrug thereof

**16.** Use of the compound according to claim 1, a salt thereof, an N-oxide thereof, a solvate thereof or a prodrug thereof, for the manufacture of a medicament for the prevention and/or treatment of the diseases induced by chemokine receptors.

**17.** A medicament comprising the compound according to claim 1, a salt thereof, an N-oxide thereof, a solvate thereof or a prodrug thereof, and one or more selected from the group consisting of a protease inhibitor, a reverse transcriptase inhibitor, a fusion inhibitor, an HIV integrase inhibitor, a chemokine inhibitor, a steroidal drug, interferon, an immunosuppressant, an aldose reductase inhibitor, a cannabinoid-2 receptor agonist, adrenocorticotropic hormone, a metalloproteinase inhibitor, a non-steroidal anti-inflammatory drug, a prostaglandin drug, a phosphodiesterase inhibitor, a disease modifying anti-rheumatic drug, an anti-inflammatory enzyme drug, a cartilage-protecting drug, a T-cell inhibitor, a TNF-$\alpha$ inhibitor, an IL-6 inhibitor, an interferon $\gamma$ agonist, an IL-1 inhibitor and an NF-$\kappa$B inhibitor.

## INTERNATIONAL SEARCH REPORT

International application No.
PCT/JP2004/005504

A. CLASSIFICATION OF SUBJECT MATTER
Int.Cl[7] C07D211/26, 211/52, 211/28, 211/70, 307/68, 295/12, 215/50, 213/60, 207/20, 409/06, 401/10, 405/10, 405/06, 409/10, 333/28, A61K31/451, 31/454, 31/44, 31/381, 31/341, 31/495, 31/496,

According to International Patent Classification (IPC) or to both national classification and IPC

B. FIELDS SEARCHED

Minimum documentation searched (classification system followed by classification symbols)
Int.Cl[7] C07D211/26, 211/52, 211/28, 211/70, 307/68, 295/12, 215/50, 213/60, 207/20, 409/06, 401/10, 405/10, 405/06, 409/10, 333/28, A61K31/451, 31/454, 31/44, 31/381, 31/341, 31/495, 31/496,

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| E,X | WO 04/52845 A1 (BAYER HEALTHCARE AG.), 24 June, 2004 (24.06.04), Full text (Family: none) | 1-9 |
| P,X | WO 04/14864 A1 (ASTEX TECHNOLOGY LTD.), 19 February, 2004 (19.02.04), Full text (Family: none) | 1-9 |
| P,X | WO 03/91256 A1 (Shionogi & Co., Ltd.), 06 November, 2003 (06.11.03), Full text (Family: none) | 1-9 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 July, 2004 (20.07.04) | 03 August, 2004 (03.08.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

INTERNATIONAL SEARCH REPORT

International application No. PCT/JP2004/005504

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 03/53912 A1 (BRISTOL-MYER SQUIBB CO.), 03 July, 2003 (03.07.03), Full text (Family: none) | 1-9 |
| P,X | WO 03/33483 A1 (GLAXO GROUP LTD.), 24 April, 2003 (24.04.03), Full text & EP 1436272 A1 | 1-9 |
| P,X | WO 03/32980 A1 (GLAXO GROUP LTD.), 24 April, 2003 (24.04.03), Full text & EP 1435942 A1 | 1-9 |
| A | WO 02/05819 A1 (SMITHKLINE BEECHAM CORP.), 24 January, 2002 (24.01.02), Claims & EP 1313477 A1 | 1-14,16,17 |
| A | JP 2002-535256 A (SMITHKLINE BEECHAM CORP.), 22 October, 2002 (22.10.02), Claims & WO 00/42852 A1 & EP 1146790 A1 | 1-14,16,17 |
| X | JP 10-306078 A (Mitsubishi Chemical Corp.), 17 November, 1998 (17.11.98), Full text (Family: none) | 1-12,16 |
| A | WO 98/25617 A1 (MERCK & CO., INC.), 18 June, 1998 (18.06.98), Claims & AU 9855224 A1 | 1-14,16,17 |
| X | JP 7-258217 A (Adir et Co.), 09 October, 1995 (09.10.95), Full text & EP 669322 A1 & CA 2143249 A & SU 5565457 A & CN 1120537 A | 1-9 |
| X | JP 3-275657 A (Kaneka Corp.), 06 December, 1991 (06.12.91), Full text & EP 407200 A1 & CA 2020437 A & US 5294643 A | 1-12,16 |
| X | WO 01/77101 A1 (ASTRAZENECA AB), 18 October, 2001 (18.10.01), Claims & EP 1274701 A1 & JP 15-530393 A & CA 2403012 A & US 2002/77337 A1 | 1-14,16,17 |

Form PCT/ISA/210 (continuation of second sheet) (January 2004)

**INTERNATIONAL SEARCH REPORT**

International application No.
PCT/JP2004/005504

C (Continuation). DOCUMENTS CONSIDERED TO BE RELEVANT

| Category* | Citation of document, with indication, where appropriate, of the relevant passages | Relevant to claim No. |
|---|---|---|
| X | WO 02/81449 A1 (NOVARTIS-ERFINDUNGEN VERWAL TUNGS G.M.B.H.), 17 October, 2002 (17.10.02), Claims & CA 2439241 A | 1-14,16,17 |
| X | WO 00/66558 A1 (SCHERING CORP.), 09 November, 2000 (09.11.00), Claims & EP 1175401 A1 & JP 14-543185 A & US 2003/69252 A1 | 1-14,16,17 |
| X | WO 00/66141 A1 (SCHERING CORP.), 09 November, 2000 (09.11.00), Claims & EP 1175224 A1 & JP 14-543144 A | 1-14,16,17 |
| X | WO 00/66559 A1 (SCHERING CORP.), 09 November, 2000 (09.11.00), Claims & EP 1175402 A1 & JP 14-543186 A | 1-14,16,17 |

Form PCT/ISA/210 (continuation of second sheet) (January 2004)

**INTERNATIONAL SEARCH REPORT**

International application No.
PCT/JP2004/005504

**Box No. I Nucleotide and/or amino acid sequence(s) (Continuation of item1.b of the first sheet)**

1. With regard to any nucleotide and/or amino acid sequence disclosed in the international application and necessary to the claimed invention, the international search was carried out on the basis of:

   a. type of material
      - [X] a sequence listing
      - [ ] table(s) related to the sequence listing

   b. format of material
      - [ ] in written format
      - [X] in computer readable form

   c. time of filing/furnishing
      - [ ] contained in the international application as filed
      - [X] filed together with the international application in computer readable form
      - [ ] furnished subsequently to this Authority for the purposes of search

2. [X] In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3. Additional comments:

Form PCT/ISA/210 (continuation of first sheet (1)) (January 2004)

**INTERNATIONAL SEARCH REPORT**

International application No.
PCT/JP2004/005504

**Box No. II Observations where certain claims were found unsearchable (Continuation of item 2 of first sheet)**

This international search report has not been established in respect of certain claims under Article 17(2)(a) for the following reasons:

1. ☒ Claim No.: 15
because they relate to subject matter not required to be searched by this Authority, namely:
The invention relates to methods for treatment of a human body by therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest.

☐ No protest accompanied the payment of additional search fees.

Form PCT/ISA/210 (continuation of first sheet (2)) (January 2004)

**INTERNATIONAL SEARCH REPORT**

International application No.
PCT/JP2004/005504

Continuation of A. CLASSIFICATION OF SUBJECT MATTER
(International Patent Classification (IPC))

Int.Cl$^7$ 31/40, 31/4709, 31/4525, 31/453, 31/45, 31/4535, 31/454, 31/4545, 31/455, A61P43/00, 29/00, 37/06, 37/08, 31/04, 31/18, 11/06, 13/12, 11/02, 1/16, 19/02, 27/02, 1/04, 17/00, 25/00, 9/10, 11/00, 3/10, 35/04

(According to International Patent Classification (IPC) or to both national classification and IPC)

Continuation of B. FIELDS SEARCHED
Minimum documentation searched (International Patent Classification (IPC))

Int.Cl$^7$ 31/40, 31/4709, 31/4525, 31/453, 31/45, 31/4535, 31/454, 31/4545, 31/455

Minimum documentation searched (classification system followed by classification symbols)

<Subject of search>

Although the general formula (I) shown in the claims of this application comprehends a vast plurality of compounds, only some thereof are disclosed in the description within the meaning of PCT Article 5.

Consequently, claims 1-14, 16 and 17 of this application lack satisfactory support within the meaning of PCT Article 6.

Therefore, the subject of the international search on this application has been limited to rational scope based on particular compounds found as being disclosed in the description.

SEQUENCE LISTING

<110> ONO PHARMACEUTICAL CO., LTD.

<120> The heterocyclic compound containing nitrogen atom and use thereof

<130> ONF-4995PCT

<150> JP 2003-114172

<151> 2003-04-18

<150> JP 2003-346384

<151> 2003-10-03

<160> 2

<170> PatentIn Ver. 2.1

<210> 1

<211> 37

<212> DNA

<213> Artificial Sequence

<220>

<223> Description of Artificial Sequence:Forword primer hCCR5Xbal

<400> 1

agctagtcta gatccgttcc cctacaagaa actctcc 37

<210> 2

<211> 37

<212> DNA

<213> Artificial Sequence

<220>

<223> Description of Artificial Sequence:Revese primer

hCCR5XbaI

<400> 2

agctagtcta gagtgcacaa ctctgactgg gtcacca 37